# EUROPEAN PATENT SPECIFICATION

(11) **EP 1 585 522 B1**
(45) Date of publication and mention of the grant of the patent: **17.11.2010**
(21) Application number: 03808616.1
(22) Date of filing: 23.12.2003
(51) Int. Cl.: A61K 31/00, C07D 239/95, C07D 401/12, C07D 487/04, C07D 495/04, C07D 491/00, A61K 31/517, A61K 31/519, A61P 25/00, A61P 27/00, A61P 9/10, A61P 29/00, A61P 25/08, A61P 25/22, A61P 25/28, A61P 25/18

(54) **QUINAZOLINONES AS POTASSIUM CHANNEL MODULATORS**
QUINAZOLINONE ALS KALIUMKANALMODULATOREN
QUINAZOLINONES COMME MODULATEURS DE CANAUX POTASSIQUES

(30) Priority: 23.12.2002 US 436145 P
(43) Date of publication of application: 19.10.2005
(73) Proprietor: Icagen, Inc., Durham, NC 27703 (US)
(72) Inventor: MCNAUGHTON-SMITH, Grant, Andrew, Morrisville, NC 27560 (US); THOMAS, James, Barnwell, Jr., Efland, NC 27243 (US); AMATO, George, Salvatore, Cary, NC 27512 (US)
(74) Representative: Harrison Goddard Foote
(86) International application number: PCT/US2003/041657
(87) International publication number: WO 2004/058704

(56) References cited:
- US-A- 3 867 384
- US-A- 4 788 199
- US-A- 4 855 289
- US-A- 5 276 038
- US-A- 5 354 755
- MODICA, MARIA ET AL: "High affinity and selectivity of [[(arylpiperazinyl)alkyl]thio]thien o[2,3-d]pyrimidinone derivatives for the 5-HT1A receptor. Synthesis and structure-affinity relationships" EUROPEAN JOURNAL OF MEDICINAL CHEMISTRY, 35(7 & 8), 677-689 CODEN: EJMCA5; ISSN: 0223-5234, 2000, XP002387040

## Description

### CROSS-REFERENCES TO RELATED APPLICATIONS

This application is a non-provisional filing of U.S. Provisional Patent Application No. 60/436145, filed December 23, 2002.

### FIELD OF THE INVENTION

This invention relates to the use of certain quinazolinones as potassium channel modulators and to the treatment of diseases in which a potassium channel is implicated. Additionally, this invention relates to novel compounds that are useful as potassium channel modulators.

### BACKGROUND OF THE INVENTION

Ion channels are cellular proteins that regulate the flow of ions, including calcium, potassium, sodium and chloride, into and out of cells. These channels are present in all human cells and affect such processes as nerve transmission, muscle contraction and cellular secretion. Among the ion channels, potassium channels are the most ubiquitous and diverse, being found in a variety of animal cells such as nervous, muscular, glandular, immune, reproductive, and epithelial tissue. These channels allow the flow of potassium in and/or out of the cell under certain conditions. For example, the outward flow of potassium ions upon opening of these channels makes the interior of the cell more negative, counteracting depolarizing voltages applied to the cell. These channels are regulated, *e.g*., by calcium sensitivity, voltage-gating, second messengers, extracellular ligands, and ATP-sensitivity.

Potassium channels are associated with a number of physiological processes, including regulation of heartbeat, dilation of arteries, release of insulin, excitability of nerve cells, and regulation of renal electrolyte transport. Potassium channels are made by alpha subunits that fall into at least 8 families, based on predicted structural and functional similarities (Wei et al., Neuropharmacology 35(7): 805-829 (1997)). Three of these families (Kv, eag-related, and KQT) share a common motif of six transmembrane domains and are primarily gated by voltage. Two other families, CNG and SK/IIC, also contain this motif but are gated by cyclic nucleotides and calcium, respectively. The three other families of potassium channel alpha subunits have distinct patterns of transmembrane domains. Slo family potassium channels, or BK channels have seven transmembrane domains (Meera et al., Proc. Natl. Acad. Sci. U.S.A. 94(25): 14066-71 (1997)) and are gated by both voltage and calcium or pH (Schreiber et al., J. Biol. Chem. 273: 3509-16 (1998)). Another family, the inward rectifier potassium channels (Kir), belongs to a structural family containing two transmembrane domains, and an eighth functionally diverse family (TP, or "two-pore") contains two tandem repeats of this inward rectifier motif.

Potassium channels are typically formed by four alpha subunits, and can be homomeric (made of identical alpha subunits) or heteromeric (made of two or more distinct types of alpha subunits). In addition, potassium channels made from Kv, KQT and Slo or BK subunits have often been found to contain additional, structurally distinct auxiliary, or beta, subunits. These subunits do not form potassium channels themselves, but instead they act as auxiliary subunits to modify the functional properties of channels formed by alpha subunits. For example, the Kv beta subunits are cytoplasmic and are known to increase the surface expression of Kv channels and/or modify inactivation kinetics of the channel (Heinemann et al., J. Physiol. 493: 625-633 (1996); Shi et al., Neuron 16(4): 843-852 (1996)). In another example, the KQT family beta subunit, minK, primarily changes activation kinetics (Sanguinetti et al., Nature 384: 80-83 (1996)).

Slo or BK potassium channels are large conductance potassium channels found in a wide variety of tissues, both in the central nervous system and periphery. They play a key role in the regulation of processes such as neuronal integration, muscular contraction and hormone secretion. They may also be involved in processes such as lymphocyte differentiation and cell proliferation, spermatocyte differentiation and sperm motility. Three alpha subunits of the Slo family have been cloned, *i.e*., Slo1, Slo2, and Slo3 (Butler et al., Science 261: 221-224 (1993); Schreiber et al., J. Biol. Chem., 273: 3509-16 (1998); and Joiner et al., Nature Neurosci. 1: 462-469 (1998)). These Slo family members have been shown to be voltage and/or calcium gated, and/or regulated by intracellular pH.

Certain members of the Kv family of potassium channels were recently renamed (see, Biervert, et al., Science 279: 403-406 (1998)). KvLQT1 was re-named KCNQ1, and the KvLQT1-related channels (KvLR1 and KvLR2) were renamed KCNQ2 and KCNQ3, respectively. More recently, additional members of the KCNQ subfamily were identified. For example, KCNQ4 was identified as a channel expressed in sensory outer hair cells (Kubisch, et al., Cell 96(3): 437-446 (1999)). KCNQ5 (Kananura et al., Neuroreport 11(9):2063 (2000)), KCNQ 2/3 (Main et al., Mol. Pharmacol. 58: 253-62 (2000), and KCNQ 3/5 (Wickenden et al., Br. J. Pharma 132: 381 (2001)) have also recently been described.

KCNQ2 and KCNQ3 have been shown to be nervous system-specific potassium channels associated with benign familial neonatal convulsions ("BFNC"), a class of idiopathic generalized epilepsy (*see,* Leppert, et al., Nature 337: 647-648 (1989)). These channels have been linked to M-current channels (*see,* Wang, et al., Science 282: 1890-1893 (1998)). The discovery and characterization of these channels and currents provides useful insights into how these voltage dependent (Kv) potassium channels function in different environments, and how they respond to various activation mechanisms. Such information has now led to the identification of modulators of KCNQ2 and KCNQ3 potassium channels or the M-current, and the use of such modulators as therapeutic agents. The modulators are the subject of the present invention.
Modica et al. disclose in Eur. J. Med. Chem., 35(7 & 8), 677-689 (2000) [[(arylpiperazinyl)alkyl]thio]thieno[2,3-d]pyrimidinone derivatives having affinity for the 5-HT_{1A} receptor. This receptor plays a role in anxiety and depression.

### SUMMARY OF THE INVENTION

The present invention provides quinazolinones and pharmaceutically acceptable salts thereof ("compounds of the invention"), which are useful in the treatment of diseases through the modulation of potassium ion flux through voltage-dependent potassium channels.

In one aspect, the present invention provides compounds of the formula:

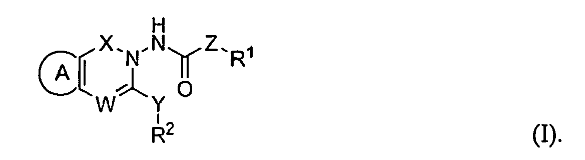

In Formula I, the symbol A represents a five- or six-membered substituted or unsubstituted aryl, five- and six-membered substituted or unsubstituted heteroaryl, substituted or unsubstituted C₄-C₈ cycloalkyl, and substituted or unsubstituted 5-8 membered heterocyclyl ring system. X represents CO.

W is N.

Z represents -CH₂-, -CHF-, -CF₂-, -CH=CH- or -N(R⁴)(CR^{4a}R^{4b})ₛ-, in which R⁴represents H or a substituted or unsubstituted C₁-C₅ alkyl group. The symbols R^{4a} and R^{4b} represent groups that are independently selected from H, substituted or unsubstituted aryl, substituted or unsubstituted heteroaryl, substituted or unsubstituted C₃-C₈ cycloalkyl, substituted or unsubstituted 5-7 membered heterocyclyl, or substituted or unsubstituted C₁-C₈ alkyl. R¹ is substituted or unsubstituted aryl, substituted or unsubstituted heteroaryl, substituted or unsubstituted C₃-C₈ cycloalkyl, substituted or unsubstituted 5-7 membered heterocyclyl, or substituted or unsubstituted C₁-C₈ alkyl. The index "s" is an integer from 1 to 3. Moreover, when "s" is greater than 1, each R^{4a} and R^{4b} are independently selected.

The symbol Y represents S(O)ₙ, in which the index "n" is an integer from 0-2. R² is CF₃, substituted or unsubstituted C₁-C₈ alkyl, substituted or unsubstituted aryl, substituted or unsubstituted heteroaryl, substituted or unsubstituted C₃-C₈ cycloalkyl, or substituted or unsubstituted 3-7-membered heterocyclyl group.

In another aspect, the present invention provides pharmaceutical compositions comprising a pharmaceutically acceptable excipient and a compound of the formula provided above.

In yet another aspect, the present invention provides a method for increasing flow through voltage dependent potassium channels in a cell, comprising contacting the cell with a compound of the formula provided above in an amount sufficient to open the potassium channels, provided that the method is not a method of treatment of the human or animal body by therapy.

In still another aspect, the present invention provides the use of a compound of the formula provided above for the manufacture of a medicament for treating a central or peripheral nervous system disorder or condition through the modulation of a voltage-dependent potassium channel.

Other objects and advantages of the present invention will be apparent from the detailed description that follows.

### BRIEF DESCRIPTION OF THE DRAWINGS

**FIGS. 1A-1G** display structures of representative compounds of the invention.

**FIGS. 2A-2B** are activity data for selected compounds of the invention.

### DETAILED DESCRIPTION OF THE INVENTION AND THE PREFERRED EMBODIMENTS

### Abbreviations and Definitions:

The abbreviations used herein have their conventional meaning within the chemical and biological arts. For example: CHO, Chinese hamster ovary; EBSS, Earl's Balanced Salt Solution; KCNQ, potassium channel Q; KCNQ2, potassium channel Q2; Et₃N, triethylamine; MeOH, methanol; and DMSO, dimethylsulfoxide.

"Compound of the invention," as used herein refers to a compound according to Formulae I or II or a combination thereof, and a pharmaceutically acceptable salt of a compound according to Formulae I or II or a combination thereof.

"Modulating," as used herein, refers to the ability of a compound of the invention to activate and/or inhibit a potassium channel, preferably, a KCNQ potassium channel.

"Opening" and "activating" are used interchangeably herein to refer to the partial or full activation of a KCNQ channel by a compound of the invention, which leads to an increase in ion flux either into or out of a cell in which a KCNQ channel is found.

Where substituent groups are specified by their conventional chemical formulae, written from left to right, they equally encompass the chemically identical substituents which would result from writing the structure from right to left, e.g., -CH₂O- is intended to also recite -OCH₂-; -NHS(O)₂- is also intended to represent. -S(O)₂HN-, etc.

The term "alkyl," by itself or as part of another substituent, means, unless otherwise stated, a straight or branched chain or combination thereof and can include di-and multivalent radicals, having the number of carbon atoms designated (*i.e*. C₁-C₁₀ means one to ten carbons). Examples of saturated hydrocarbon radicals include groups such as methyl, ethyl, n-propyl, isopropyl, n-butyl, t-butyl, isobutyl, sec-butyl, homologs and isomers of, for example, n-pentyl, n-hexyl, n-heptyl and n-octyl. Alkyl groups which are limited to hydrocarbon groups are termed "homoalkyl".

The term "alkylene" by itself or as part of another substituent means a divalent radical derived from an alkane, as exemplified by CH₂CH₂CH₂CH₂-. Typically, an alkyl (or alkylene) group will have from 1 to 24 carbon atoms, with those groups having 10 or fewer carbon atoms being preferred in the present invention. A "lower alkyl" or "lower alkylene" is a shorter chain alkyl or alkylene group, generally having eight or fewer carbon atoms.

The terms "alkoxy," "alkylamino" and "alkylthio" (or thioalkoxy) are used in their conventional sense, and refer to those alkyl groups attached to the remainder of the molecule via an oxygen atom, an amino group, or a sulfur atom, respectively.

The term "heteroalkyl," by itself or in combination with another term, means, unless otherwise stated, a stable straight or branched chain, or combinations thereof, consisting of the stated number of carbon atoms and at least one heteroatom selected from the group consisting of O, N, Si and S, and wherein the nitrogen and sulfur atoms may optionally be oxidized and the nitrogen heteroatom may optionally be quaternized. The heteroatom(s) O, N and S and Si may be placed at any interior position of the heteroalkyl group or at the position at which the alkyl group is attached to the remainder of the molecule. Examples include -CH₂-CH₂-O-CH₃, -CH₂-CH₂-NH-CH₃, -CH₂-CH₂-N(CH₃)-CH₃, -CH₂-S-CH₂-CH₃, -CH₂-CH₂,-S(O)-CH₃, -CH₂-CH₂-S(O)₂-CH₃, -CH=CH-O-CH₃, -Si(CH₃)₃, -CH₂-CH=N-OCH₃, and - CH=CH-N(CH₃)-CH₃. Up to two heteroatoms may be consecutive, such as, for example, -CH₂-NH-OCH₃ and -CH₂-O-Si(CH₃)₃. Similarly, the term "heteroalkylene" by itself or as part of another substituent means a divalent radical derived from heteroalkyl, as exemplified CH₂-CH₂-S-CH₂-CH₂- and -CH₂-S-CH₂-CH₂-NH-CH₂-. For heteroalkylene groups, heteroatoms can also occupy either or both of the chain termini (*e.g*., alkyleneoxy, alkylenedioxy, alkyleneamino and alkylenediamino. Still further, for alkylene and heteroalkylene linking groups, no orientation of the linking group is implied by the direction in which the formula of the linking group is written. For example, the formula -C(O)₂R'- represents both -C(O)₂R'- and R'C(O)₂-.

The terms "cycloalkyl" and "heterocycloalkyl", by themselves or in combination with other terms, represent, unless otherwise stated, cyclic versions of "alkyl" and "heteroalkyl", respectively. Additionally, for heterocycloalkyl, a heteroatom can occupy the position at which the heterocycle is attached to the remainder of the molecule. Examples of cycloalkyl include cyclopentyl, cyclohexyl, 1-cyclohexenyl, 3-cyclohexenyl and cycloheptyl. Examples of heterocycloalkyl include 1 -(1,2,5,6-tetrahydropyridyl), 1-piperidinyl, 2-piperidinyl, 3-piperidinyl, 4-morpholinyl, 3-morpholinyl, tetrahydrofuran-2-yl, tetrahydrofuran-3-yl, tetrahydrothien-2-yl, tetrahydrothien-3-yl, 1 -piperazinyl, and 2-piperazinyl.

The terms "halo" or "halogen," by themselves or as part of another substituent, mean, unless otherwise stated, a fluorine, chlorine, bromine, or iodine atom. Additionally, terms such as "haloalkyl," are meant to include monohaloalkyl and polyhaloalkyl. For example, the term "halo(C₁-C₄)alkyl" is mean to include trifluoromethyl, 2,2,2-trifluoroethyl, 4-chlorobutyl and 3-bromopropyl.

The term "aryl" means, unless otherwise stated, a polyunsaturated, aromatic, hydrocarbon substituent which can be a single ring or multiple rings (preferably from 1 to 3 rings) which are fused together or linked covalently. The term "heteroaryl" refers to aryl groups (or rings) that contain from one to four heteroatoms selected from N, O, and S, wherein the nitrogen and sulfur atoms are optionally oxidized, and the nitrogen atom(s) are optionally quaternized. A heteroaryl group can be attached to the remainder of the molecule through a heteroatom. Examples of aryl and heteroaryl groups include phenyl, 1-naphthyl, 2-naphthyl, 4-biphenyl, 1-pyrrolyl, 2-pyrrolyl, 3-pyrrolyl, 3-pyrazolyl, 2-imidazolyl, 4-imidazolyl, pyrazinyl, 2-oxazolyl, 4-oxazolyl, 2-phenyl-4-oxazolyl, 5-oxazolyl, 3-isoxazolyl, 4-isoxazolyl, 5-isoxazolyl, 2-thiazolyl, 4-thiazolyl, 5-thiazolyl, 2-furyl, 3-furyl, 2-thienyl, 3-thienyl, 2-pyridyl, 3-pyridyl, 4-pyridyl, 2-pyrimidyl, 4-pyrimidyl, 5-benzothiazolyl, purinyl, 2-benzimidazolyl, 5-indolyl, 1-isoquinolyl, 5-isoquinolyl, 2-quinoxalinyl, 5-quinoxalinyl, 3-quinolyl, and 6-quinolyl. Substituents for each of the above noted aryl and heteroaryl ring systems are selected from the group of acceptable substituents described below.

For brevity, the term "aryl" when used in combination with other terms (*e.g*., aryloxy, arylthioxy, arylalkyl) includes aryl rings as defined above. Thus, the term "arylalkyl" is meant to include those radicals in which an aryl group is attached to an alkyl group (*e.g*., benzyl and phenethyl).

Each of the above terms (*e.g*., "alkyl," "heteroalkyl," "aryl" and "heteroaryl") include both substituted and unsubstituted forms of the indicated radical. Preferred substituents for each type of radical are provided below.

Substituents for the alkyl and heteroalkyl radicals (including those groups often referred to as alkylene, alkenyl, heteroalkylene, heteroalkenyl, alkynyl, cycloalkyl, heterocycloalkyl, cycloalkenyl, and heterocycloalkenyl) can be one or more of a variety of groups selected from -OR', =O, =NR', =N-OR', -NR'R", -SR', -halogen, -SiR'R"R"', -OC(O)R', -C(O)R', -CO₂R', -CONR'R", -OC(O)NR'R", -NR"C(O)R', -NR'-C(O)NR"R"', -NR"C(O)₂R', -NR-C(NR'R")=NR"', -S(O)R', -S(O)₂R', -S(O)₂NR'R", -NRSO₂R', -CN and -NO₂ in a number ranging from zero to (2m'+1), where m' is the total number of carbon atoms in such radical. R', R", R"' and R"" each preferably independently refer to hydrogen, substituted or unsubstituted heteroalkyl, substituted or unsubstituted aryl, e.g., aryl substituted with 1-3 halogens, substituted or unsubstituted alkyl, alkoxy or thioalkoxy groups, or arylalkyl groups. When a compound of the invention includes more than one R group, for example, each of the R groups is independently selected as are each R', R", R"' and R"" groups when more than one of these groups is present. When R' and R" are attached to the same nitrogen atom, they can be combined with the nitrogen atom to form a 5-, 6-, or 7-membered ring. For example, -NR'R" is meant to include 1-pyrrolidinyl and 4-morpholinyl. From the above discussion of substituents, one of skill in the art will understand that the term "alkyl" with alkyl group substituents is meant to include groups including carbon atoms bound to groups other than hydrogen groups, such as haloalkyl (*e.g*., -CF₃ and -CH₂CF₃) and acyl (*e.g*., -C(O)CH₃, -C(O)CF₃ and -C(O)CH₂OCH₃). These substituents are referred to herein generically as "alkyl group substituents."

Similar to the substituents described for the alkyl radical, substituents for the aryl and heteroaryl groups, referred to generically herein as "aryl group substituents," are varied and are selected from halogen, -OR', =O, =NR', =N-OR', -NR'R", - SR', -halogen, -SiR'R"R"', -OC(O)R', -C(O)R', -CO₂R', -CONR'R", -OC(O)NR'R", - NR^{"}C(O)R', -NR'-C(O)NR"R'", -NR"C(O)₂R', -NR-C(NR²R")=NR"', -S(O)R',-S(O)₂R', -S(O)₂NR'R", -NRSO₂R', -CN and -NO₂, -R', -N₃, -CH(Ph)₂, fluoro (C₁-C₄)alkoxy, and fluoro(C₁-C₄)alkyl, in a number ranging from zero to the total number of open valences on the aromatic ring system; and where R', R", R"' and R"" are preferably independently selected from hydrogen, (C₁-C₈)alkyl and heteroalkyl, unsubstituted aryl and heteroaryl, (unsubstituted aryl)-(C₁-C₄)alkyl, and (unsubstituted aryl)oxy-(C₁-C₄)alkyl. When a compound of the invention includes more than one R group, for example, each of the R groups is independently selected as are each R', R", R"' and R"" groups when more than one of these groups is present.

Two of the substituents on adjacent atoms of the aryl or heteroaryl ring may optionally be replaced with a substituent of the formula-T-C(O)-(CRR')_{q}-U-, wherein T and U are independently NR-, -O-, -CRR'- or a single bond, and q is an integer of from 0 to 3. Alternatively, two of the substituents on adjacent atoms of the aryl or heteroaryl ring may optionally be replaced with a substituent of the formula -A-(CH₂)ᵣ-B-, wherein A and B are independently -CRR'-, -O-, -NR-, -S-, -S(O)-, -S(O)₂-, -S(O)₂NR'- or a single bond, and r is an integer of from 1 to 4. One of the single bonds of the new ring so formed may optionally be replaced with a double bond. Alternatively, two of the substituents on adjacent atoms of the aryl or heteroaryl ring may optionally be replaced with a substituent of the formula -(CRR')ₛ-X-(CR"R"')_{d}-, where s and d are independently integers of from 0 to 3, and X is -O-, -NR'-, -S-, -S(O)-, -S(O)₂-, or - S(O)₂NR'-. The substituents R, R', R" and R"' are preferably independently selected from hydrogen or substituted or unsubstituted (C₁-C₆)alkyl.

As used herein, the term "heteroatom" is meant to include oxygen (O), nitrogen (N), sulfur (S) and silicon (Si).

The term "pharmaceutically acceptable salts" is meant to include salts of the active compounds which are prepared with relatively nontoxic acids or bases, depending on the particular substituents found on the compounds described herein. When compounds of the present invention contain relatively acidic functionalities, base addition salts can be obtained by contacting the neutral form of such compounds with a sufficient amount of the desired base, either neat or in a suitable inert solvent. Examples of pharmaceutically acceptable base addition salts include sodium, potassium, calcium, ammonium, organic amino, or magnesium salt, or a similar salt. When compounds of the present invention contain relatively basic functionalities, acid addition salts can be obtained by contacting the neutral form of such compounds with a sufficient amount of the desired acid, either neat or in a suitable inert solvent. Examples of pharmaceutically acceptable acid addition salts include those derived from inorganic acids like hydrochloric, hydrobromic, nitric, carbonic, monohydrogencarbonic, phosphoric, monohydrogenphosphoric, dihydrogenphosphoric, sulfuric, monohydrogensulfuric, hydriodic, or phosphorous acids, as well as the salts derived from relatively nontoxic organic acids like acetic, propionic, isobutyric, maleic, malonic, benzoic, succinic, suberic, fumaric, lactic, mandelic, phthalic, benzenesulfonic, p-tolylsulfonic, citric, tartaric and methanesulfonic. Also included are salts of amino acids such as arginate, and salts of organic acids like glucuronic or galactunoric acids (*see*, for example, Berge et al., "Pharmaceutical Salts", Journal of Pharmaceutical Science, 1977, 66, 1-19). Certain specific compounds of the present invention contain both basic and acidic functionalities that allow the compounds to be converted into either base or acid addition salts.

The neutral forms of the compounds are preferably regenerated by contacting the salt with a base or acid and isolating the parent compound in the conventional manner. The parent form of the compound differs from the various salt forms in certain physical properties, such as solubility in polar solvents, but otherwise the salts are equivalent to the parent form of the compound for the purposes of the present invention.

Certain compounds of the present invention can exist in unsolvated forms as well as solvated forms, including hydrated forms. In general, the solvated forms are equivalent to unsolvated forms and are encompassed within the scope of the present invention. Certain compounds of the present invention may exist in multiple crystalline or amorphous forms. In general, all physical forms are equivalent for the uses contemplated by the present invention and are intended to be within the scope of the present invention.

Certain compounds of the present invention possess asymmetric carbon atoms (optical centers) or double bonds; the racemates, diastereomers, geometric isomers and individual isomers are encompassed within the scope of the present invention.

The compounds of the present invention may also contain unnatural proportions of atomic isotopes at one or more of the atoms that constitute such compounds. For example, the compounds may be radiolabeled with radioactive isotopes, such as for example tritium (³H), iodine-125 (¹²⁵I) or carbon-14(¹⁴C). All isotopic variations of the compounds of the present invention, whether radioactive or not, are intended to be encompassed within the scope of the present invention.

### Introduction

The present invention provides compounds which, *inter alia,* are useful in the treatment of diseases through the modulation of potassium ion flux through voltage-dependent potassium channels. More particularly, the invention provides compounds and compositions that are useful in the treatment of central or peripheral nervous system disorders (e.g., migraine, ataxia, Parkinson's disease, bipolar disorders, trigeminal neuralgia, spasticity, mood disorders, brain tumors, psychotic disorders, myokymia, seizures, epilepsy, hearing and vision loss, Alzheimer's disease, age-related memory loss, learning deficiencies, anxiety and motor neuron diseases), and as neuroprotective agents (e.g., to prevent stroke and retinal degeneration). Compounds of the invention have use as agents for treating convulsive states, for example that following grand mal, petit mal, psychomotor epilepsy or focal seizure. The compounds of the invention are also useful in treating disease states such as gastroesophogeal reflux disorder and gastrointestinal hypomotility disorders.

Moreover, compounds of the invention are useful in the treatment of pain, for example, neuropathic pain, diabetic pain, inflammatory pain, cancer pain, migraine pain, and musculoskeletal pain. The compounds are also useful to treat conditions, which may themselves be the origin of pain, for example, inflammatory conditions, including arthritic conditions (*e.g*., rheumatoid arthritis, rheumatoid spondylitis, osteoarthritis and gouty arthritis) and non-articular inflammatory conditions (e.g., herniated, ruptured and prolapsed disc syndrome, bursitis, tendonitis, tenosynovitis, fibromyalgia syndrome, and other conditions associated with ligamentous sprain and regional musculoskeletal strain). Particularly preferred compounds of the invention are less ulcerogenic than other antiinflammatory agents (e.g., ibuprofen, naproxen and aspirin). Furthermore, the compounds of the invention are useful in treating conditions and pain associated with abnormally raised skeletal muscle tone.

The compounds of the invention are also of use in treating anxiety (e.g. anxiety disorders). Anxiety disorders are defined in the Diagnostic and Statistical Manual of Mental Disorders (Third Edition-revised 1987, published by the American Psychiatric Association, Washington, D.C., see, pages 235 to 253), as psychiatric conditions having symptoms of anxiety and avoidance behavior as characteristic features. Included amongst such disorders are generalized anxiety disorder, simple phobia and panic disorder.

Anxiety also occurs as a symptom associated with other psychiatric disorders, for example, obsessive compulsive disorder, post-traumatic stress disorder, schizophrenia, mood disorders and major depressive disorders, and with organic clinical conditions including Parkinson's disease, multiple sclerosis, and other physically incapacitating disorders.

In view of the above-noted discovery, the present invention provides compounds, compositions, and methods (provided that it is not a method of treatment of the human or animal body by therapy) for increasing ion flux in voltage-dependent potassium channels, particularly those channels responsible for the M-current. As used herein, the term "M-current," "channels responsible for the M-current" and the like, refers to a slowly activating, non-inactivating, slowly deactivating voltage-gated K⁺ channel. M-current is active at voltages close to the threshold for action potential generation in a wide variety of neuronal cells, and thus, is an important regulator of neuronal excitability.

Recently, members of the voltage-dependent potassium channel family were shown to be directly involved in diseases of the central or peripheral nervous system. The quinazilinones provided herein are now shown to act as potassium channel modulators, particularly openers, for KCNQ2 and KCNQ3, KCNQ4, and KCNQ5 as well as the heteromultimer channels such as KCNQ2/3, KCNQ3/5 or the M-current.

### Description of the Embodiments

### I. MODULATORS OF VOLTAGE-DEPENDENT POTASSIUM CHANNELS

The present invention provides a novel class of potassium ion channel modulators, particularly effective at modulating KCNQ, according to Formula I:

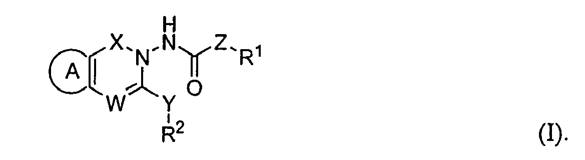

In Formula I, the symbol A represents a five- or six-membered substituted or unsubstituted aryl, five- and six-membered substituted or unsubstituted heteroaryl, substituted or unsubstituted C₄-C₈ cycloalkyl, and substituted or unsubstituted 5-8 membered heterocyclyl ring system. X represents CO.

Z represents -CH₂-, -CHF-, -CF₂-, -CH=CH- or -N(R⁴)(CR^{4a}R^{4b})ₛ-, in which R⁴represents H or a substituted or unsubstituted C₁-C₅ alkyl group. The symbols R^{4a} and R^{4b} represent groups that are independently selected from H, substituted or unsubstituted aryl, substituted or unsubstituted heteroaryl, substituted or unsubstituted C₃-C₈ cycloalkyl, substituted or unsubstituted 5-7 membered heterocyclyl, or substituted or unsubstituted C₁-C₈ alkyl. R¹ is substituted or unsubstituted aryl, substituted or unsubstituted heteroaryl, substituted or unsubstituted C₃-C₈ cycloalkyl, substituted or unsubstituted 5-7 membered heterocyclyl, or substituted or unsubstituted C₁-C₈ alkyl. The index "s" is an integer from 1 to 3. Moreover, when "s" is greater than 1, each R^{4a} and R^{4b} are independently selected.

W is N.

The symbol Y represents S(O)ₙ, in which the index "n" is an integer from 0-2. R² is CF₃, substituted or unsubstituted C₁-C₈ alkyl, substituted or unsubstituted aryl, substituted or unsubstituted heteroaryl, substituted or unsubstituted C₃-C₈ cycloalkyl, or substituted or unsubstituted 3-7-membered heterocyclyl.

In an exemplary embodiment according to Formula I, the invention provides a compound in which, when A is phenyl, Z is -CH₂- or -NH-, and R¹ is phenyl, substituted phenyl or heteroaryl, then R² is other than a benzyl, substituted benzyl, alkylheteroaryl, alkylheterocyclyl or cyanomethyl group.

In another exemplary embodiment, A is substituted or unsubstituted aryl, e.g., substituted or unsubstituted phenyl, or and substituted or unsubstituted heteroaryl. When A is substituted phenyl, it is generally substituted one, two or more times with an "aryl substituent" as defined herein. Exemplary "aryl substituents" include halogen, nitrile, substituted or unsubstituted C₁-C₄ alkyl, SCF₃, trifluoromethyl and trifluoromethoxy.

As discussed above, in selected compounds of the invention, R¹ is a substituted or unsubstituted aryl, e.g., substituted or unsubstituted phenyl, or substituted or unsubstituted heteroaryl. When R¹ is substituted phenyl, it is generally substituted with one, two or more aryl group substituents, as defined herein, such as substituted halogen, CF₃ and OCF₃.

In a selected embodiment, R² is a substituted or unsubstituted C₁-C₆ saturated acyclic alkyl group. In yet another embodiment, R² is a C₁-C₄ saturated acyclic alkyl group.

In a further exemplary embodiment, the invention provides compounds having the formula: 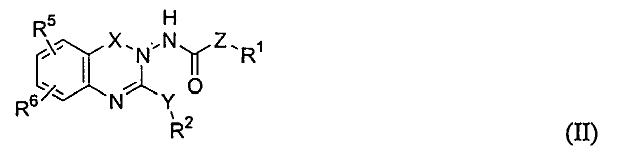 in which the substituents are generally identical to those discussed in the context of Formula I. Additionally, R⁵ and R⁶ are independently selected aryl group substituents, as defined herein selected from H, halo, CF₃, CF₃O, NO₂, CN, S(O)ₘR⁷ COOR⁸, CONR⁹R¹⁰, SO₂NR¹¹R¹², S(O)ₘCF₃, substituted or unsubstituted C₁-C₆ alkyl, or substituted or unsubstituted C₃-C₇ cycloalkyl. The symbols R⁷ and R⁸ represent groups that are independently substituted or unsubstituted C₁-C₅ alkyl, and substituted or unsubstituted C₃-C₇ cycloalkyl. The index "m" is an integer from 0 to 2. R⁹, R¹⁰, R¹¹ and R¹² are independently H, substituted or unsubstituted C₁-C₅ alkyl, or substituted or unsubstituted C₃-C₇ cycloalkyl. R⁹ and R¹⁰ or R¹¹ and R¹², together with the nitrogen atom to which they are attached, are optionally joined to form a 5- to 7-membered ring.
In yet another exemplary embodiment, the invention provides compounds according to Formula III: 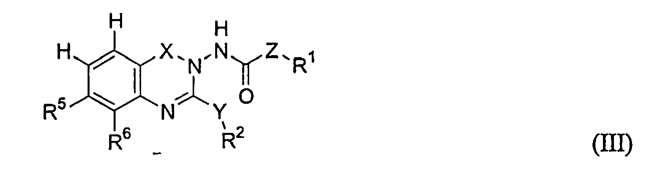 in which the substituents are generally identical to those discussed in the context of Formulae I and II.

### Preparation of Potassium Channel Modulators

Compounds of the present invention can be prepared using readily available starting materials or known intermediates. However, some intermediates produced during the synthesis are themselves novel, as indicated. Briefly, the synthesis of the compounds of the invention is performed starting with an appropriately substituted ortho-nitro or - amino carboxylic acid. The acid has the general formula:

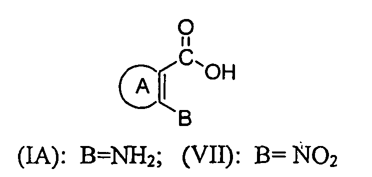

By reaction involving a phenylacetylhydrazide (Z¹ = CH₂) (IIIA) 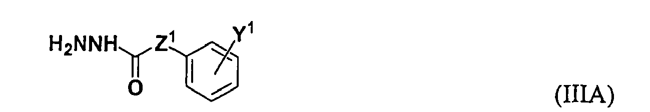 or an analog that, depending on the identity of Z¹ in the desired product, will contain an appropriate group in place of the methylene group (and Y¹ represents hydrogen or one or more various "aryl group substituents" as defined herein), there is formed an intermediate (IV) or (VIII):

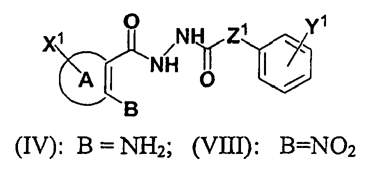

Nitro groups in intermediates (VHI) are reduced to amino groups to form the corresponding amino intermediates (IV) (and X¹ represents hydrogen or one or more various "aryl group substituents" as defined herein).

The amino compounds (IV) are reacted with a suitable reactant, such as O-ethylxanthic acid, potassium salt, forming intermediate (V) 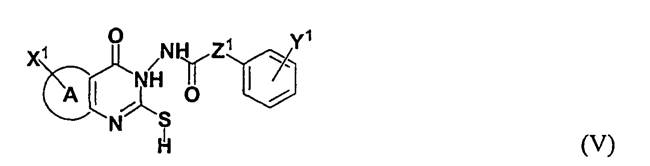 which is then alkylated, yielding the desired product, a compound of Formula I in which R² is -S-.

Compounds in which R² is S(O) or S(O)₂ are prepared by specific oxidation of these compounds using, for example, m-chloroperbenzoic acid or ozone as oxidizing agents.

Thus, to produce compounds in which the pyrimidine-containing ring is a quinazoline ring, an ortho-amino or ortho-nitrobenzoic acid is converted by use of a phenylacetylhydrazide or analogous compound IIIA to intermediate (IV) or (VIII)

Intermediates (IV), (V), and (VIII) above are novel and form an aspect of this invention.

The process is further illustrated by Scheme 1

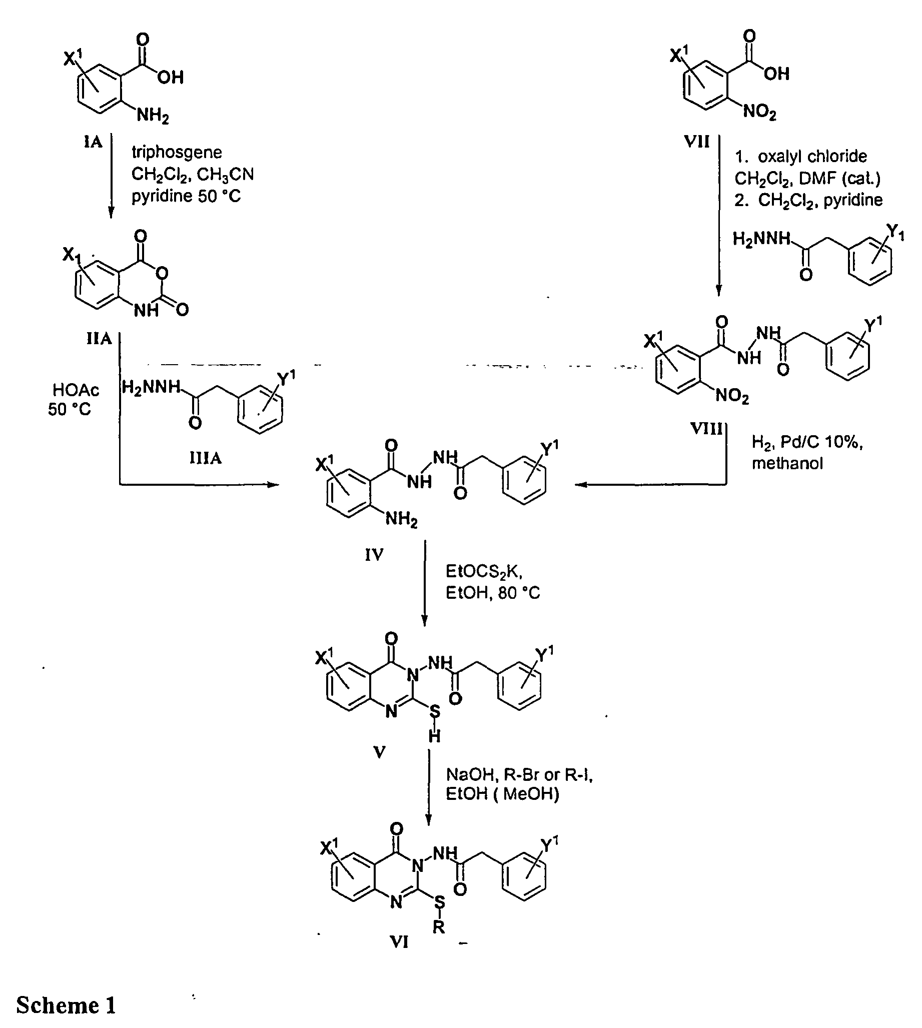

### II. ASSAYS FOR MODULATORS OF KCNQ CHANNELS

Assays for determining the ability of a compound of the invention to modulate, e.g., open, a potassium ion channel are generally known in the art. One of skill in the art is able to determine an appropriate assay for investigating the activity of a selected compound of the invention towards a particular ion channel. For simplicity, portions of the following discussion focuses on KCNQ2 as a representative example, however, the discussion is equally applicable to other KCNQ potassium ion channels.

KCNQ monomers as well as KCNQ alleles and polymorphic variants are subunits of potassium channels. The activity of a potassium channel comprising KCNQ subunits can be assessed using a variety of *in vitro* and *in vivo* assays, *e.g*., measuring current, measuring membrane potential, measuring ion flux, *e.g*., potassium or rubidium, measuring potassium concentration, measuring second messengers and transcription levels, using potassium-dependent yeast growth assays, and using *e.g*., voltage-sensitive dyes, radioactive tracers, and patch-clamp electrophysiology.

Furthermore, such assays can be used to test for inhibitors and activators of channels comprising KCNQ. Such modulators of a potassium channel are useful for treating various disorders involving potassium channels, including central and peripheral nervous system disorders (*e.g*., migraine, ataxia, Parkinson's disease, bipolar disorders, trigeminal neuralgia, spasticity, mood disorders, brain tumors, psychotic disorders, myokymia, seizures, epilepsy, hearing and vision loss, Alzheimer's disease, age-related memory loss, learning deficiencies, anxiety and motor neuron diseases, and can also be used as neuroprotective agents (*e.g*., to prevent stroke). Such modulators are also useful for investigation of the channel diversity provided by KCNQ and the regulation/modulation of potassium channel activity provided by KCNQ.

Modulators of the potassium channels are tested using biologically active KCNQ, either recombinant or naturally occurring, or by using native cells, like cells from the nervous system expressing the M-current. KCNQ can be isolated, co-expressed or expressed in a cell, or expressed in a membrane derived from a cell. In such assays, KCNQ2 is expressed alone to form a homomeric potassium channel or is co-expressed with a second subunit (*e.g*., another KCNQ family member, preferably KCNQ3) so as to form a heteromeric potassium channel. Modulation is tested using one of the *in vitro* or *in vivo* assays described above. Samples or assays that are treated with a potential potassium channel inhibitor or activator are compared to control samples without the test compound, to examine the extent of modulation. Control samples (untreated with activators or inhibitors) are assigned a relative potassium channel activity value of 100. Activation of channels comprising KCNQ2 is achieved when the potassium channel activity value relative to the control is 130%, more preferably 150%, more preferably 170% higher. Compounds that increase the flux of ions will cause a detectable increase in the ion current density by increasing the probability of a channel comprising KCNQ2 being open, by decreasing the probability of it being closed, by increasing conductance through the channel, and increasing the number or expression of channels.

The activity of the compounds of the invention can also be represented by EC50. Preferred compounds of the invention have an EC50 in a potassium ion channel assay of from about 1 nM to about 10 µM, preferably from about 1 nM to about 1 µM, and more preferably from about 1 nM to about 500 nM.

Changes in ion flux may be assessed by determining changes in polarization (*i.e*., electrical potential) of the cell or membrane expressing an exemplary potassium channel such as KCNQ2, KCNQ2/3 or the M-current. A preferred means to determine changes in cellular polarization is by measuring changes in current or voltage with the voltage-clamp and patch-clamp techniques, using the "cell-attached" mode, the "inside-out" mode, the "outside-out" mode, the "perforated cell" mode, the "one or two electrode" mode, or the "whole cell" mode (*see, e.g.,* Ackerman et al., New Engl. J. Med. 336: 1575-1595 (1997)). Whole cell currents are conveniently determined using the standard methodology (*see, e.g.,* Hamil et al., Pflugers. Archiv. 391: 85 (1981). Other known assays include: radiolabeled rubidium flux assays and fluorescence assays using voltage-sensitive dyes (*see, e.g.,* Vestergarrd-Bogind et al., J. Membrane Biol. 88: 67-75 (1988); Daniel et al., J. Pharmacol. Meth. 25: 185-193 (1991); Holevinsky et al., J. Membrane Biology 137: 59-70 (1994)). Assays for compounds capable of inhibiting or increasing potassium flux through the channel proteins comprising KCNQ2 or heteromultimers of KCNQ subunits can be performed by application of the compounds to a bath solution in contact with and comprising cells having a channel of the present invention (*see, e.g.,* Blatz et al., Nature 323: 718-720 (1986); Park, J. Physiol. 481: 555-570 (1994)). Generally, the compounds to be tested are present in the range from about 1 pM to about 100 mM, preferably from about 1 pM to about 1 µM.

The effects of the test compounds upon the function of the channels can be measured by changes in the electrical currents or ionic flux or by the consequences of changes in currents and flux. Changes in electrical current or ionic flux are measured by either increases or decreases in flux of ions such as potassium or rubidium ions. The cations can be measured in a variety of standard ways. They can be measured directly by concentration changes of the ions or indirectly by membrane potential or by radio-labeling of the ions. Consequences of the test compound on ion flux can be quite varied. Accordingly, any suitable physiological change can be used to assess the influence of a test compound on the channels of this invention. The effects of a test compound can be measured by a toxin-binding assay. When the functional consequences are determined using intact cells or animals, one can also measure a variety of effects such as transmitter release (*e.g*., dopamine), hormone release (*e.g*., insulin), transcriptional changes to both known and uncharacterized genetic markers (*e.g*., northern blots), cell volume changes *(e.g.,* in red blood cells), immunoresponses (*e.g*., T cell activation), changes in cell metabolism such as cell growth or pH changes, and changes in intracellular second messengers such as Ca²⁺, or cyclic nucleotides.

KCNQ2 orthologs will generally confer substantially similar properties on a channel comprising such KCNQ2, as described above. In a preferred embodiment, the cell placed in contact with a compound that is suspected to be a KCNQ2 homolog is assayed for increasing or decreasing ion flux in a eukaryotic cell, *e.g*., an oocyte of *Xenopus (e.g., Xenopus laevis)* or a mammalian cell such as a CHO or HeLa cell. Channels that are affected by compounds in ways similar to KCNQ2 are considered homologs or orthologs of KCNQ2.

### III. PHARMACEUTICAL COMPOSITIONS OF POTASSIUM CHANNEL MODULATORS

In another aspect, the present invention provides pharmaceutical compositions comprising a pharmaceutically acceptable excipient and a compound of Formula I provided above.

### Formulation of the Compounds (Compositions)

The compounds of the present invention can be prepared and administered in a wide variety of oral, parenteral and topical dosage forms. Thus, the compounds of the present invention can be administered by injection, that is, intravenously, intramuscularly, intracutaneously, subcutaneously, intraduodenally, or intraperitoneally. Also, the compounds described herein can be administered by inhalation, for example, intranasally. Additionally, the compounds of the present invention can be administered transdermally. Accordingly, the present invention also provides pharmaceutical compositions comprising a pharmaceutically acceptable carrier or excipient and either a compound of Formula I, or a pharmaceutically acceptable salt thereof.

For preparing pharmaceutical compositions from the compounds of the present invention, pharmaceutically acceptable carriers can be either solid or liquid. Solid form preparations include powders, tablets, pills, capsules, cachets, suppositories, and dispersible granules. A solid carrier can be one or more substances, which may also act as diluents, flavoring agents, binders, preservatives, tablet disintegrating agents, or an encapsulating material.

In powders, the carrier is a finely divided solid, which is in a mixture with the finely divided active component. In tablets, the active component is mixed with the carrier having the necessary binding properties in suitable proportions and compacted in the shape and size desired.

The powders and tablets preferably contain from 5% or 10% to 70% of the active compound. Suitable carriers are magnesium carbonate, magnesium stearate, talc, sugar, lactose, pectin, dextrin, starch, gelatin, tragacanth, methylcellulose, sodium carboxymethylcellulose, a low melting wax, and cocoa butter. The term "preparation" is intended to include the formulation of the active compound with encapsulating material as a carrier providing a capsule in which the active component with or without other carriers, is surrounded by a carrier, which is thus in association with it. Similarly, cachets and lozenges are included. Tablets, powders, capsules, pills, cachets, and lozenges can be used as solid dosage forms suitable for oral administration.

For preparing suppositories, a low melting wax, such as a mixture of fatty acid glycerides or cocoa butter, is first melted and the active component is dispersed homogeneously therein, as by stirring. The molten homogeneous mixture is then poured into convenient sized molds, allowed to cool, and thereby to solidify.

Liquid form preparations include solutions, suspensions, and emulsions, for example, water or water/propylene glycol solutions. For parenteral injection, liquid preparations can be formulated in solution in aqueous polyethylene glycol solution.

Aqueous solutions suitable for oral use can be prepared by dissolving the active component in water and adding suitable colorants, flavors, stabilizers, and thickening agents as desired. Aqueous suspensions suitable for oral use can be made by dispersing the finely divided active component in water with viscous material, such as natural or synthetic gums, resins, methylcellulose, sodium carboxymethylcellulose, and other well-known suspending agents.

Also included are solid form preparations, which are intended to be converted, shortly before use, to liquid form preparations for oral administration. Such liquid forms include solutions, suspensions, and emulsions. These preparations may contain, in addition to the active component, colorants, flavors, stabilizers, buffers, artificial and natural sweeteners, dispersants, thickeners, and solubilizing agents.

The pharmaceutical preparation is preferably in unit dosage form. In such form the preparation is subdivided into unit doses containing appropriate quantities of the active component. The unit dosage form can be a packaged preparation, the package containing discrete quantities of preparation, such as packeted tablets, capsules, and powders in vials or ampoules. Also, the unit dosage form can be a capsule, tablet, cachet, or lozenge itself, or it can be the appropriate number of any of these in packaged form.

The quantity of active component in a unit dose preparation may be varied or adjusted from 0.1 mg to 10000 mg, more typically 1.0 mg to 1000 mg, most typically 10 mg to 500 mg, according to the particular application and the potency of the active component. The composition can, if desired, also contain other compatible therapeutic agents.

### Effective Dosages

Pharmaceutical compositions provided by the present invention include compositions wherein the active ingredient is contained in a therapeutically effective amount, *i.e*., in an amount effective to achieve its intended purpose. The actual amount effective for a particular application will depend, *inter alia,* on the condition being treated. For example, when administered to treat pain or anxiety, such compositions will contain an amount of active ingredient effective to achieve a clinically relevant degree of reduction in the condition being treated. Similarly, when the pharmaceutical composition is used to treat or prevent a central or peripheral nervous system disorder, e.g., Parkinson's disease a therapeutically effective amount will reduce one or more symptoms characteristic of the diseases (e.g., tremors) to below a predetermined pressure threshold. Determination of a therapeutically effective amount of a compound of the invention is well within the capabilities of those skilled in the art, especially in light of the detailed disclosure herein.

For any compound described herein, the therapeutically effective amount can be initially determined from cell culture assays. Target plasma concentrations will be those concentrations of active compound(s) that are capable of modulating, e.g., activating or opening the KCNQ channel. In preferred embodiments, the KCNQ channel activity is altered by at least 30%. Target plasma concentrations of active compound(s) that are capable of inducing at least about 50%, 70%, or even 90% or higher alteration of the KCNQ channel potassium flux are presently preferred. The percentage of alteration of the KCNQ channel in the patient can be monitored to assess the appropriateness of the plasma drug concentration achieved, and the dosage can be adjusted upwards or downwards to achieve the desired percentage of alteration.

As is well known in the art, therapeutically effective amounts for use in humans can also be determined from animal models. For example, a dose for humans can be formulated to achieve a circulating concentration that has been found to be effective in animals. A particularly useful animal model for predicting anticonvulsant dosages is the maximal electroshock assay (Fischer RS, Brain Res. Rev. 14: 245-278 (1989)). The dosage in humans can be adjusted by monitoring KCNQ channel activation and adjusting the dosage upwards or downwards, as described above.

A therapeutically effective dose can also be determined from human data for compounds which are known to exhibit similar pharmacological activities, such as retigabine (Rudnfeldt et al., Neuroscience Lett. 282: 73-76 (2000)).

Adjusting the dose to achieve maximal efficacy in humans based on the methods described above and other methods as are well-known in the art is well-within the capabilities of the ordinarily skilled artisan.

By way of example, when a compound of the invention is used in the prophylaxis and/or treatment of an exemplary disease such as epilepsy, a circulating concentration of administered compound of about 0.001 µM to 20 µM is considered to be effective, with about 0.01 µM to 5 µM being preferred.

Patient doses for oral administration of the compounds described herein, which is the preferred mode of administration for prophylaxis and for treatment of an exemplary disease such as epilepsy, typically range from about 1 mg/day to about 10,000 mg/day, more typically from about 10 mg/day to about 1,000 mg/day, and most typically from about 1 mg/day to about 500 mg/day. Stated in terms of patient body weight, typical dosages range from about 0.01 to about 150 mg/kg/day, more typically from about 0.1 to about 15 mg/kg/day, and most typically from about 0.5 to about 10 mg/kg/day.

For other modes of administration, dosage amount and interval can be adjusted individually to provide plasma levels of the-administered compound effective for the particular clinical indication being treated. For example, if acute epileptic seizures are the most dominant clinical manifestation, in one embodiment, a compound according to the invention can be administered in relatively high concentrations multiple times per day. Alternatively, if the patient exhibits only periodic epileptic seizures on an infrequent, periodic or irregular basis, in one embodiment, it may be more desirable to administer a compound of the invention at minimal effective concentrations and to use a less frequent administration regimen. This will provide a therapeutic regimen that is commensurate with the severity of the individual's disease.

Utilizing the teachings provided herein, an effective prophylactic or therapeutic treatment regimen can be planned which does not cause substantial toxicity and yet is entirely effective to treat the clinical symptoms demonstrated by the particular patient. This planning should involve the careful choice of active compound by considering factors such as compound potency, relative bioavailability, patient body weight, presence and severity of adverse side effects, preferred mode of administration and the toxicity profile of the selected agent.

### Compound Toxicity

The ratio between toxicity and therapeutic effect for a particular compound is its therapeutic index and can be expressed as the ratio between LD₅₀ (the amount of compound lethal in 50% of the population) and ED₅₀ (the amount of compound effective in 50% of the population). Compounds that exhibit high therapeutic indices are preferred. Therapeutic index data obtained from cell culture assays and/or animal studies can be used in formulating a range of dosages for use in humans. The dosage of such compounds preferably lies within a range of plasma concentrations that include the ED₅₀ with little or no toxicity. The dosage may vary within this range depending upon the dosage form employed and the route of administration utilized. See, *e.g*. Fingl et al., In: THE PHARMACOLOGICAL BASIS OF THERAPEUTICS, Ch.1, p.1, 1975. The exact formulation, route of administration and dosage can be chosen by the individual physician in view of the patient's condition and the particular method in which the compound is used.

### IV. METHODS FOR INCREASING ION FLOW IN VOLTAGE-DEPENDENT POTASSIUM CHANNELS

In yet another aspect, the present invention provides methods for increasing ion flow through voltage dependent potassium channels in a cell provided that they are not methods of treatment of the human or animal body by therapy. The method includes contacting a cell containing the target ion channels with an amount of a compound of the invention sufficient to enhancer the activity of a potassium channel.

The methods provided in this aspect of the invention are useful for the diagnosis of conditions that can be treated by modulating ion flux through voltage-dependent potassium channels, or for determining if a patient will be responsive to therapeutic agents, which act by opening potassium channels. In particular, a patient's cell sample can be obtained and contacted with a compound of the invention and the ion flux can be measured relative to a cell's ion flux in the absence of a compound of the invention. An increase in ion flux will typically indicate that the patient will be responsive to a therapeutic regimen of ion channel openers.

### V. METHODS FOR TREATING CONDITIONS MEDIATED BY VOLTAGE-DEPENDENT POTASSIUM CHANNELS

In still another aspect, the present invention provides the use of an effective amount of a compound having the formula provided above, for the manufacture of a medicament for the treatment of a central or peripheral nervous system disorder or condition through modulation of a voltage-dependent potassium channel.

The compounds provided herein are useful as potassium channel modulators and find therapeutic utility via modulation of voltage-dependent potassium channels in the treatment of diseases or conditions. The potassium channels targets for the compounds of the invention are described herein as voltage-dependent potassium channels such as the KCNQ potassium channels. As noted above, these channels may include homomultimers and heteromultimers of KCNQ2, KCNQ3, KCNQ4, and KCNQ5. A heteromultimer of two proteins, *e.g*., KCNQ2 and KCNQ3 is referred to as, for example, KCNQ2/3, KCNQ3/5, etc. The conditions that can be treated with the compounds and compositions of the present invention include central or peripheral nervous system disorders (*e.g*., migraine, ataxia, Parkinson's disease, bipolar disorders, trigeminal neuralgia, spasticity, mood disorders, brain tumors, psychotic disorders, myokymia, seizures, epilepsy, hearing and vision loss, Alzheimer's disease, age-related memory loss, learning deficiencies, anxiety, and motor neuron diseases). The compounds and compositions of the present invention may also serve as neuroprotective agents (*e.g*., to prevent stroke, or retinal degeneration). In a preferred embodiment, the condition or disorder to be treated is epilepsy or seizures. In another preferred embodiment, the condition or disorder is hearing loss.

In therapeutic use for the treatment of epilepsy or other neurological conditions, the compounds utilized in the invention are administered at the initial dosage of about 0.001 mg/kg to about 1000 mg/kg daily. A daily dose range of about 0.1 mg/kg to about 100 mg/kg is more typical. The dosages, however, may be varied depending upon the requirements of the patient, the severity of the condition being treated, and the compound being employed. Determination of the proper dosage for a particular situation is within the skill of the practitioner. Generally, treatment is initiated with smaller dosages, which are less than the optimum dose of the compound. Thereafter, the dosage is increased by small increments until the optimum effect under circumstances is reached. For convenience, the total daily dosage may be divided and administered in portions during the day, if desired.

### EXAMPLES

The following examples are offered to illustrate the claimed invention.

In the examples below, unless otherwise stated, temperatures are given in degrees Celsius (°C); operations were carried out at room or ambient temperature (typically a range of from about 18-25 °C; evaporation of solvent was carried out using a rotary evaporator under reduced pressure (typically, 4.5-30 mmHg) with a bath temperature of up to 60 °C; the course of reactions was typically followed by TLC and reaction times are provided for illustration only; melting points are uncorrected; products exhibited satisfactory ¹H-NMR and/or microanalytical data; yields are provided for illustration only; and the following conventional abbreviations are also used: mp (melting point), L (liter(s)), mL (milliliters), mmol (millimoles), g (grams), mg (milligrams), min (minutes), and h (hours).

### EXAMPLES

In the description below, unless otherwise stated, temperatures are given in degrees Celsius (°C). The course of reactions was typically followed by TLC and reaction times are provided for illustration only. Melting points are uncorrected; products exhibited satisfactory ¹H-NMR and/or microanalytical data; yields are provided for illustration only; and the following conventional abbreviations are also used: mp (melting point), L (liter(s)), mL (milliliters), eq (equivalents), mmol (millimoles), g (grams), mg (milligrams), min (minutes), and h (hours).

Unless otherwise specified, all solvents (HPLC grade) and reagents were purchased from suppliers and used without further purification. Analytical thin layer chromatography (TLC) was performed on Whatman Inc. 60 silica gel plates (0.25 mm thickness). Compounds were visualized under UV lamp (254 nM) or by developing with KMnO₄/KOH, ninhydrin or Hanessian's solution. Flash chromatography was done using silica gel from Selectro Scientific (particle size 32-63). ¹H NMR, ¹⁹F NMR and ¹³C NMR spectra were recorded on a Varian 300 machine at 300 MHz, 282 MHz and 75.7 MHz, respectively. Melting points were recorded on an Electrothermal IA9100 apparatus and were uncorrected.

### EXAMPLE 1

### 1.1 General procedure for preparation of isatoic anhydrides (II)

Anhydrous-pyridine (2 eq) was added to a solution of 2-aminobenzoic acid derivative (IA) (1 eq) in dry methylene chloride and acetonitrile (1:1, 40 mL/g of 2-aminobenzoic acid) at room temperature. Solid triphosgene (1/3 eq) was then added in one portion and the resulting mixture was heated at 50 °C for 2 h. The resulting solid was collected by filtration and dried in vacuo. The crude isatoic anhydrides (IIA) were typically obtained in 50 - 80% yields. Though contaminated with some pyridinium hydrochloride, the anhydrides were used in the next step without further purification.

### 1.2 General procedure for the preparation of acylhydrazides (IV)

A mixture of isatoic anhydride (IIA, 1 eq) and appropriate phenylacetylhydrazide (IIIA, 1.1 eq) were heated in glacial AcOH (2 mL/mmol) at 50 °C for 2-6 h. The resulting solution was cooled and water was added while shaking. The white precipitate was collected by filtration, washed with water and dried *in vacuo* at 50 °C for 4 h. The desired products (IV) were obtained as white solids in high purity (typically >90%) and moderate yields (typically 45-60%).

### 1.3 Representative compounds

### 1.3a 2-Amino-4-fluoro-benzoic acid N'-phenylacetyl-hydrazide

Obtained as a white powder. ¹H NMR (300 MHz, d-6 DMSO) δ 3.05(2H, s), 6.30 (1H, dt, *J =* 8.5, 2.4 Hz), 6.45 (1H, dd, *J =* 12.0, 2.6 Hz), 6.72 (2H, brs), 7.18-7.34 (5H, m), 7.57 (1H, dd, *J* = 8.9, 6.8 Hz), 10.02 (2H, brs).

### 1.3b 2-Amino-4-fluoro-benzoic acid N'-[2-(3-fluoro-phenyl)-acetyl]-hydrazide.

Obtained as a white powder.¹H NMR (300 MHz, d-6 DMSO) δ 3.54(2H, s), 6.30 (1H, dt, *J* = 8.7, 2.6 Hz), 6.46 (1H, dd, *J* = 11.8, 2.4 Hz), 6.72 (2H, brs), 7.02-7.19 (3H, m), 7.34 (1H, dt; *J =* 14.4, 7.8 Hz), 7.57 (1H, dd, *J =* 8.7, 6.8 Hz), 10.05 (2H, brs).

### EXAMPLE 2

### 2.1 General procedure for the preparation of thioureas (V)

A mixture of an acylhydrazide (IV, 1 eq) and KSCSOEt (1.5-2 eq) in ethanol (10 mL/mmol) was heated at 70 °C overnight. The resulting yellow mixture was cooled and neutralized with 1N aqueous HC1. Water was added (10-20 mL/mmol) and the mixture was vortexed for 10 sec. The resulting white solid was collected by filtration, washed with water and dried in vacuo at 50 °C for 6-10 h. The desired product (V) was obtained as a white solid in high purity (typically >90%) and high yield (typically >80%).

### 2.2 Representative compounds

### 2.2a N-(5-Fluoro-2-mercapto-4-oxo-4H-quinazolin-3-yl)-2-phenyl-acetamide

¹H NMR (300 MHz, d-6 DMSO) δ 3.65 (2H, s), 7.09-7.17 (3H, m), 7.20 (1H, d, *J* = 8.4 Hz), 7.34-7.41 (2H, m), 7.76 (1H, dt, *J* = 8.2, 5.4 Hz), 11.11 (1H, brs), 13.21 (1H, brs).

### 2.2b N-(7-Fluoro-2-mercapto-4-oxo-4H-quinazolin-3-yl)-2-(3-fluoro-phenyl)-acetamide

¹H NMR (300 MHz, d-6 DMSO) δ 3.70 (2H, s), 7.02-7.24 (5H, m), 7.36 (1H, dt, *J* = 8.0, 7.6 Hz), 8.02 (1H, dd, *J* = 8.9, 5.9 Hz), 11.27 (1H, brs), 13.21 (1H, brs).

### 2.2c N-(7-Fluoro-2-mercapto-4-oxo-4H-quinazolin-3-yl)-2-phenyl-acetamide

¹H NMR (300 MHz, d-6 DMSO) δ 3.65 (2H, s), 7.12 (1H, dd, *J* = 9.7, 2.3 Hz), 7.17-7.39 (6H, m), 8.02 (1H, dd, *J* = 8.7, 8.9 Hz), 11.16 (1H, brs), 13.20 (1H, brs).

### EXAMPLE 3

### 3.1 General procedure for preparation of sulfides (VI)

Aqueous sodium hydroxide (3N, 1.1 eq) was added to the appropriate sulfide intermediate (V, 1 eq) in methanol or ethanol at room temperature. To the resulting homogeneous solution was added the appropriate alkylating agent (1.1 eq) in one portion. The reaction was then shaken at 20-60 °C. When the reaction was judged to be complete (by LCMS), water was added to the reaction mixture (75 mL/g of intermediate) and the pH was adjusted to 7 with 6N HCl. The alcoholic solvent was removed under reduced pressure and the resulting solids were collected by filtration, washed with water and dried in vacuo affording the desired final products (VI) in 40-90% yields.

### EXAMPLE 4

### 4.1 General procedure for preparation of nitrobenzoic-N'-phenylacetyl hydrazides (VIII)

The appropriate nitrobenzoic acid derivative (VII) (1 eq) was stirred in dry methylene chloride (100 mL/g of acid) at room temperature and to this was added two drops of N,N-dimethylformamide (DMF). Neat oxalyl chloride (2 eq) was then added to the mixture dropwise at such a rate as to control gas evolution. After stirring for 2 h, the volatiles were removed by rotary evaporation and the remaining material was redissolved in dry methylene chloride (100 mL/g of acid). Pyridine (2 eq) and the appropriate hydrazide derivative (IIIA) (1 eq) were added consecutively and the mixture was allowed to stir at room temperature until the reaction was judged to be complete by HPLC analysis, whereupon the reaction mixture was poured into water. The organic layer was removed and the water layer was extracted three times with ethyl acetate. The combined organic layers were dried (Na₂SO₄) and concentrated to provide the desired nitrobenzoic-N'-phenylacetyl hydrazides (VIII) in high purity (typically >95%) and yields ranging from 40 to 90%.

### EXAMPLE 5

### 5.1 General procedure for preparation of 2-aminobenzoic acid N'-phenylacetyl-hydrazides (IV) from nitrobenzoic-N'-phenylacetyl hydrazides (VIII)

1 atmosphere of hydrogen was applied to a mixture of nitrobenzoic-N'-phenylacetyl hydrazide (VIII) in methanol (100 mL/g of hydrazide) and 10% palladium on activated carbon (100 mg/g of hydrazide). The reaction mixture was stirred at room temperature for 1-10 h. The resulting mixture was filtered through celite/silica gel and concentrated under reduced pressure. The desired 2-aminobenzoic acid N'-phenylacetyl-hydrazides (IV) were, in general, used directly in the next step without any further purification. In those instances when the desired products were contaminated with significant impurities, the hydrazides were purified by silica gel chromatography using EtOAc/hexanes.

### EXAMPLE 6

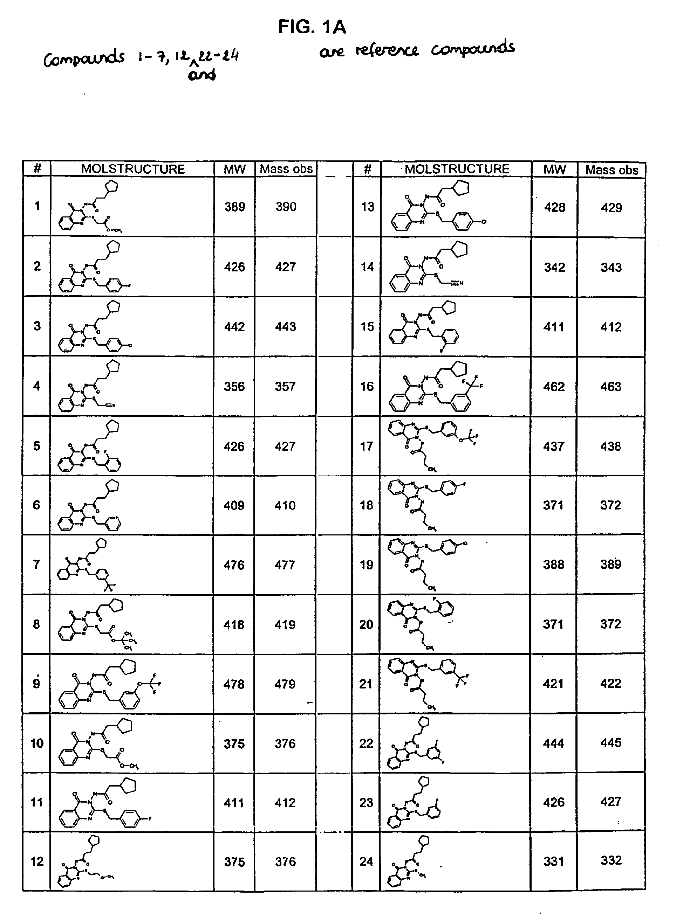

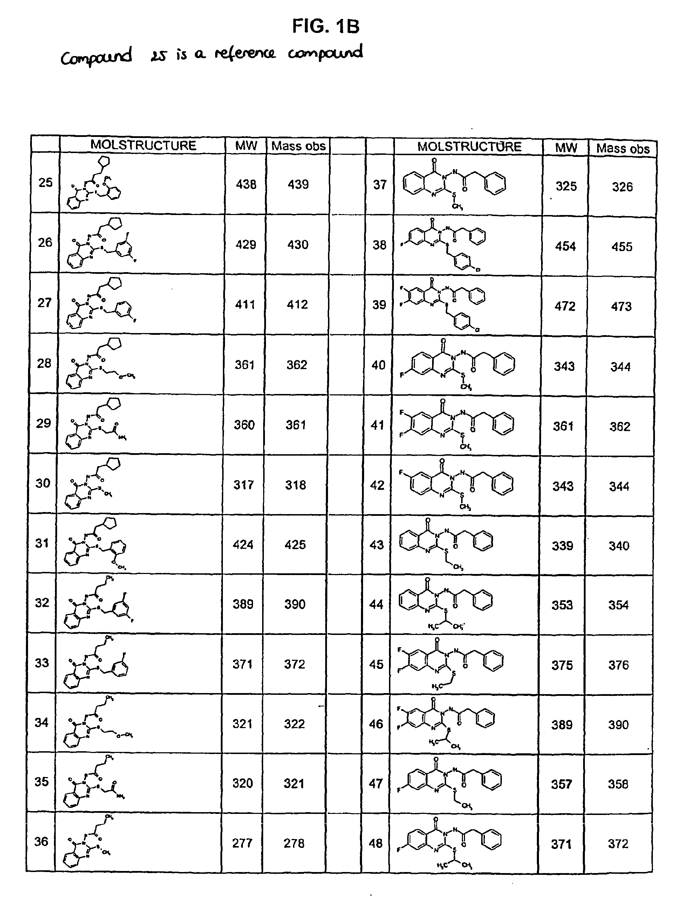

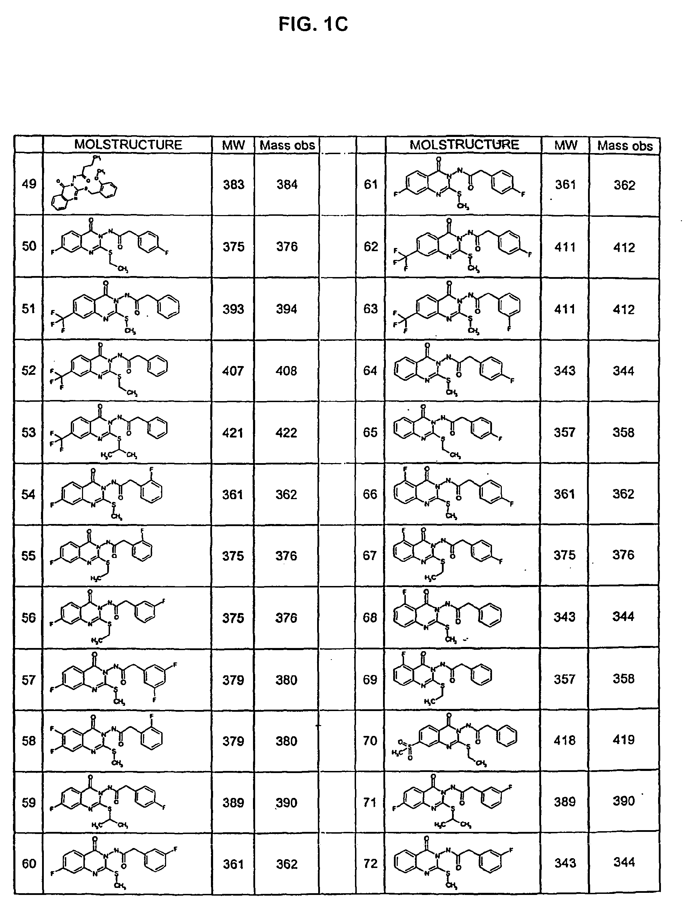

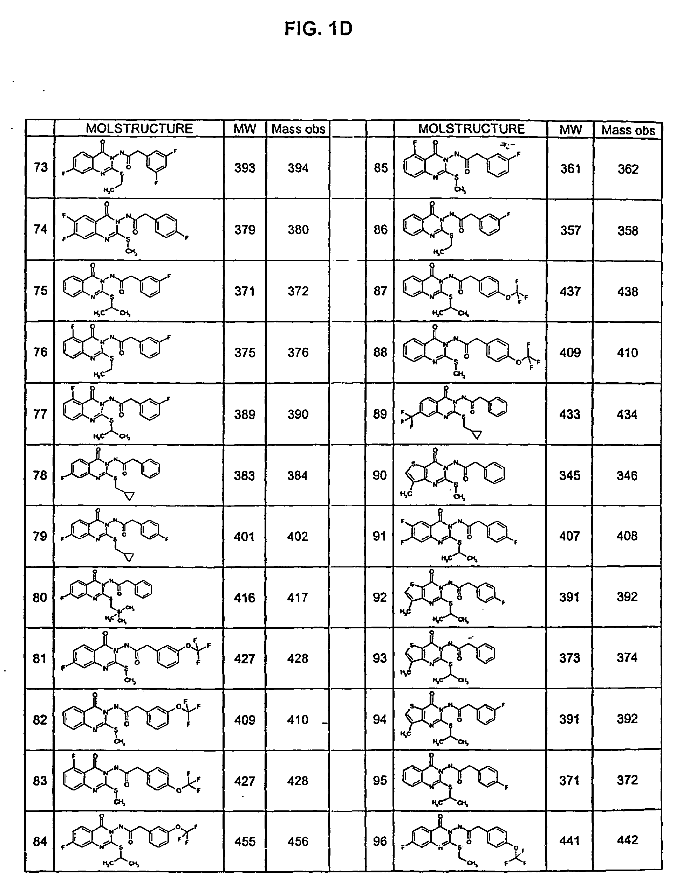

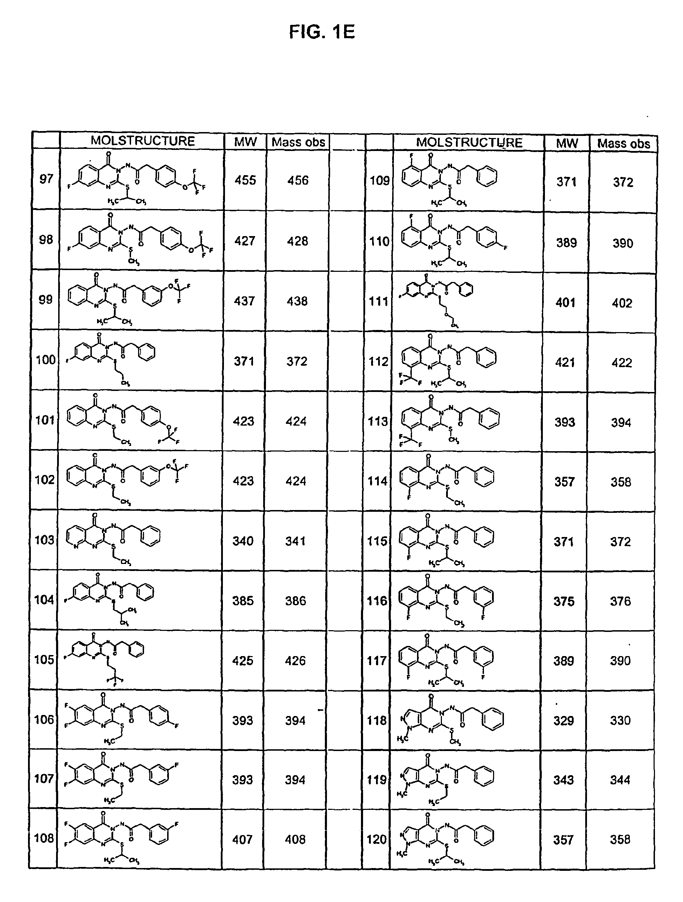

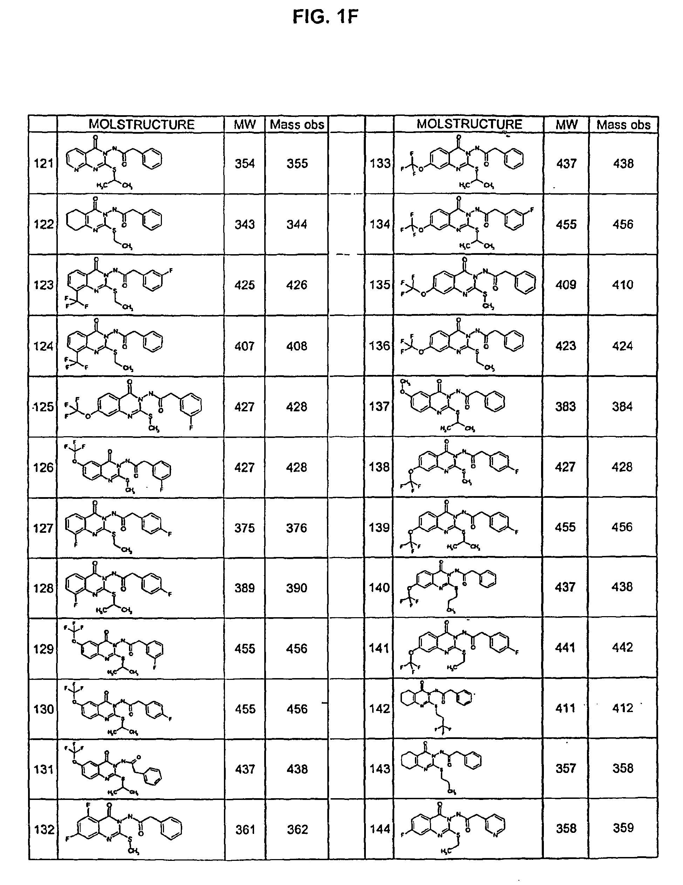

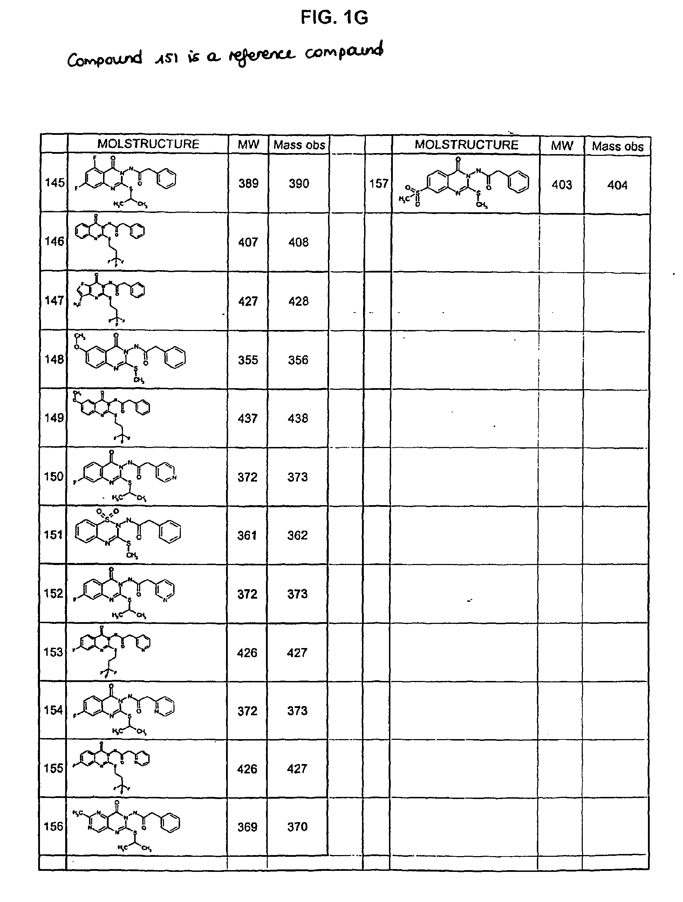

Example 6 sets forth the characterization of a number of representative compounds of the invention, using the general procedures above. The compounds were characterized using a combination of melting point, ¹H NMR and mass spectrometry. The results of the characterization are presented below. The structures for the compounds set forth below are provided in **FIG. 1**. Numbers in parentheses refer to compound numbers in **FIG. 1**.

### 6.1 2-(3-Fluoro-phenyl)-N-(2-methylsulfanyl-4-oxo-4H-quinazolin-3-yl)-acetamide (72)

Obtained as a white powder in 49% yield and 98% purity. ¹H NMR (300 MHz, CDCl₃) δ 2.51 (3H, s), 3.82 (2H, AB, *J* = 16.0, 2.4 Hz), 7.01 (1H, td, *J* = 8.4, 1.7 Hz), 7.14 (1H, d, *J* = 9.4Hz), 7.19 (1H, d, *J* = 7.7Hz), 7.56 (1H, d, *J* = 8.0Hz), 7.35 (2H, m), 7.70 (1H, td, *J* = 1.6Hz), 8.15 (2H, m).

### 6.2 N-(2-Ethylsulfanyl-4-oxo-4H-quinazolin-3-yl)-2-(4-fluoro-phenyl)-acetamide (65)

Obtained as a white powder in 35% yield and >95% purity. ¹H NMR (300 MHz, CDCl₃) δ 1.37 (3H, t, *J* = 7.3Hz), 3.12 (2H, m), 3.80 (2H, AB, *J* = 16.0, 3.4Hz), 7.20 (2H, m), 7.35 (3H, m), 7.53 (1H, d, *J* = 8.2Hz), 7.69 (1H, t, *J* = 8Hz), 8.13 (1H, d, *J* = 8.0Hz).

### 6.3 N-(7-Fluoro-2-methylsulfanyl-4-oxo-4H-quinazolin-3-yl)-2-phenyl-acetamide (40)

Obtained as a white solid in 61% yield and 98% purity. ¹H NMR (300 MHz, CDCl₃) δ 2.50 (3H, s), 3.84 (2H, dd, *J* = 18.1, 16.7Hz), 7.07 (1H, dt, *J* = 8.5, 2.4Hz), 7.20 (1H, dd, *J* = 9.7, 2.4Hz), 7.30-7.46 (4H, m), 7.77 (1H, s), 8.14 (1H, dd, *J* = 8.9, 6.1Hz).

### 6.4 N-(7-Fluoro-2-isopropylsulfanyl-4-oxo-4H-quinazolin-3-yl)-2-(3-fluorophenyl)-acetamide (71)

Obtained as a tan solid in 27% yield and 97% purity. ¹H NMR (300 MHz, CDCl₃) δ 1.35 (6H, dd, *J* = 12.6, 6.8Hz), 3.55 (1H, d, *J* = 14.6Hz), 3.70 (1H, d, *J* = 15.0Hz), 3.82 (1H, pentet, *J* = 7.1Hz), 6.75 (1H, t, *J* = 6.4Hz), 6.89 (1H, t, *J* = 8.7Hz), 6.96-7.08 (2H, m), 7.18 (1R, q, *J* = 7.1Hz), 7.81 (1H, t, *J* = 6.1Hz).

### 6.5 N-(2-Ethylsulfanyl-7-fluoro-4-oxo-4H-quinazolin-3-yl)-2-(4-fluoro-phenyl)-acetamide (50)

Obtained as a white solid in 86% yield and 97% purity.. ¹H NMR (300 MHz, CDCl₃) δ 1.37 (3H, t, *J*= 7.5Hz), 3.01-3.19 (2H, m), 3.79 (2H, s), 7.03-7.12 (3H, m), 7.19 (1H, dd, *J*= 9.7, 2.4 Hz), 7.38 (1H, dd, *J=* 8.5, 5.4Hz), 7.96 (1H, s), 8.13 (1H, dd, *J*= 8.9, 6.2 Hz).

### 6.6 N-(2-Ethylsulfanyl-5-fluoro-4-oxo-4H-quinazolin-3-yl)-2-phenyl-acetamide (69)

Obtained as a white solid in 78% yield and 100% purity. ¹H NMR (300 MHz, CDCl₃) δ 1.37 (3H, t, *J*= 7.5Hz), 3.08-3.18 (2H, m), 3.13 (2H, dd, *J*=12.0, 7.4Hz), 6.96 (1H, t, *J*= 8.7Hz), 7.30-7.46 (5H, m), 7.55-7.63 (1H, m).

### 6.7 N-(7-Fluoro-2-isopropylsulfanyl-4-oxo-4H-quinazolin-3-yl)-2-phenyl-acetamide (48)

Obtained as a white solid in 56% yield and 99% purity. ¹H NMR (300 MHz, CDCl₃) δ 1.40 (6H, dd, *J=* 7.1, 1.8 Hz), 3.82 (2H, dd, *J=* 18.6, 16.3 Hz), 3.92 (1H, septet, *J*= 6.9 Hz), 7.05 (1H, dt, *J* = 8.7, 2.4 Hz), 7.17 (1H, dd, *J*= 9.7, 2.4 Hz), 7.28-7.44 (4H, m), 7.86 (1H, s), 8.13 (1H, dd, *J*= 8.7, 6.0 Hz).

### 6.8 N-(5-Fluoro-2-methylsulfanyl-4-oxo-4H-quinazolin-3-yl)-2-(4-fluoro-phenyl)-acetamide (66)

Obtained as a white solid in 70% yield and 100% purity. ¹H NMR (300 MHz, CD₃OD) δ 2.52 (3H, s), 3.77 (2H, s), 7.08 (3H, m), 7.41(3H, m), 7.74 (1H, m).

### 6.9 N-(2-Methylsulfanyl-4-oxo-7-trifluoromethyl-4H-quinazolin-3-yl)-2-phenyl-acetamide (51)

Obtained as a white solid in 50% yield and 98% purity. ¹H NMR (300 MHz, CDCl₃) δ 2.53 (3H, s), 3.86 (2H, s), 7.30-7.47 (5H, m), 7.56 (1H, d, *J*= 8.4Hz), 7.76 (1H, s), 7.84 (1H, s), 8.24 (1H, d, *J*= 8.3Hz).

### 6.10 N-(2-Ethylsulfanyl-4-oxo-7-trifluoromethyl-4H-quinazolin-3-yl)-2-phenyl-acetamide (52)

Obtained as a white solid in 23% yield and 98% purity. ¹H NMR (300 MHz, CDCl₃) δ 1.40 (3H, t, *J*= 7.5 Hz), 3.10-3.20 (2H, m), 3.87 (3H, s), 7.33-7.48 (5H, m), 7.56 (1H, d, *J* = 8.5 Hz), 7.60 (1H, s), 7.84 (1H, s), 8.25 (1H, d, *J* = 8.0 Hz).

### 6.11 N-(2-Isopropylsulfanyl-4-oxo-7-trifluoromethyl-4H-quinazolin-3-yl)-2-phenyl-acetamide (53)

Obtained as a white solid in 23% yield and 98% purity. ¹H NMR (300 MHz, CDCl₃) δ 1.43 (6H, dd, *J* = 6.8, 4.6Hz), 3.86 (2H, s), 3.97 (1H, pentet, J= 6.8Hz), 7.34-7.48 (4H, m), 7.52-7.58 (2H, m), 7.82 (1H, s), 8.25 (1H, d, *J*= 8.4Hz).

### 6.12 2-(3-Fluoro-phenyl)-N-(2-methylsulfanyl-4-oxo-7-trifluoromethyl-4H-quinazolin-3-yl)-acetamide (63)

Obtained as an off-white solid in 100% yield and 98% purity. ¹H NMR (300 MHz, CDCl₃) δ 2.54 (3H, s), 3.85 (3H, s), 7.05 (1H, dt, *J* = 8.5, 2.4Hz), 7.15 (1H, d, *J*= 9.4Hz), 7.21 (1H, d, *J* = 7.6Hz), 7.38 (1H, dt, *J*= 7.8, 6.1Hz), 7.57 (1H, d, *J*= 7.5Hz), 7.82 (1H, s), 7.86 (1H, s), 8.26 (1H, d, *J* = 8.2Hz).

### 6.13 2-(4-Fluoro-phenyl)-N-(2-methylsulfanyl-4-oxo-7-trifluoromethyl-4H-quinazolin-3-yl)-acetamide (62)

Obtained as a tan solid in 95% yield and 98% purity. ¹H NMR (300 MHz, CDCl₃) δ 2.54 (3H, s), 3.82 (2H, s), 7.39 (2H, m), 7.57(1H, d, *J*= 8.4Hz), 7.85 (1H, s), 7.90 (2H, t, *J*= 8.5Hz), 8.25 (1H, d, *J*= 8.2Hz).

### 6.14 N-[4-Oxo-2-(3,3,3-trifluoro-propylsulfanyl)-5,6,7,8-tetrahydro-4H-quinazolin-3-yl]-2-phenyl-acetamide (142)

¹H NMR (300 MHz, d-6 DMSO) δ 1.55-1.78 (5H, m), 2.26-2.39 (2H, m), 2.50-2.65 (3H, m), 3.05-3.22 (2H, m), 3.67 (2H, s), 7.23-7.32 (5H, m), 11.25 (1H, brs).

### 6.15 N-(7-Methyl-2-methylsulfanyl-4-oxo-4H-thieno[3,2-d]pyrimiidin-3-yl)-2-phenyl-acetamide (90)

¹H NMR (300 MHz, d-6 DMSO) δ 2.30 (3H, s), 2.48 (3H, s), 3.72 (3H, s), 7.23-7.35 (5H, m), 7.86 (1H, s), 11.39 (1H, brs).

### 6.16 N-(7-Methanesufonyl-2-methylsulfanyl-4-oxo-4H-quinazolin-3-yl)-2-phenyl-acetamide (157)

¹H NMR (300 MHz, d-6 DMSO) δ 2.52 (3H, s), 2.33 (3H, s), 3.74 (3H, s), 7.24-7.35 (5H, m), 7.92 (1H, dd, *J*= 8.4, 1.6 Hz), 8.07 (1H, d, *J*= 1.4 Hz), 8.28 (1H, d, *J*= 8.4 Hz), 11.50 (1H, brs).

### 6.17 2-(3-Fluoro-phenyl)-N-(2-isopropylsulfanyl-7-methyl-4-oxo-4H-thieno[3,2-d]pyrimidin-3-yl)-acetamide (94)

¹H NMR (300 MHz, d-6 DMSO) δ 1.35 (3H, d, *J=* 6.9 Hz), 1.38 (3H, d, *J*= 6.8 Hz), 2.29 (3H, s), 3.74 (3H, s), 3.83 (1H, sept, *J*= 7.0 Hz), 7.08 (1H, d, *J*= 8.9, 2.3 Hz), 7.17-7.20 (2H, m), 7.33-7.41 (1H, m), 7.87 (1H,s), 11.37 (1H, brs).

### EXAMPLE 7

This example illustrates a screening protocol for evaluating compounds of the present invention for the ability to open voltage-gated potassium channels.

NG108-15 cells, a mouse neuroblastoma, rat glioma hybrid cell line, functionally express M-currents (Robbins et al., J. Physiol. 451: 159-85 (1992). NG108-15 M-currents are likely comprised, at least in part, of KCNQ2, KCNQ3 and KCNQ5, since these genes are reportedly robustly expressed in differentiated NG108-15 cells (Selyanko et al., J. Neurosci. 19(18): 7742-56 (1999); Schroeder et al., J. Biol. Chem. 275(31): 24089-95 (2000)) and KCNQ3 dominant- negative constructs reduce M-current density in these cells (Selyanko et al., J. Neurosci. 22(5): RC212 (2002).

NG108-15 were maintained in DMEM (high glucose) supplemented with 10% fetal bovine serum, 0.05 mM pyridoxine, 0.1 mM hypoxanthine, 400 nM aminopterin, 16 mM thymidine, 50 µgml⁻¹ gentamycin and 10 mM HEPES, in an incubator at 37°C with a humidified atmosphere of 5 % CO₂. Cells were plated in 96 well plates differentiated by addition of 10 µM PGE1 and 50 µM isomethylbutylxanthine to the growth media prior to study.

Differentiated NG108-15 cells were loaded with voltage-sensitive dye by incubation in Earls Balanced Salt Solution (EBSS) containing 5 mM DiBAC for 1h. Following loading, drug solution containing 5 mM DiBAC was added to each well. Changes in fluorescence were measured every 30 s for 25 min. The maximum change in fluorescence was measured and expressed as a percentage of the maximum response obtained in the presence of a positive control agent.

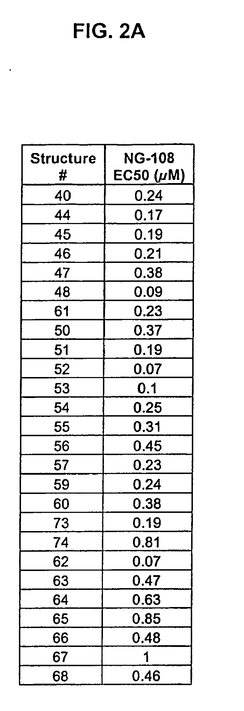

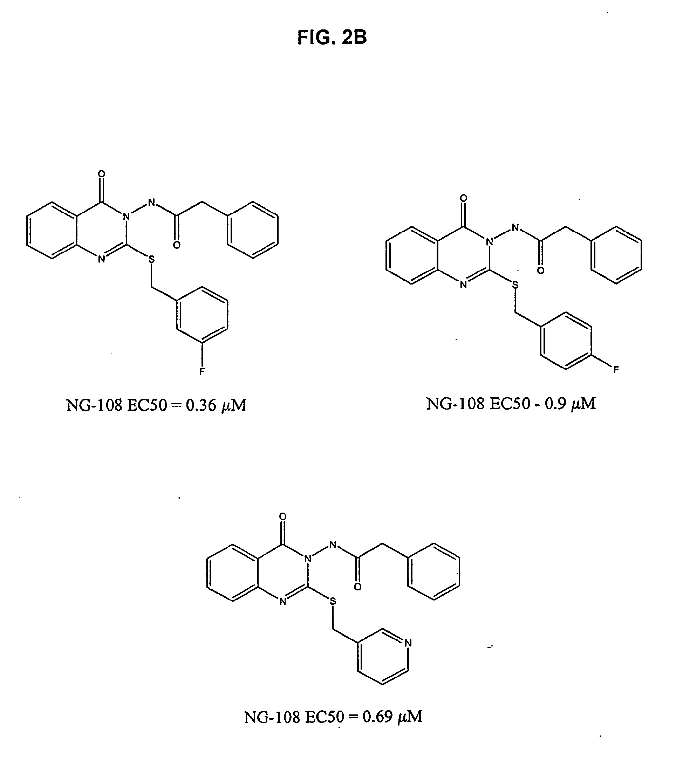

**FIG. 2** includes results of assays of compounds of the invention by the above procedure.

## Claims

1. A compound having the formula: 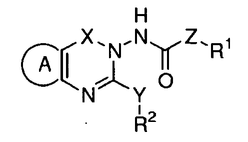 in which
A is a member selected from the group of five- and six-membered substituted or unsubstituted aryl, five- and six-membered substituted or unsubstituted heteroaryl, substituted or unsubstituted C₄-C₈ cycloalkyl, and substituted or unsubstituted 5-8 membered heterocyclyl;
X CO;
Z is a member selected from the group consisting of -CH₂-, -CHF-, -CF₂-, - CH=CH- and -N(R⁴)(CR^{4a}R^{4b}₎ₛ-
wherein R⁴ is a member selected from H and a substituted or unsubstituted C₁-C₅ alkyl group;
R^{4a} and R^{4b} are members independently selected from H, substituted or unsubstituted aryl, substituted or unsubstituted heteroaryl, substituted or unsubstituted C₃-C₈ cycloalkyl, substituted or unsubstituted 5-7 membered heterocyclyl, and substituted or unsubstituted C₁-C₈ alkyl;
s is an integer from 1 to 3;
Y is S(O)ₙ, wherein n is an integer from 0-2;
R¹ is a member selected from substituted or unsubstituted aryl, substituted or unsubstituted heteroaryl, substituted or unsubstituted C₃-C₈ cycloalkyl, substituted or unsubstituted 5-7 membered heterocyclyl, and substituted or unsubstituted C₁-C₈ alkyl; and
R² is a member selected from the group consisting of CF₃, substituted or unsubstituted C₁-C₈ alkyl, substituted or unsubstituted aryl, substituted or unsubstituted heteroaryl, substituted or unsubstituted C₃-C₈ cycloalkyl, substituted or unsubstituted 3-7-membered heterocyclyl.

2. A compound according to claim **1** provided that when A is phenyl, Z is -CH₂-, and R¹ is phenyl, substituted phenyl or heteroaryl, then R² is other than a benzyl, substituted benzyl, alkylheteroaryl, alkylheterocyclyl or cyanomethyl group.

3. A compound according to claim **1** or claim **2** in which A is a member selected from substituted or unsubstituted aryl, and substituted or unsubstituted heteroaryl.

4. A compound according to claim **3** in which A is substituted or unsubstituted phenyl.

5. A compound according to claim **4** in which A is phenyl substituted by one or two groups selected from halogen, nitrile, substituted or unsubstituted C₁-C₄ alkyl, SCF₃, trifluoromethyl and trifluoromethoxy.

6. A compound according to any preceding claim in which Z is -CH₂-.

7. A compound according to any preceding claim in which R¹ is a member selected from substituted or unsubstituted aryl, and substituted or unsubstituted heteroaryl.

8. A compound according to claim **7** in which R¹ is substituted or unsubstituted phenyl.

9. A compound according to claim 8 in which R¹ is a member selected from phenyl, and phenyl substituted with one or more of halogen, CF₃ and OCF₃.

10. A compound according to any preceding claim in which R² is a substituted or unsubstituted C₁-C₆ saturated acyclic alkyl group.

11. A compound according to claim **10** in which R² is a C₁-C₄ saturated acyclic alkyl group.

12. The compound according to claim **1** having the formula: 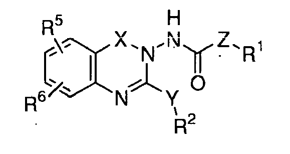 in which
X is CO;
Z is a member selected from -CH₂-, -CHF-, -CF₂- and -CH=CH- and -N(R⁴)(CR^{4a}R^{4b})ₛ-
wherein R⁴ is a member selected from H and a substituted or unsubstituted C₁-C₅ alkyl group;
R^{4a} and R^{4b} are members independently selected from H, substituted or unsubstituted aryl, substituted or unsubstituted heteroaryl, substituted or unsubstituted C₃-C₈ cycloalkyl, substituted or unsubstituted 5-7 membered heterocyclyl, and substituted or unsubstituted C₁-C₈ alkyl;
s is an integer from 1 to 3;
Y is S;
R¹ is a member selected from substituted or unsubstituted aryl, substituted or unsubstituted heteroaryl, substituted or unsubstituted C₃-C₈ cycloalkyl, substituted or unsubstituted 5-7 membered heterocyclyl, and substituted or unsubstituted C₁-C₈ alkyl;
R² is a member selected from CF₃, substituted or unsubstituted C₁-C₈ alkyl, substituted or unsubstituted aryl, substituted or unsubstituted heteroaryl, substituted or unsubstituted C₃-C₈ cycloalkyl, substituted or unsubstituted 3-7 membered saturated heterocyclyl;
R⁵ and R⁶ are independently selected from H, halo, CF₃, CF₃O, NO₂, CN, S(O)ₘR⁷ COOR⁸, CONR⁹R¹⁰, SO₂NR¹¹R¹², S(O)ₘCF₃, substituted or unsubstituted C₁-C₆ alkyl, and substituted or unsubstituted C₃-C₇ cycloalkyl
wherein R⁷ and R⁸ are independently selected from substituted or unsubstituted C₁-C₅ alkyl, and substituted or unsubstituted C₃-C₇ cycloalkyl;
m is an integer from 0 to 2; and
R⁹, R¹⁰, R¹¹ and R¹² are independently selected from H, substituted or unsubstituted C₁-C₅ alkyl, substituted or unsubstituted C₃-C₇ cycloalkyl, and R⁹ and R¹⁰ or R¹¹ and R¹², together with the nitrogen atom to which they are attached, are optionally joined to form a 5- to 7-membered ring.

13. The compound according to claim **12** wherein
Z is CH₂; and
R¹ is substituted or unsubstituted phenyl.

14. The compound according to claim **12** or **13** wherein
R² is a member selected from substituted or unsubstituted C₁-C₄ alkyl, substituted or unsubstituted C₃-C₆ cycloalkyl and substituted or unsubstituted C₃-C₆ heterocyclyl; and
R⁵ and R⁶ are members independently selected from H, halo, CF₃, OCF₃, substituted or unsubstituted C₁-C₅ alkyl, SCF₃ and CN.

15. A compound according to claim 1 having the formula: 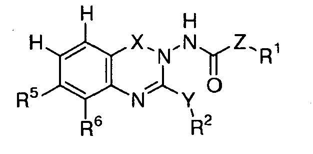 in which
X is CO;
Z is a member selected from -CH₂-, -CHF-, -CF₂- and -CH=CH- and -N(R⁴)(CR^{4a}R^{4b})ₛ-
wherein R⁴ is a member selected from H and a substituted or unsubstituted C₁-C₅ alkyl group;
R^{4a} and R^{4b} are members independently selected from H, substituted or unsubstituted aryl, substituted or unsubstituted heteroaryl, substituted or unsubstituted C₃-C₈ cycloalkyl, substituted or unsubstituted 5-7 membered heterocyclyl, and substituted or unsubstituted C₁-C₈ alkyl;
s is an integer from 1 to 3;
R¹ is a member selected from substituted or unsubstituted aryl, substituted or unsubstituted heteroaryl, substituted or unsubstituted C₃-C₈ cycloalkyl, substituted or unsubstituted 5-7 membered heterocyclyl, substituted or unsubstituted C₁-C₈ alkyl;
Y is S;
R² is a member selected from CF₃, substituted or unsubstituted C₁-C₈ alkyl, substituted or unsubstituted aryl, substituted or unsubstituted heteroaryl, substituted or unsubstituted C₃-C₈ cycloalkyl, substituted or unsubstituted 3-7 membered saturated heterocyclyl;
R⁵ and R⁶ are independently selected from halo, CF₃, CF₃O, NO₂, CN, S(O)ₘR⁷ COOR⁸, CONR⁹R¹⁰, SO₂NR¹¹R¹², S(O)ₘCF₃, substituted or unsubstituted C₁-C₆ alkyl, and substituted or unsubstituted C₃-C₇ cycloalkyl
wherein R⁷ and R⁸ are independently selected from substituted or unsubstituted C₁-C₅ alkyl, and substituted or unsubstituted C₃-C₇ cycloalkyl;
m is an integer from 0 to 2; and
R⁹, R¹⁰, R¹¹ and R¹² are independently selected from H, substituted or unsubstituted C₁-C₅ alkyl, substituted or unsubstituted C₃-C₇ cycloalkyl, and R⁹ and R¹⁰ or R¹¹ and R¹², together with the nitrogen atom to which they are attached, are optionally joined to form a 5- to 7-membered ring.

16. The compound according to claim **15** wherein
Z is CH₂;
and
R¹ is substituted or unsubstituted phenyl.

17. The compound according to claim **15** wherein
R² is a member selected from substituted or unsubstituted C₁-C₄ alkyl, substituted or unsubstituted C₃-C₆ cycloalkyl and substituted or unsubstituted C₃-C₇ heterocyclyl; and
R⁵ and R⁶ are members independently selected from halo, CF₃, OCF₃, substituted or unsubstituted C₁-C₅ alkyl, SCF₃ and CN.

18. A compound of claim **1**, wherein the compound has a structure selected from the following formulae:

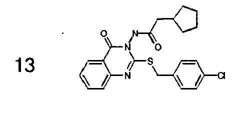

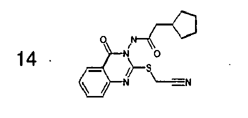

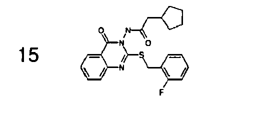

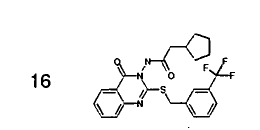

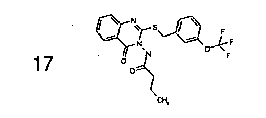

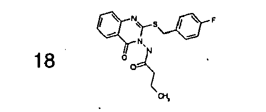

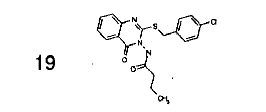

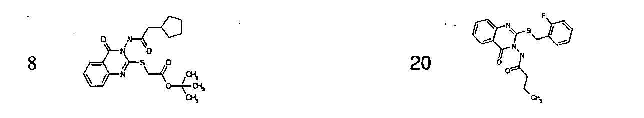

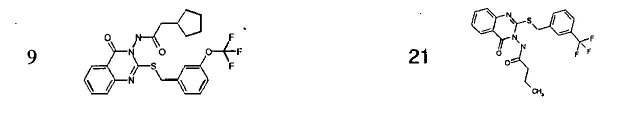

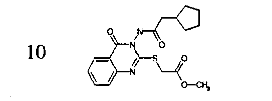

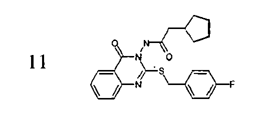

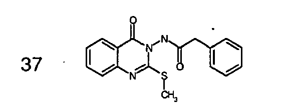

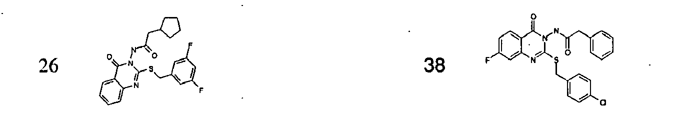

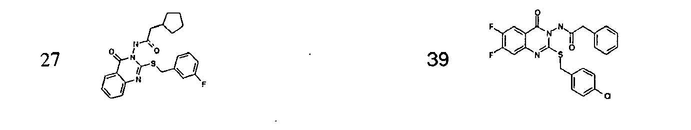

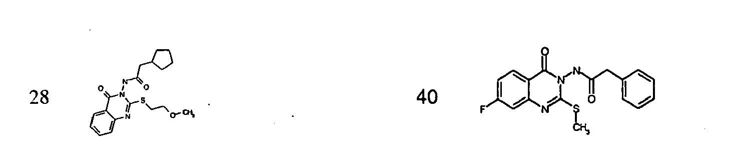

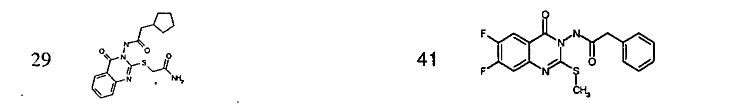

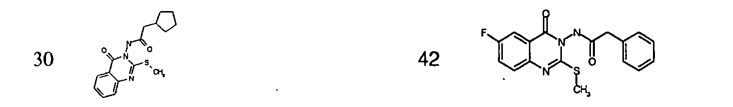

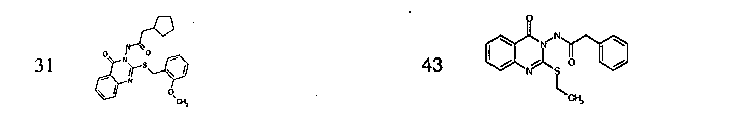

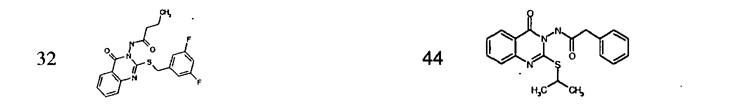

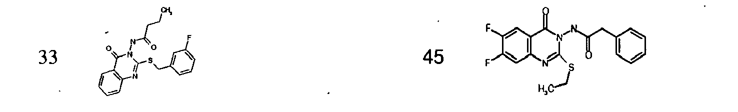

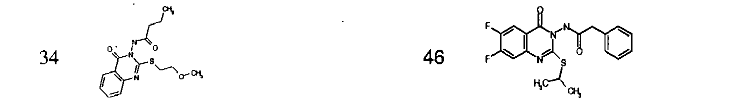

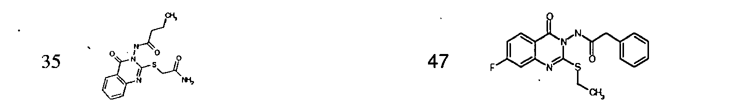

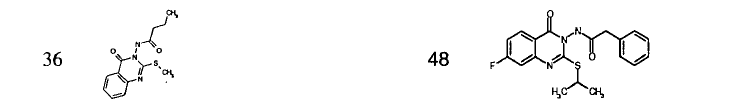

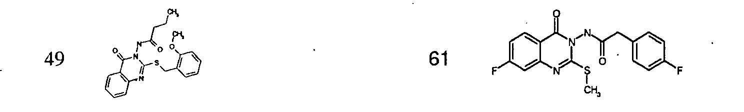

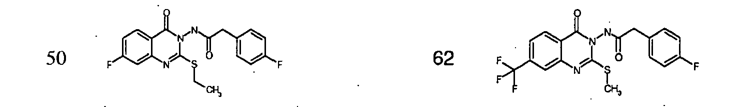

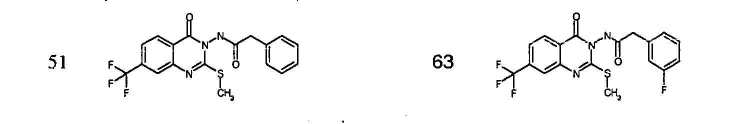

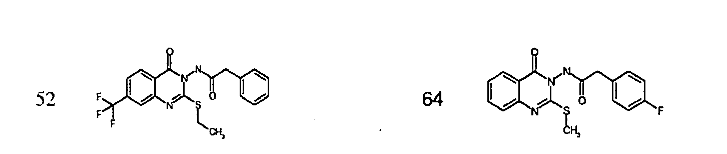

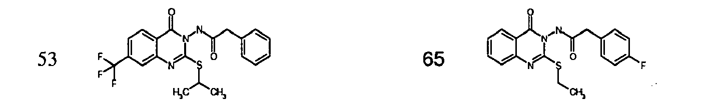

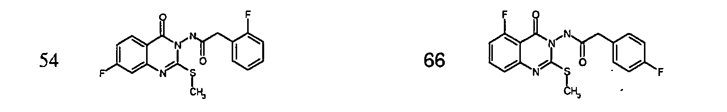

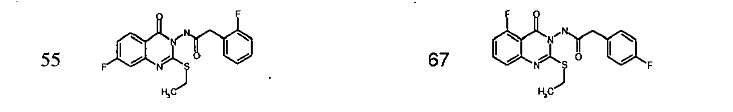

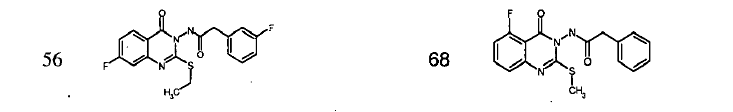

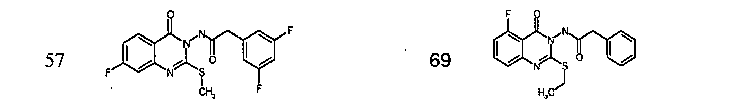

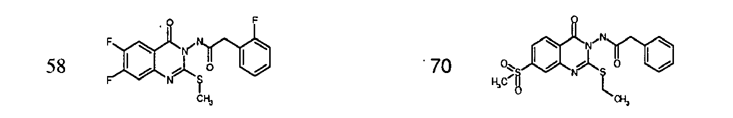

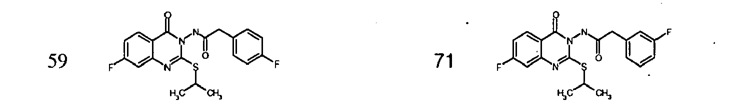

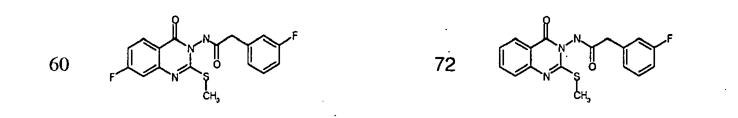

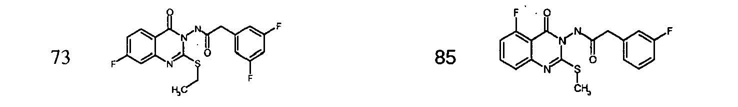

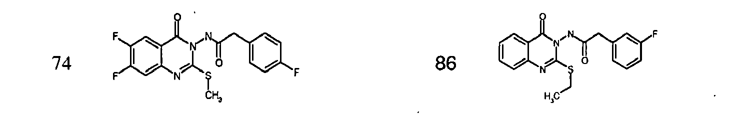

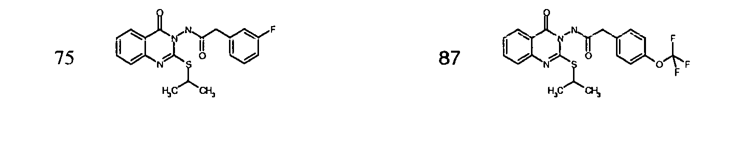

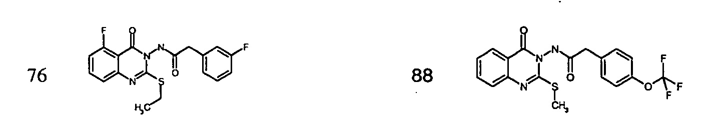

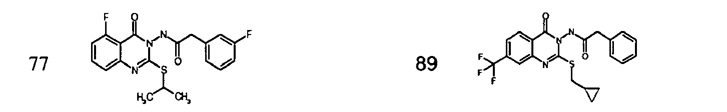

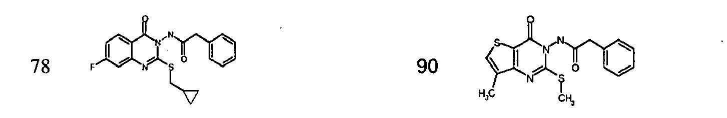

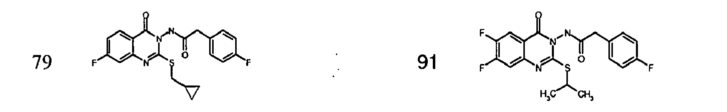

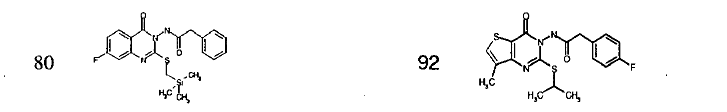

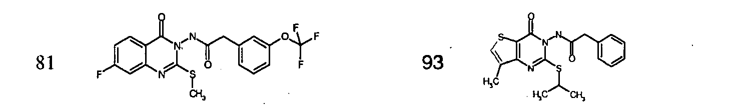

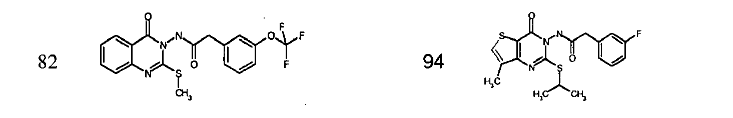

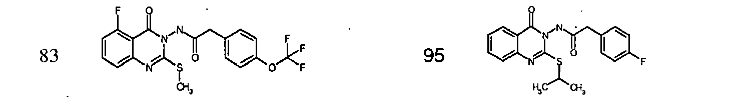

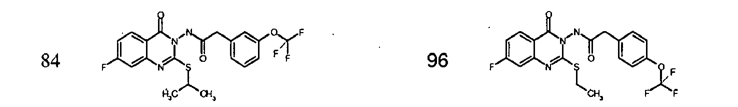

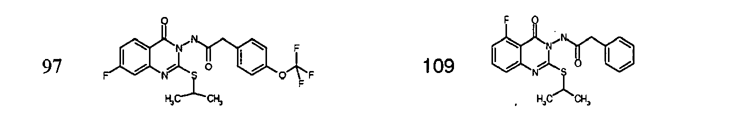

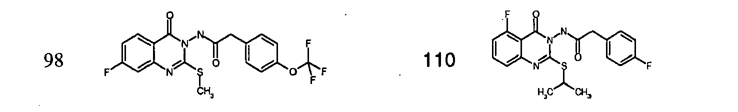

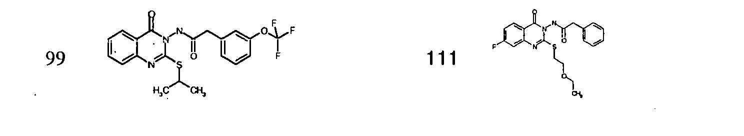

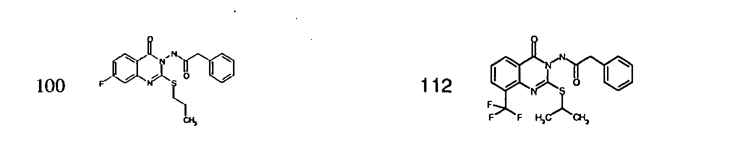

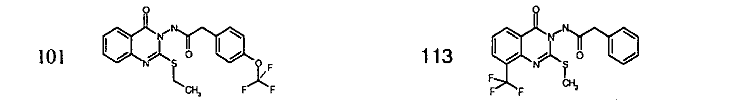

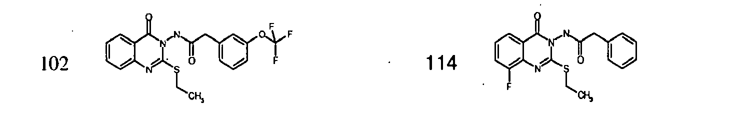

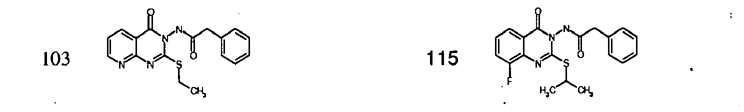

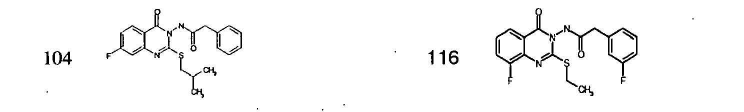

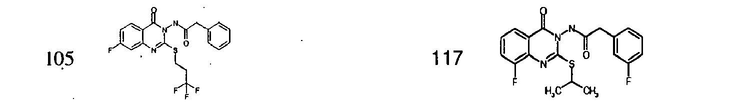

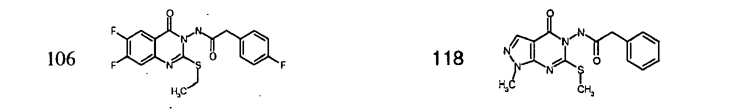

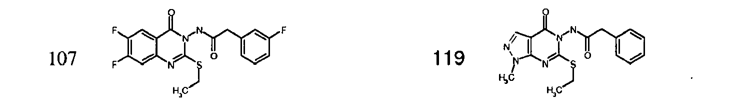

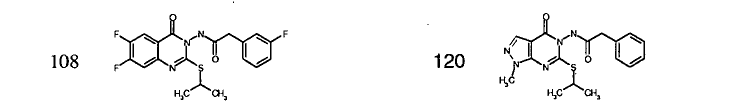

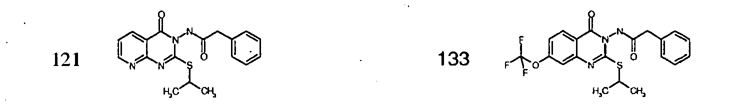

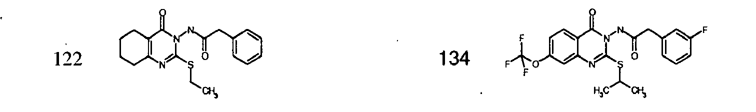

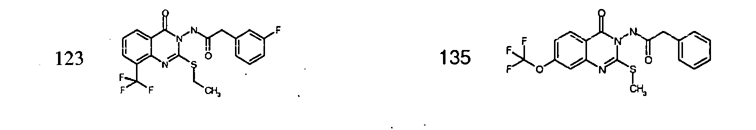

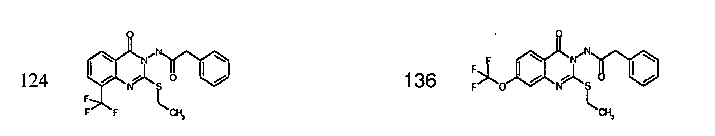

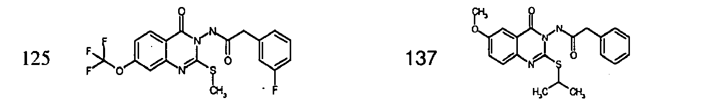

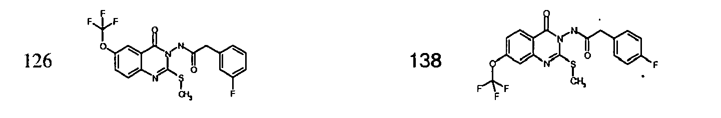

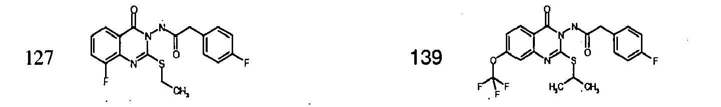

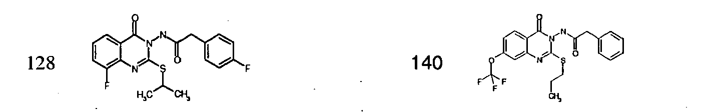

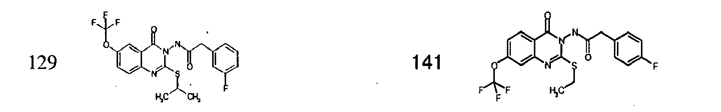

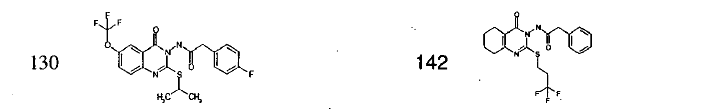

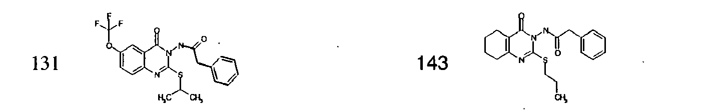

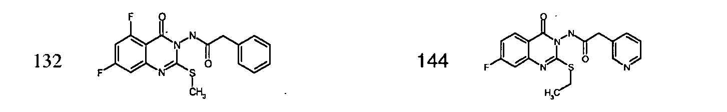

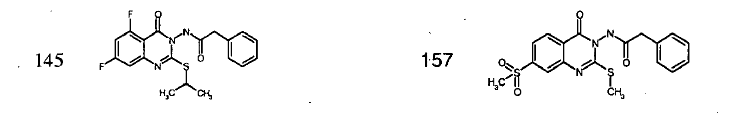

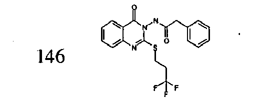

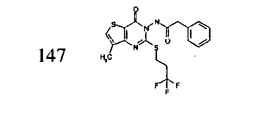

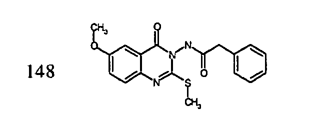

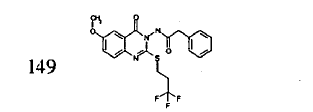

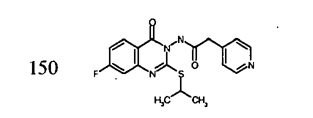

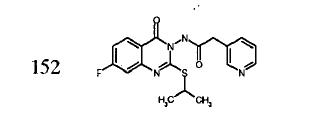

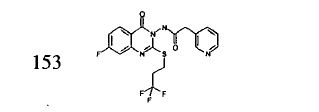

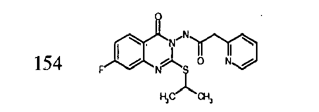

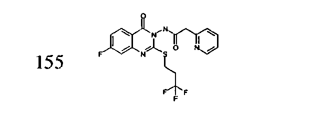

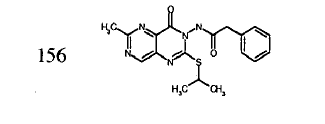

19. The compound of claim **18,** wherein the compound is selected from compounds 40, 44, 45, 46, 47, 48, 50, 51, 52, 53, 54, 55, 56, 57, 59, 60, 61, 62, 63, 64, 65, 66, 67, 68, 73 or 74.

20. The compound of claim **18,** wherein the compound is selected from compounds 41, 42, 71, 78-81, 84, 91, 96-98, 100, 104-108, 111, 132 and 145.

21. A compound of claim **1,** wherein the compound has a structure selected from the following formulae: 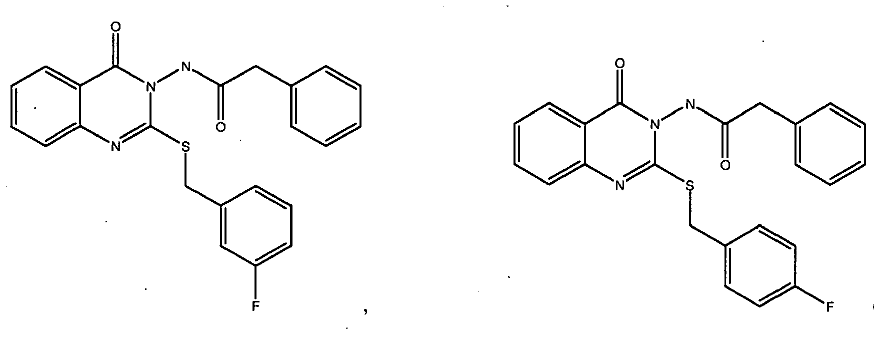 or

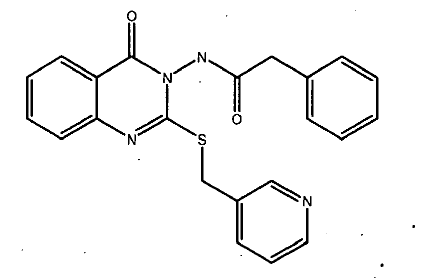

22. A pharmaceutical composition comprising a pharmaceutically acceptable excipient and an effective amount of a compound according to any previous claim.

23. A compound of any of claims **1** to **21** for use as a pharmaceutical.

24. A compound of any of claims **1** to **21** for increasing ion flow in a voltage-dependent potassium channel, for use in the treatment of a condition mediated by voltage-dependent potassium channels.

25. A method of increasing ion flow through voltage-dependent potassium channels in a cell, said method comprising contacting said cell with a potassium channel-opening amount of a compound according to any of claims **1** to **21,** provided that the method is not a method of treatment of the human or animal body by therapy.

26. The method according to claim **25,** wherein said voltage-dependent potassium channel is responsible for the M-current and/or the channels are KCNQ voltage-dependent potassium channels in a cell.

27. A use of a compound of any of claims **1** to **21** for the manufacture of a medicament for treating a central or peripheral nervous system disorder or condition through modulation of a KCNQ voltage-dependent potassium channels.

28. The use according to claim **27,** wherein said disorder or condition is selected from neuronal degeneration disorders, migraine, ataxia, Parkinson's disease, bipolar disorders, spasticity, mood disorders, brain tumors, psychotic disorders, myokymia, seizures, epilepsy, hearing loss, vision loss, Alzheimer's disease, age-related memory loss, learning deficiencies, motor neuron diseases, trigeminal neuralgia, retinal degeneration , elevated intraocular pressure, glaucoma and stroke.

29. The use in accordance with claim **28,** wherein said condition or disorder is epilepsy or seizures.

30. The use in accordance with claim **28,** wherein said condition or disorder is hearing loss.

31. The use in accordance with claim **27,** wherein said condition or disorder is pain or anxiety.

32. The use in accordance with claim **28,** wherein said condition or disorder is neuronal degeneration.

33. The use in accordance with claim **28,** wherein said condition or disorder is retinal degeneration.

34. The use of a compound of any claims **1** to **21** for the manufacture of a medicament for treating a member selected from epilepsy, retinal degeneration, pain, anxiety, neuronal degeneration and bipolar disorder through modulation of a voltage-dependent potassium channel.

35. A use according to claim **34** wherein said pain is a member selected from neuropathic pain, diabetic pain, somatic pain, cutaneous pain, visceral pain, inflammatory pain, cancer pain, migraine pain, or musculoskeletal pain.

## Patentansprüche

1. Verbindung mit der Formel 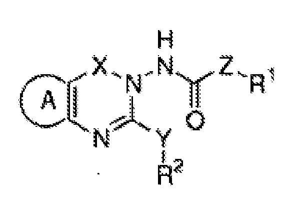 worin
A ein Element ist, das gewählt ist aus der Gruppe 5- und 6-gliedriges substituiertes oder unsubstituiertes Aryl, 5- und 6-gliedriges substituiertes oder unsubstituiertes Heteroaryl, substituiertes oder unsubstituiertes C₄- bis C₈-Cycloalkyl und substituiertes oder unsubstituiertes 5- bis 8-gliedriges Heterocyclyl;
X für CO steht;
Z ein Element ist, das gewählt ist aus der Gruppe, die besteht aus -CH₂-, -CHF-, -CF₂-, -CH=CH- und -N(R⁴)(CR^{4a}R^{4b})ₛ-,
worin R⁴ ein Element ist, das gewählt ist aus H und einer substituierten oder unsubstituierten C₁- bis C₅-Alkyl-Gruppe;
R^{4a} und R^{4b} Elemente sind, die unabhängig voneinander gewählt sind aus H, substituiertem oder unsubstituiertem Aryl, substituiertem oder unsubstituiertem Heteroaryl, substituiertem oder unsubstituiertem C₃- bis C₈-Cycloalkyl, substituiertem oder unsubstituiertem 5- bis 7-gliedrigem Heterocyclyl und substituiertem oder unsubstituiertem C₁- bis C₈-Alkyl;
s eine ganze Zahl von 1 bis 3 ist;
Y für S(O)ₙ steht, worin n eine ganze Zahl von 0 bis 2 ist;
R¹ ein Element ist, das gewählt ist aus substituiertem oder unsubstituiertem Aryl, substituiertem oder unsubstituiertem Heteroaryl, substituiertem oder unsubstituiertem C₃- bis C₈-Cycloalkyl, substituiertem oder unsubstituiertem 5-bis 7-gliedrigem Heterocyclyl und substituiertem oder unsubstituiertem C₁- bis C₈-Alkyl; und
R² ein Element ist, das gewählt ist aus der Gruppe, die besteht aus CF₃, substituiertem oder unsubstituiertem C₁- bis C₈-Alkyl, substituiertem oder unsubstituiertem Aryl, substituiertem oder unsubstituiertem Heteroaryl, substituiertem oder unsubstituiertem C₃- bis C₈-Cycloalkyl, substituiertem oder unsubstituiertem 3- bis 7-gliedrigem Heterocyclyl.

2. Verbindung nach Anspruch 1, mit der Maßgabe, dass dann, wenn A für Phenyl steht, Z für -CH₂- steht und R¹ für Phenyl, substituiertes Phenyl oder Heteroaryl steht, R² eine andere Bedeutung hat als eine Benzyl-Gruppe, substituierte Benzyl-Gruppe, Alkylheteroaryl-Gruppe, Alkylheterocyclyl-Gruppe oder Cyanomethyl-Gruppe.

3. Verbindung nach Anspruch 1 oder Anspruch 2, in der A ein Element ist, das gewählt ist aus substituiertem oder unsubstituiertem Aryl und substituiertem oder unsubstituiertem Heteroaryl.

4. Verbindung nach Anspruch 3, worin A substituiertes oder unsubstituiertes Phenyl ist.

5. Verbindung nach Anspruch 4, in der A Phenyl ist, das substituiert ist durch eine oder zwei Gruppen, gewählt aus Halogen, Nitril, substituiertes oder unsubstituiertes C₁- bis C₄-Alkyl, SCF₃, Trifluormethyl und Trifluormethoxy.

6. Verbindung nach irgendeinem vorangehenden Anspruch, in der Z für -CH₂-steht.

7. Verbindung nach irgendeinem vorangehenden Anspruch, in der R¹ ein Element ist, das gewählt ist aus substituiertem oder unsubstituiertem Aryl und substituiertem oder unsubstituiertem Heteroaryl.

8. Verbindung nach Anspruch 7, in der R¹ für substituiertes oder unsubstituiertes Phenyl steht.

9. Verbindung nach Anspruch 8, in der R¹ ein Element ist, das gewählt ist aus Phenyl und Phenyl, das substituiert ist mit einem oder mehreren Substituenten aus der Gruppe Halogen, CF₃ und OCF₃.

10. Verbindung nach irgendeinem vorangehenden Anspruch, in der R2 für eine substituierte oder unsubstituierte gesättigte, acyclische C₁- bis C₆-Alkyl-Gruppe steht.

11. Verbindung nach Anspruch 10, in der R² eine gesättigte acyclische C₁- bis C₃-Alkyl-Gruppe ist.

12. Verbindung nach Anspruch 1 mit der Formel 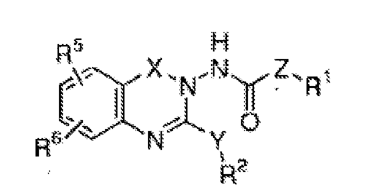 in der
X für CO steht;
Z ein Element ist, das gewählt ist aus -CH₂-, -CHF-, -CF₂- und -CH=CH- und -N(R⁴)(CR^{4a}R^{4b})ₛ-,
worin R⁴ ein Element ist, das gewählt ist aus H und einer substituierten oder unsubstituierten C₁- bis C₅-Alkyl-Gruppe;
R^{4a} und R^{4b} Elemente sind, die unabhängig voneinander gewählt sind aus H, substituiertem oder unsubstituiertem Aryl, substituiertem oder unsubstituiertem Heteroaryl, substituiertem oder unsubstituiertem C₃- bis C₈-Cycloalkyl, substituiertem oder unsubstituiertem 5- bis 7-gliedrigem Heterocyclyl und substituiertem oder unsubstituiertem C₁- bis C₈-Alkyl;
s eine ganze Zahl von 1 bis 3 ist;
Y für S steht;
R¹ ein Element ist, das gewählt ist aus substituiertem oder unsubstituiertem Aryl, substituiertem oder unsubstituiertem Heteroaryl, substituiertem oder unsubstituiertem C₃- bis C₈-Cycloalkyl, substituiertem oder unsubstituiertem 5-bis 7-gliedrigem Heterocyclyl und substituiertem oder unsubstituiertem C₁- bis C₈-Alkyl;
R² ein Element ist, das gewählt ist aus CF₃, substituiertem oder unsubstituiertem C₁- bis C₈-Alkyl, substituiertem oder unsubstituiertem Aryl, substituiertem oder unsubstituiertem Heteroaryl, substituiertem oder unsubstituiertem C₃- bis C₈-Cycloalkyl, substituiertem oder unsubstituiertem 3-bis 7-gliedrigem gesättigtem Heterocyclyl;
R⁵ und R⁶ unabhängig voneinander gewählt sind aus H, Halogen, CF₃, CF₃O, NO₂, CN, S(O)ₘR⁷, COOR⁸, CONR⁹R¹⁰, SO₂NR¹¹R¹², S(O)ₘCF₃, substituiertem oder unsubstituiertem C₁- bis C₆-Alkyl und substituiertem oder unsubstituiertem C₃- bis C₇-Cycloalkyl:
worin R⁷ und R⁸ unabhängig voneinander gewählt sind aus substituiertem oder unsubstituiertem C₁- bis C₅-Alkyl und substituiertem oder unsubstituiertem C₃-bis C₇-Cycloalkyl;
m eine ganze Zahl von 0 bis 2 ist; und
R⁹, R¹⁰, R¹¹ und R¹² unabhängig voneinander gewählt sind aus H, substituiertem oder unsubstituiertem C₁- bis C₅-Alkyl, substituiertem oder unsubstituiertem C₃- bis C₇-Cycloalkyl und R⁹ und R¹⁰ oder R¹¹ und R¹² zusammen mit dem Stickstoffatom, an das sie gebunden sind, gegebenenfalls unter Bildung eines 5- bis 7-gliedrigen Rings vereinigt sind.

13. Verbindung nach Anspruch 12, worin
Z für CH₂ steht; und
R¹ für substituiertes oder unsubstituiertes Phenyl steht.

14. Verbindung nach Anspruch 12 oder 13, worin R² ein Element ist, das gewählt ist aus substituiertem oder unsubstituiertem C₁- bis C₄-Alkyl, substituiertem oder unsubstituiertem C₃- bis C₆-Cycloalkyl und substituiertem oder unsubstituiertem C₃- bis C₆-Heterocyclyl; und
R⁵ und R⁶ Elemente sind, die unabhängig voneinander gewählt sind aus H, Halogen, CF₃, OCF₃ substituiertem oder unsubstituiertem C₁- bis C₅-Alkyl, SCF₃ und CN.

15. Verbindung nach Anspruch 1 mit der Formel 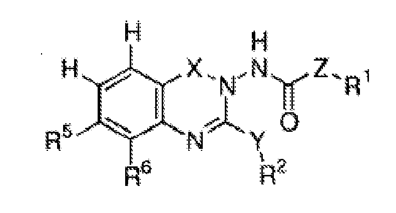 in der
X für CO steht;
Z ein Element ist, das gewählt ist aus -CH₂-, CHF-, -CF₂- und -CH=CH- und -N(R⁴)(CR^{4a}R^{4b})ₛ-;
worin R⁴ ein Element ist, das gewählt ist aus H und einer substituierten oder unsubstituierten C₁- bis C₅-Alkyl-Gruppe;
R^{4a} und R^{4b} Elemente sind, die unabhängig voneinander gewählt sind aus H, substituiertem oder unsubstituiertem Aryl, substituiertem oder unsubstituiertem Heteroaryl, substituiertem oder unsubstituiertem C₃- bis C₈-Cycloalkyl, substituiertem oder unsubstituiertem 5- bis 7-gliedrigem Heterocyclyl und substituiertem oder unsubstituiertem C₁- bis C₈-Alkyl;
s eine ganze Zahl von 01 bis 3 ist;
R¹ ein Element ist, das gewählt ist aus substituiertem oder unsubstituiertem Aryl, substituiertem oder unsubstituiertem Heteroaryl, substituiertem oder unsubstituiertem C₃- bis C₈-Cycloalkyl, substituiertem oder unsubstituiertem 5-bis 7-gliedrigem Heterocyclyl, substituiertem oder unsubstituiertem C₁- bis C₈-Alkyl;
Y für S steht;
R² ein Element ist, das gewählt ist aus CF₃, substituiertem oder unsubstituiertem C₁- bis C₈-Alkyl, substituiertem oder unsubstituiertem Aryl, substituiertem oder unsubstituiertem Heteroaryl, substituiertem oder unsubstituiertem C₃- bis C₈-Cycloalkyl, substituiertem oder unsubstituiertem 3- bis 7-gliedrigem gesättigten Heterocyclyl,
R⁵ und R⁶ unabhängig voneinander gewählt sind aus Halogen, CF₃, CF₃O, NO₂, CN, S(O)ₘR⁷, COOR⁸, ,CONR⁹R¹⁰, SO₂NR¹¹R¹², S(O)ₘCF₃, substituiertem oder unsubstituiertem C₁- bis C₆-Alkyl und substituiertem oder unsubstituiertem C₃-bis C₇-Cycloalkyl,
worin R⁷ und R⁸ unabhängig voneinander gewählt sind aus substituiertem oder unsubstituiertem C₁- bis C₅-Alkyl und substituiertem oder unsubstituiertem C₃-bis C₇-Cycloalkyl;
m eine ganze Zahl von 0 bis 2 ist; und
R⁹, R¹⁰, R¹¹ und R¹² unabhängig voneinander gewählt sind aus H, substituiertem oder unsubstituiertem C₁- bis C₅-Alkyl, substituiertem oder unsubstituiertem C₃-bis C₇-Cycloalkyl und R⁹ und R¹⁰ oder R¹¹ und R¹² zusammen mit dem Stickstoffatom, an das sie gebunden sind, gegebenenfalls unter Bildung eines 5-bis 7-gliedrigen Rings vereinigt sind.

16. Verbindung nach Anspruch 15, worin
Z für CH₂ steht;
und
R¹ für substituiertes oder unsubstituiertes Phenyl steht.

17. Verbindung nach Anspruch 15, worin
R² ein Element ist, das gewählt ist aus substituiertem oder unsubstituiertem C₁-bis C₄-Alkyl, substituiertem oder unsubstituiertem C₃- bis C₆-Cycloalkyl und substituiertem oder unsubstituiertem C₃- bis C₇-Heterocyclyl; und
R⁵ und R⁶ Elemente sind, die unabhängig voneinander gewählt sind aus Halogen, CF₃, OCF₃, substituiertem oder unsubstituiertem C₁- bis C₅-Alkyl, SCF₃ und CN.

18. Verbindung nach Anspruch 1, worin die Verbindung eine Struktur aufweist, die gewählt ist aus den folgenden Formeln:

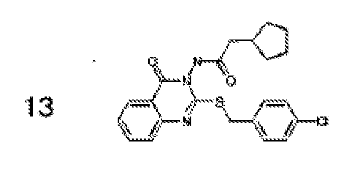

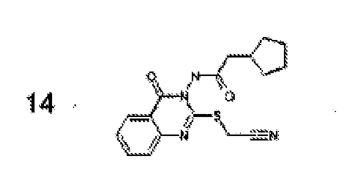

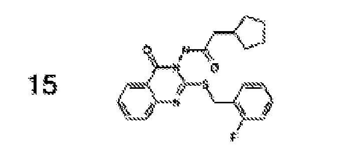

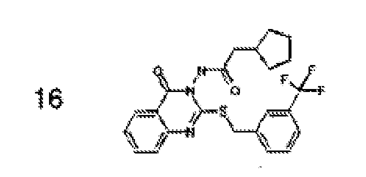

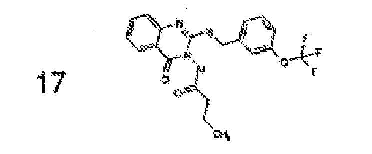

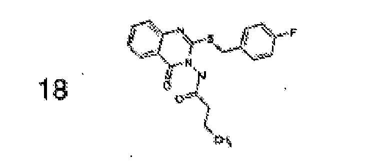

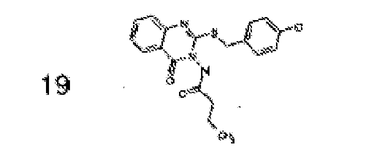

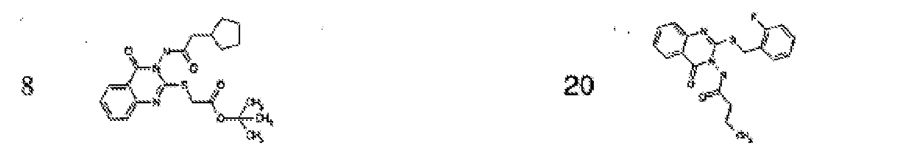

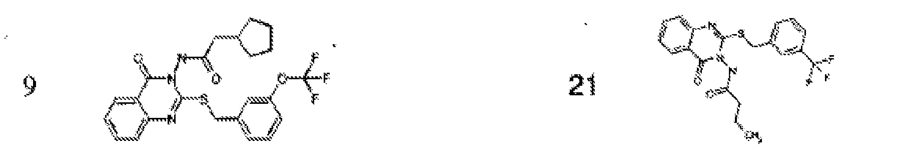

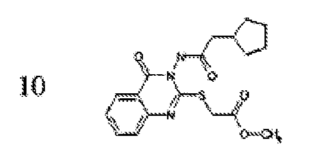

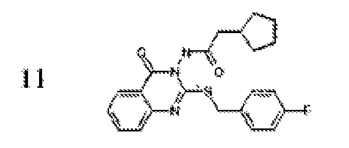

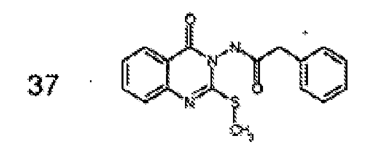

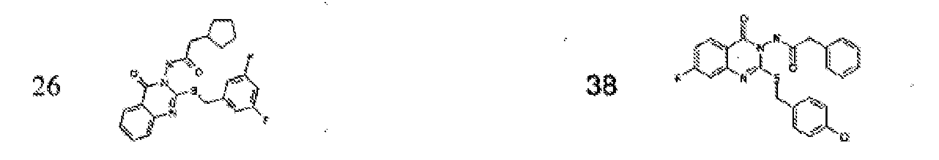

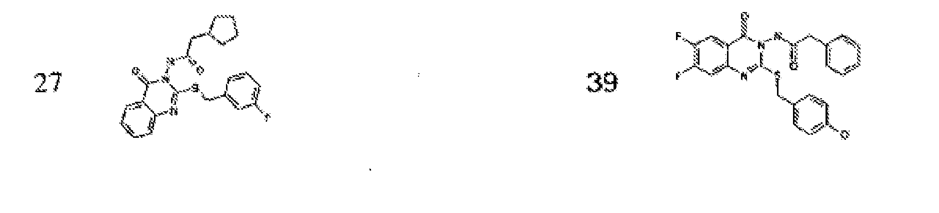

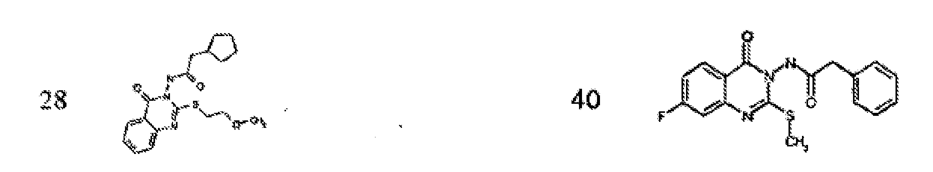

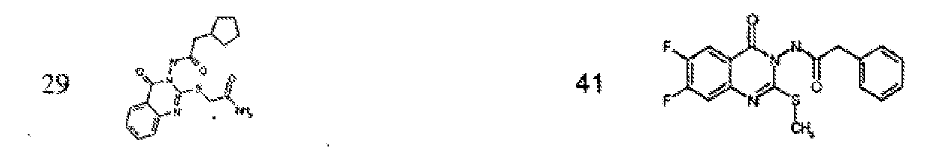

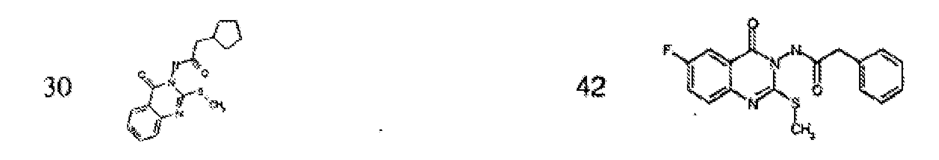

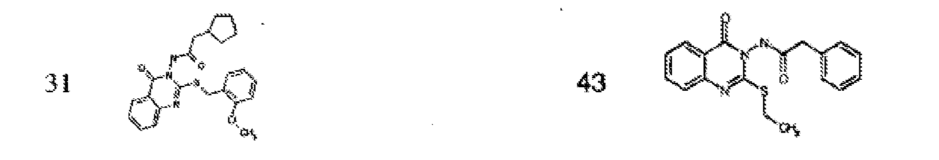

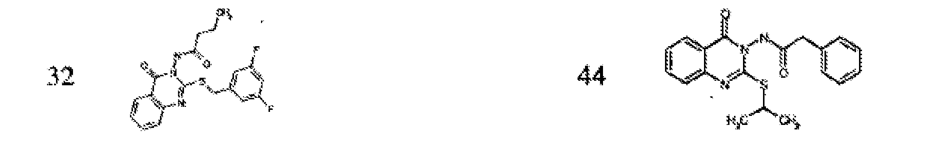

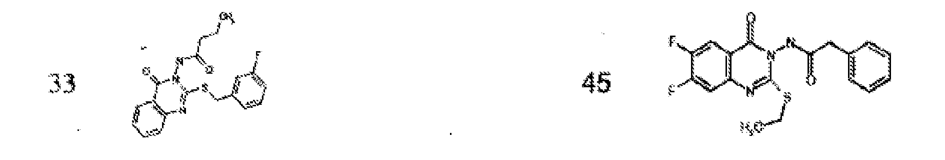

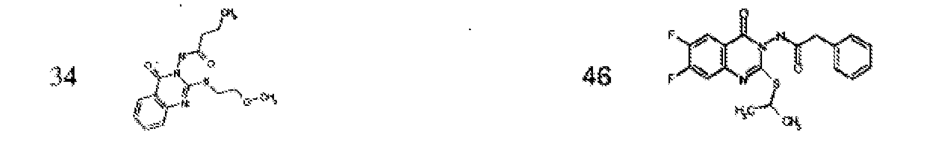

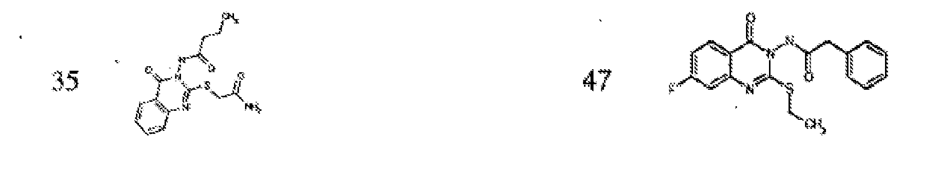

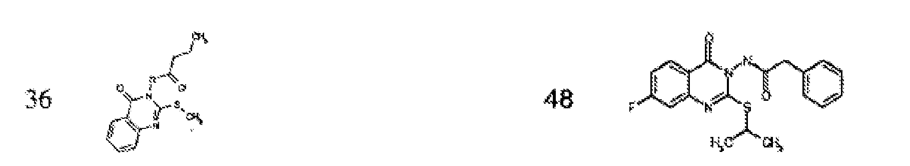

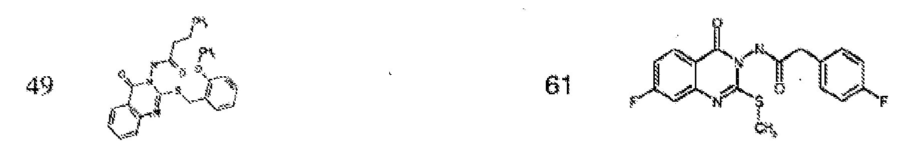

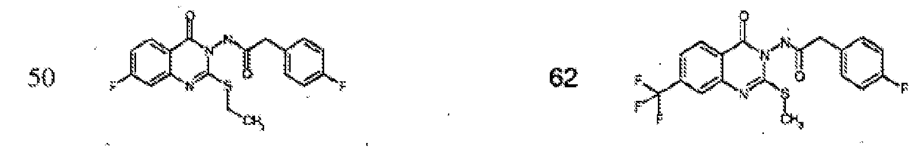

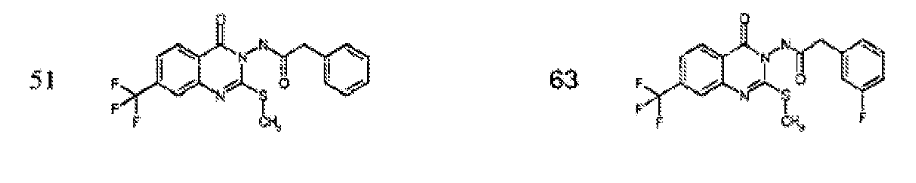

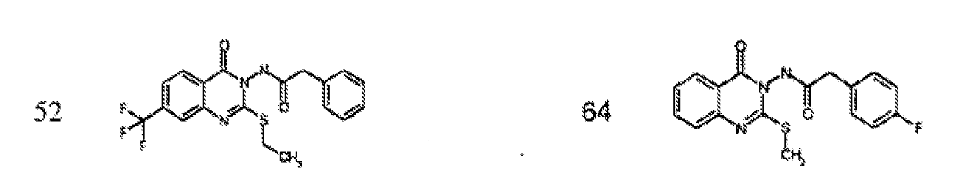

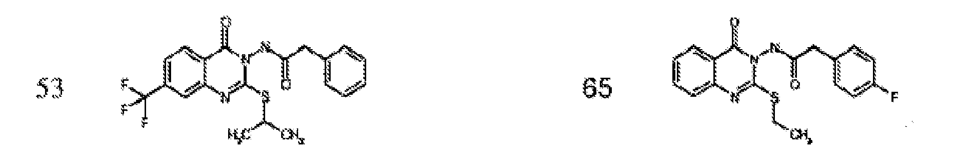

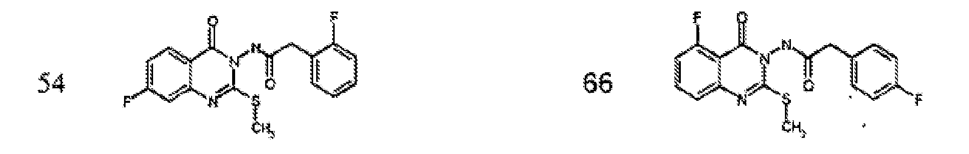

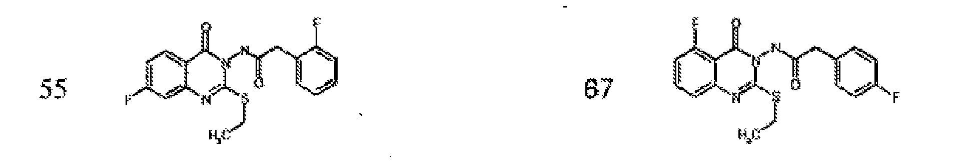

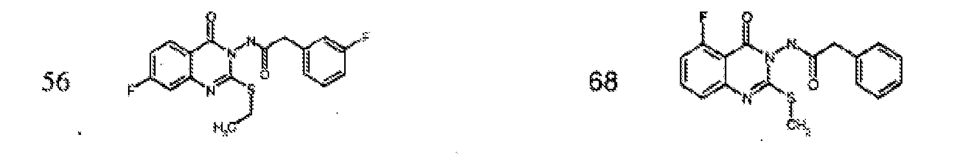

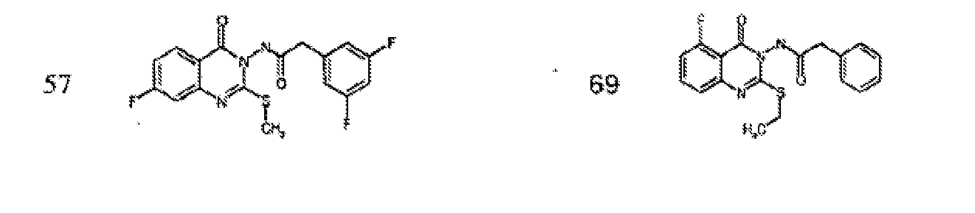

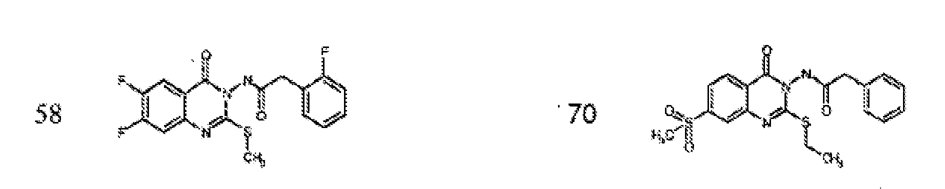

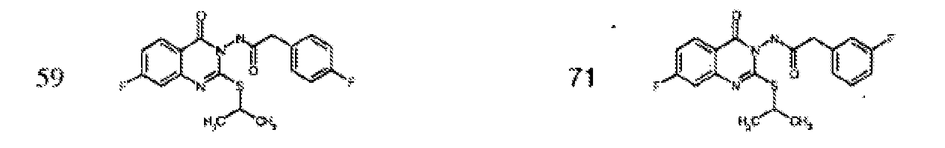

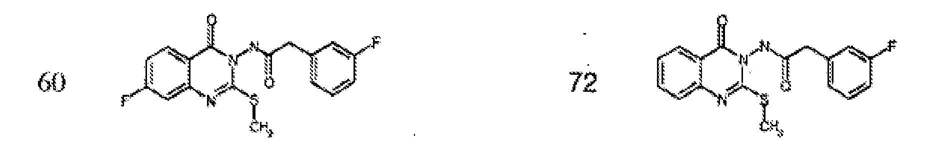

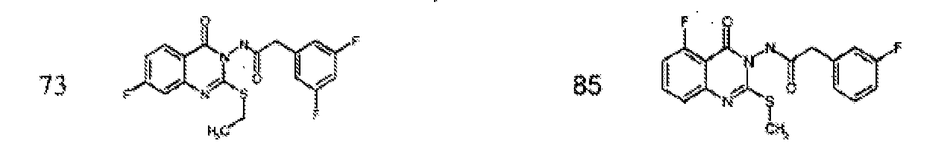

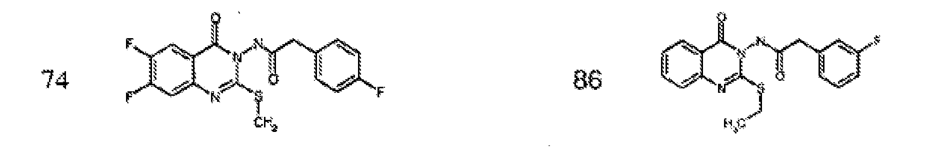

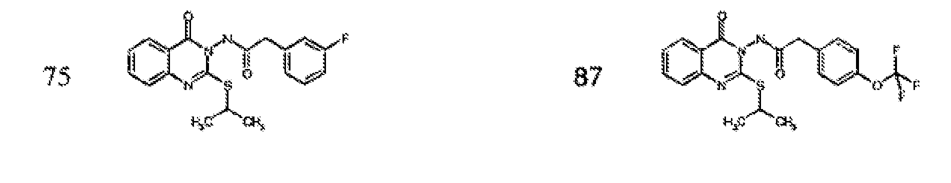

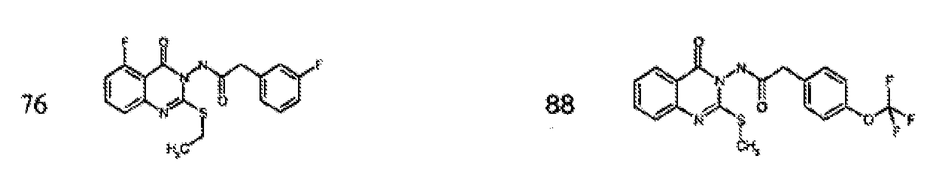

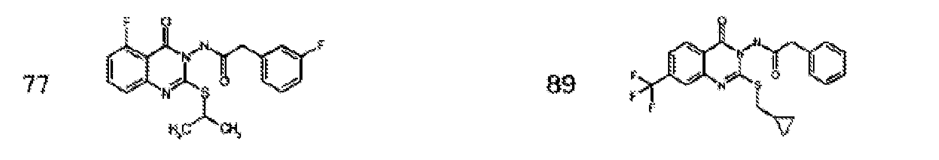

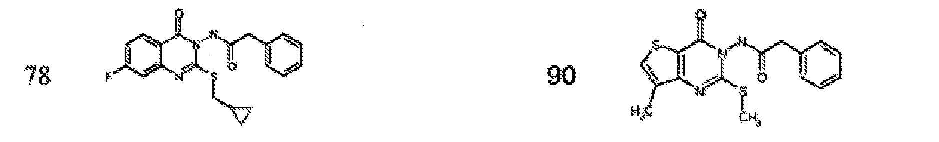

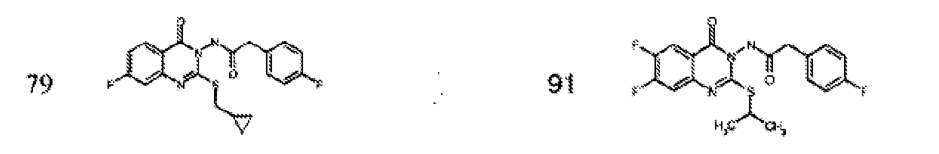

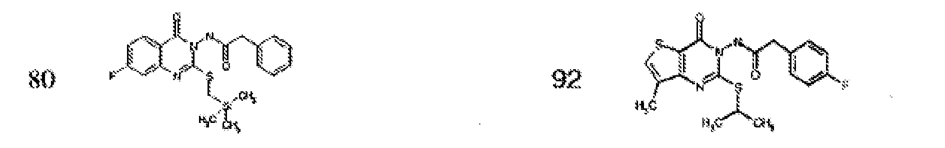

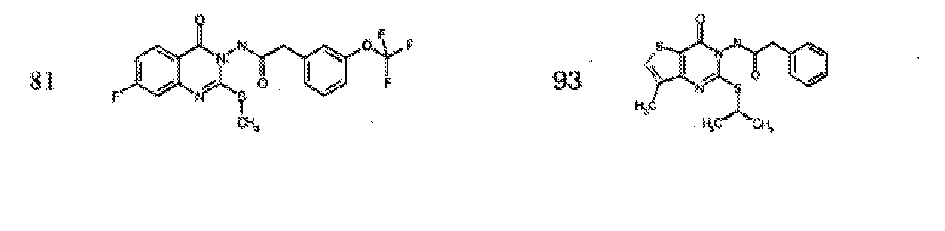

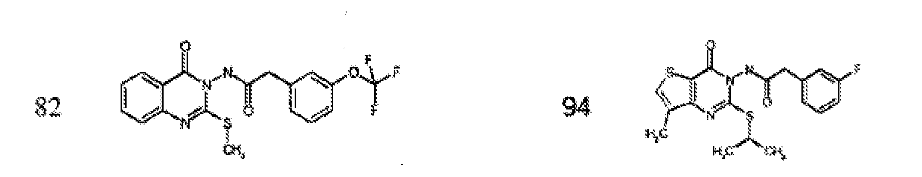

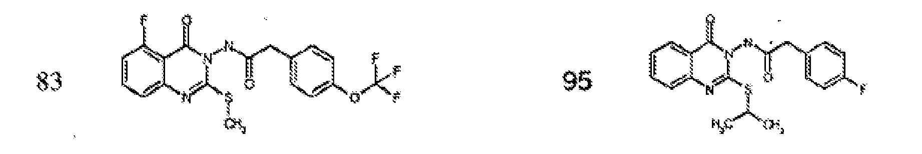

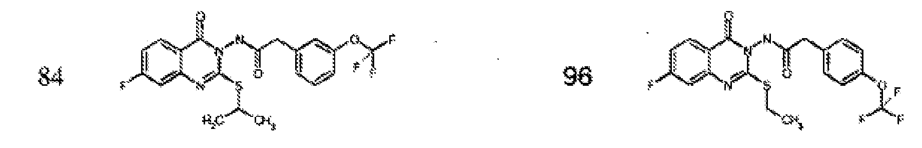

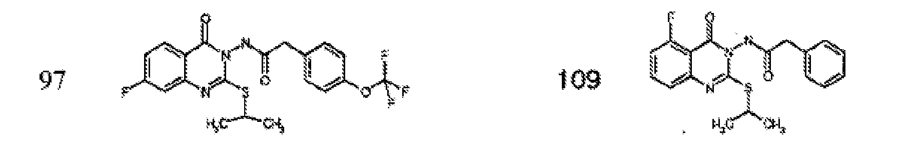

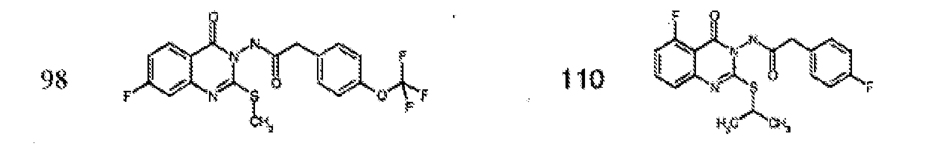

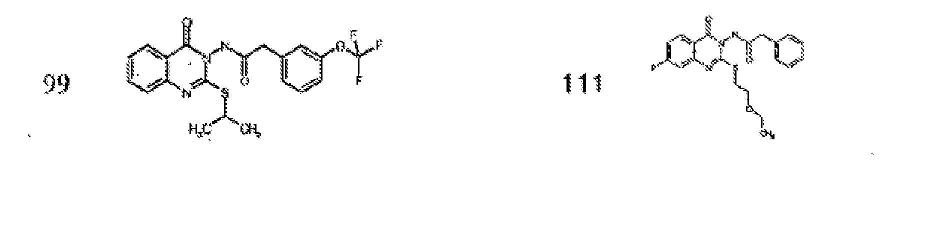

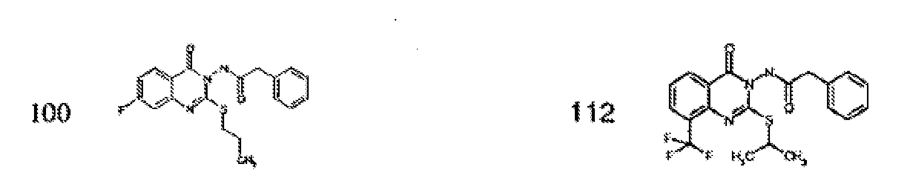

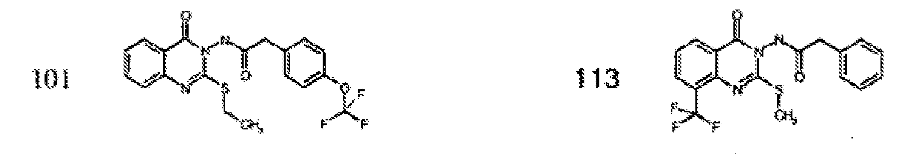

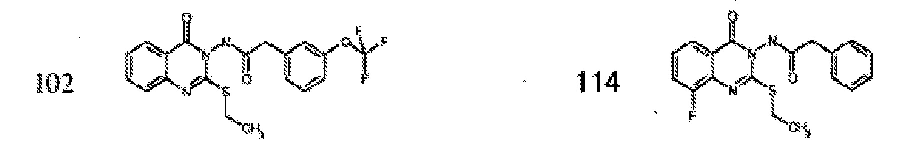

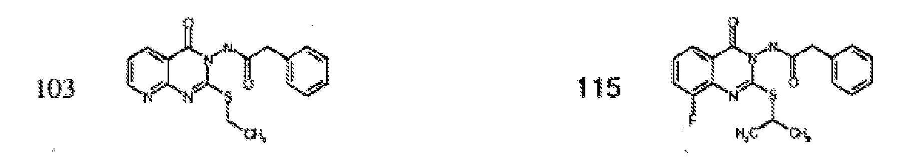

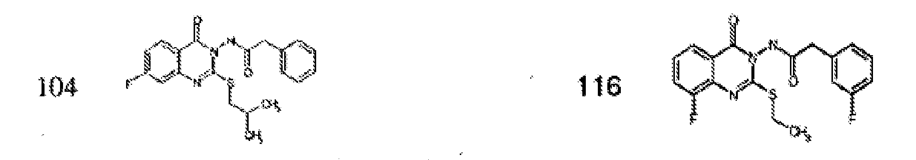

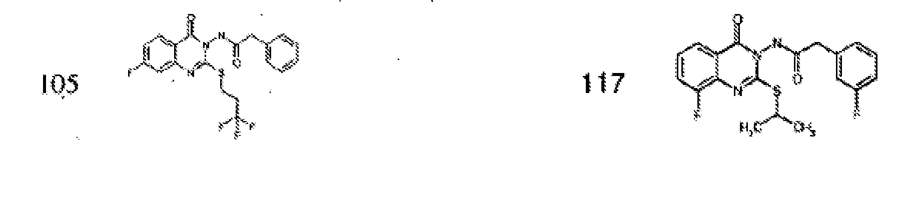

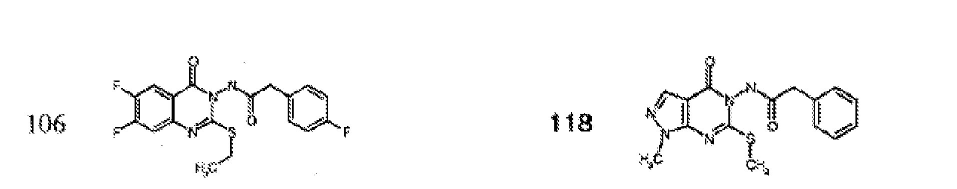

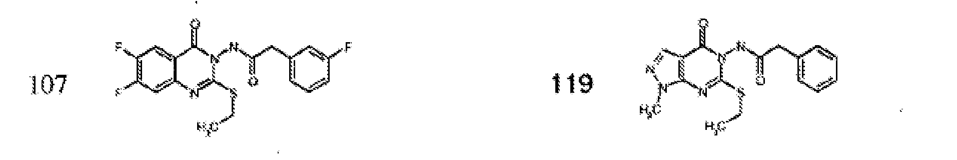

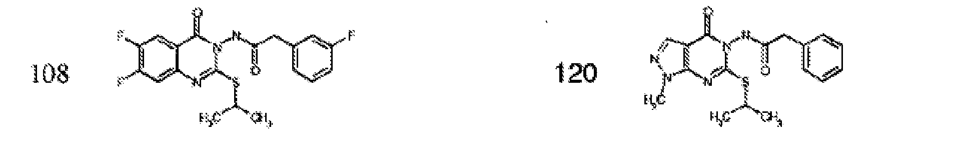

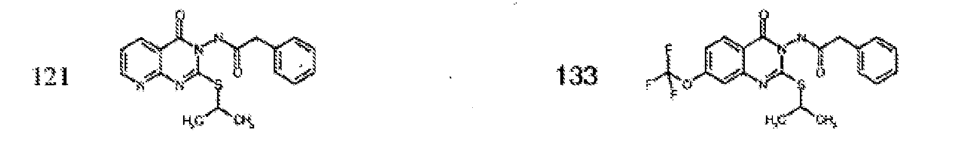

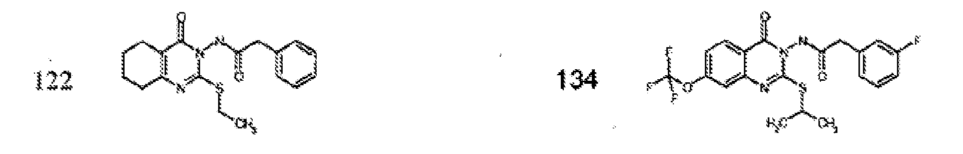

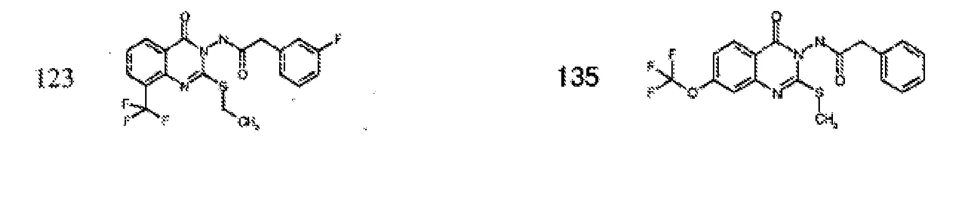

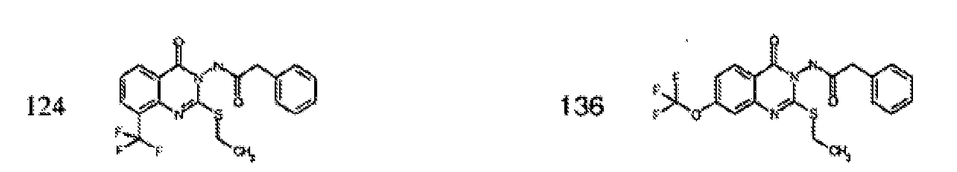

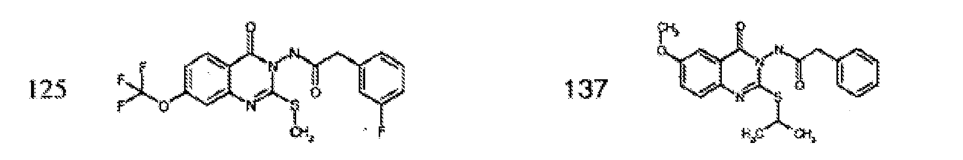

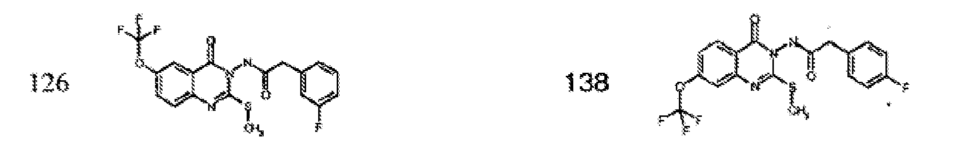

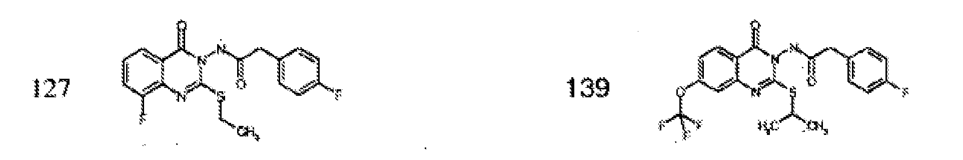

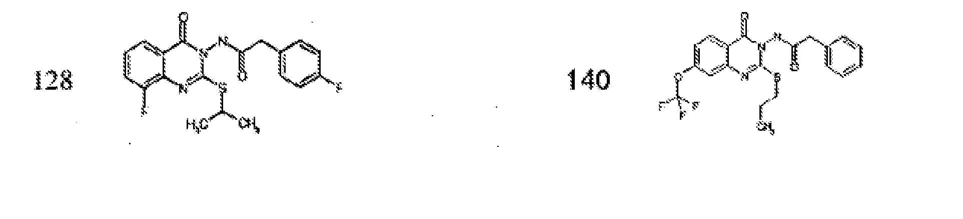

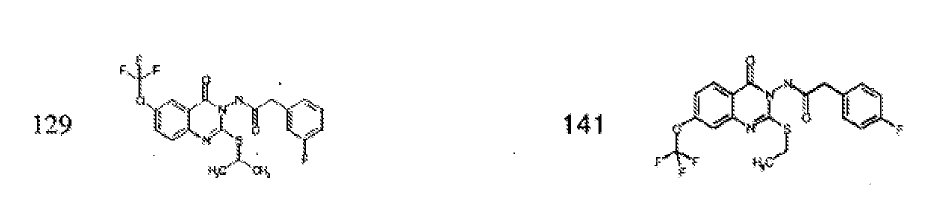

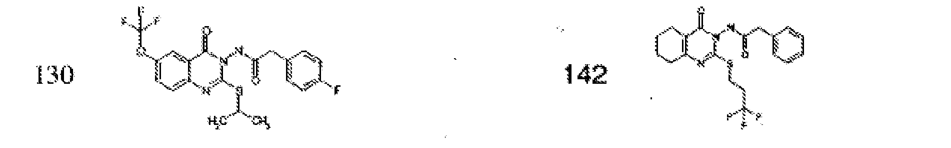

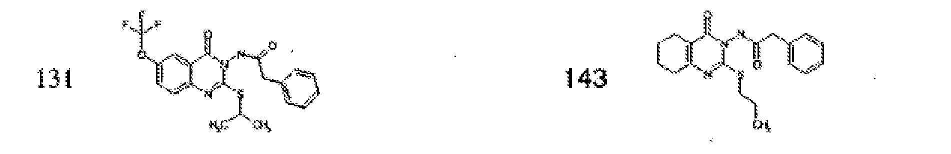

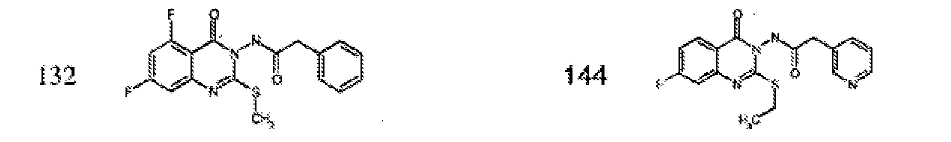

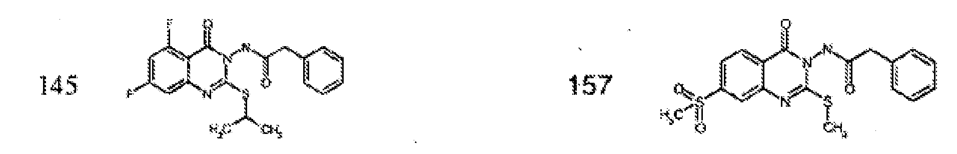

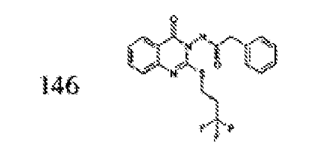

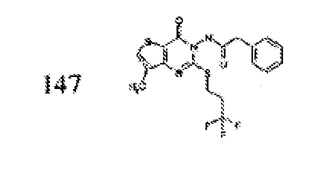

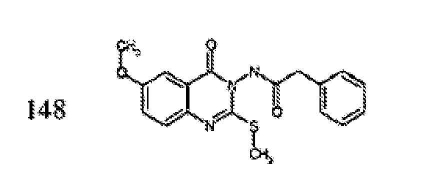

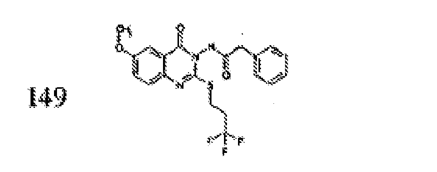

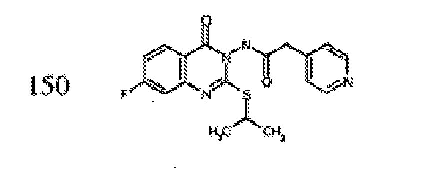

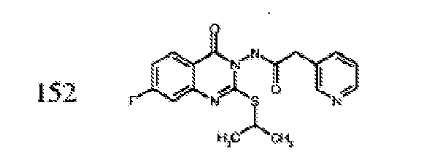

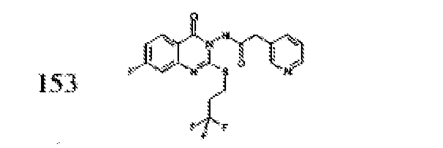

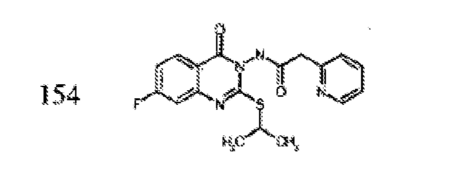

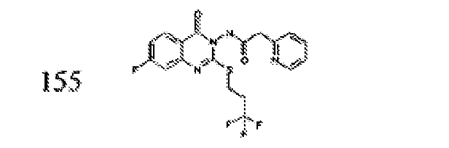

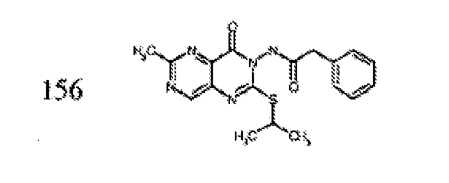

19. Verbindung nach Anspruch 18, worin die Verbindung gewählt ist aus den Verbindungen 40, 44, 45, 46, 47, 48, 50, 51, 52, 53, 54, 55, 56, 57, 59, 60, 61, 62, 63, 64, 65, 66, 67, 68, 73 oder 74.

20. Verbindung nach Anspruch 18, worin die Verbindung gewählt ist aus den Verbindungen 41, 42, 71, 78-81, 84, 91 , 96-98, 100, 104-108, 111, 132 und 145.

21. Verbindung nach Anspruch 1, worin die Verbindung eine Struktur aufweist, die aus den folgenden Formeln gewählt ist:

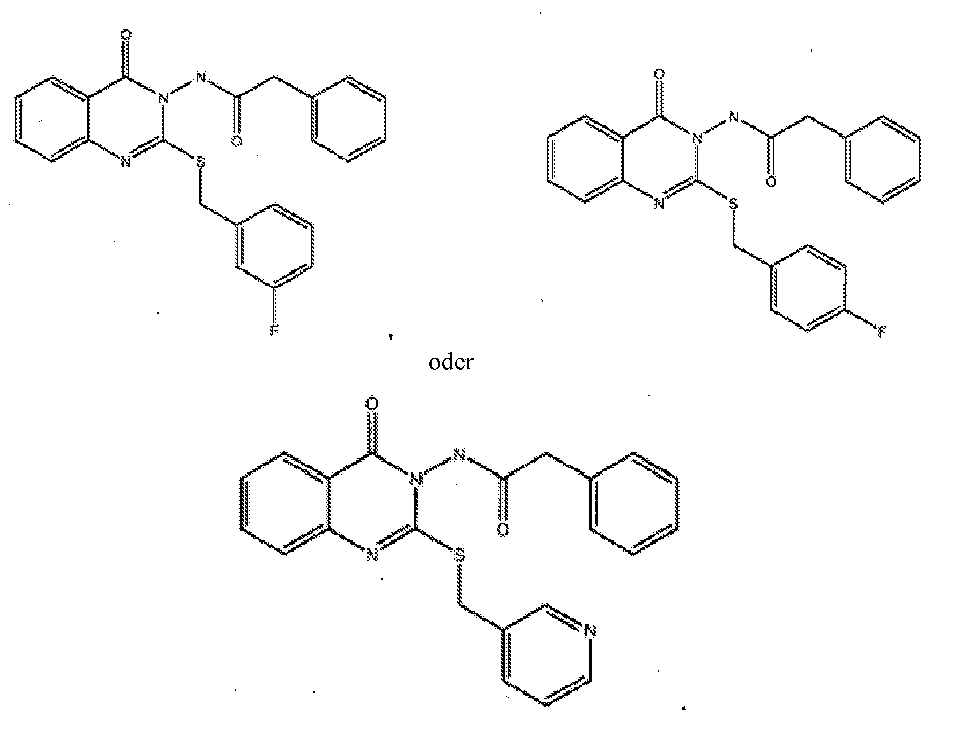

22. Pharmazeutische Zubereitung, umfassend einen pharmazeutisch annehmbaren Träger und eine wirksame Menge einer Verbindung gemäß irgendeinem der vorangehenden Ansprüche.

23. Verbindung nach irgendeinem der Ansprüche 1 bis 21 zur Verwendung als pharmazeutische Substanz.

24. Verbindung nach irgendeinem der Ansprüche 1 bis 21 zur Erhöhung des Ionen-Flusses in einem spannungsabhängigen Kalium-Kanal zur Verwendung bei der Behandlung eines Zustands, der durch spannungsabhängige Kalium-Kanäle vermittelt wird.

25. Verfahren zum Erhöhen des Ionen-Flusses durch spannungsabhängige Kalium-Kanäle in einer Zelle, wobei das Verfahren umfasst ein In-Kontakt-Bringen der Zelle mit einer einen Kalium-Kanal öffnenden Menge einer Verbindung nach irgendeinem der Ansprüche 1 bis 21, mit der Maßgabe, dass das Verfahren kein Verfahren zur Behandlung des menschlichen oder tierischen Körpers durch Therapie ist.

26. Verfahren nach Anspruch 25, worin der spannungsabhängige Kalium-Kanal verantwortlich für den M-Strom ist und/oder die Kanäle spannungsabhängige KCNQ-Kalium-Kanäle in einer Zelle sind.

27. Verwendung einer Verbindung nach irgendeinem der Ansprüche 1 bis 21 für die Herstellung eines Arzneimittels zur Behandlung einer Störung oder eines Zustands des zentralen oder peripheren Nervensystems durch Modulation spannungsabhängiger KCNQ-Kalium-Kanäle.

28. Verwendung nach Anspruch 27, worin die Störung oder der Zustand gewählt ist aus neuronalen Degenerationsstörungen, Migräne, Ataxie, Parkinson'sche Krankheit, bipolare Störungen, Spastizität, Stimmungsstörungen, Gehirntumore, psychotische Störungen, Myokymie, Anfälle, Epilepsie, Verlust des Hörens, Verlust des Sehens, Alzheimer'sche Krankheit, altersbezogener Gedächtnisverlust, Lemmängel, motorische neuronale Krankheiten, trigeminale Neuralgie, Netzhaut-Degeneration, erhöhter intraokularer Druck, Glaucom und Schlaganfall.

29. Verwendung nach Anspruch 28, worin der Zustand oder die Störung Epilepsie oder Anfälle ist.

30. Verwendung nach Anspruch 28, worin der Zustand oder die Störung Hörverlust ist.

31. Verwendung nach Anspruch 27, worin der Zustand oder die Störung Schmerz oder Beklemmung ist.

32. Verwendung nach Anspruch 28, worin der Zustand oder die Störung eine neuronale Degeneration ist.

33. Verwendung nach Anspruch 28, worin der Zustand oder die Störung eine Netzhaut-Degeneration ist.

34. Verwendung einer Verbindung nach irgendeinem der Ansprüche 1 bis 21 für die Herstellung eines Medikaments zur Behandlung eines Zustands gewählt aus Epilepsie, Netzhaut-Degeneration, Angst, Beklemmung, neuronale Degeneration und bipolare Störung durch Modulation eines spannungsabhängigen Kalium-Kanals.

35. Verwendung nach Anspruch 34, worin der Schmerz ein Zustand ist, der gewählt ist aus neuropathischem Schmerz, diabetischem Schmerz, somatischem Schmerz, Hautschmerz, Eingeweide-Schmerz, Entzündungsschmerz, Krebsschmerz, Migräne-Schmerz oder musculoskeletalem Schmerz.

## Revendications

1. Composé ayant la formule : 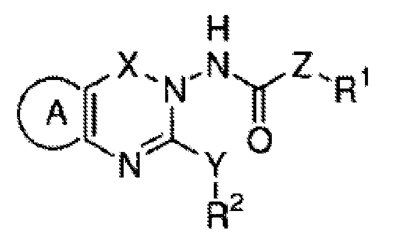 dans laquelle
- A est un élément choisi dans le groupe constitué par aryle substitué ou non substitué à cinq et six chaînons, hétéroaryle substitué ou non substitué à cinq et six chaînons, cycloalkyle en C₄-C₈ substitué ou non substitué et hétérocyclyle à 5-8 chaînons substitué ou non substitué ;
- X représente CO ;
- Z est un élément choisi dans le groupe constitué par - CH₂-, -CHF-, -CF₂-, -CH=CH- et -N(R⁴) (CR^{4a}R^{4b})ₛ⁻, où
- R⁴ est un élément choisi parmi H et un groupe alkyle en C₁-C₅ substitué ou non substitué ;
- R^{4a} et R^{4b} sont des éléments choisis indépendamment parmi H, aryle substitué ou non substitué, hétéroaryle substitué ou non substitué, cycloalkyle en C₃-C₈ substitué ou non substitué, hétérocyclyle à 5-7 chaînons substitué ou non substitué et alkyle en C₁-C₈ substitué ou non substitué ;
- s est un entier de 1 à 3 ;
- Y représente S(O)ₙ, où n est un entier de 0-2 ;
- R¹ est un élément choisi parmi aryle substitué ou non substitué, hétéroaryle substitué ou non substitué, cycloalkyle en C₃-C₈ substitué ou non substitué, hétérocyclyle à 5-7 chaînons substitué ou non substitué et alkyle en C₁-C₈ substitué ou non substitué ; et
- R² est un élément choisi parmi le groupe constitué par CF₃, alkyle en C₁-C₈ substitué ou non substitué, aryle substitué ou non substitué, hétéroaryle substitué ou non substitué, cycloalkyle en C₃-C₈ substitué ou non substitué, hétérocyclyle à 3-7 chaînons substitué on non substitué.

2. Composé selon la revendication 1, à la condition que, lorsque A représente phényle, Z représente -CH₂-, et R¹ représente phényle, phényle substitué ou hétéroaryle, alors R² est autre qu'un groupe benzyle, benzyle substitué, alkylhétéroaryle, alkylhétérocyclyle ou cyanométhyle.

3. Composé selon la revendication 1 ou la revendication 2, dans lequel A est un élément choisi parmi aryle substitué ou non substitué et hétéroaryle substitué on non substitué.

4. Composé selon la revendication 3, dans lequel A représente phényle substitué on non substitué.

5. Composé selon la revendication 4, dans lequel A représente phényle substitué par un ou deux groupes choisis parmi halogène, nitrile, alkyle en C₁-C₄ substitué ou non substitué, SCF₃, trifluorométhyle et trifluorométhoxy.

6. Composé selon l'une quelconque des revendications précédentes, dans lequel Z représente -CH₂-.

7. Composé selon l'une quelconque des revendications précédentes, dans lequel R¹ est un élément choisi parmi aryle substitué ou non substitué et hétéroaryle substitué ou non substitué.

8. Composé selon la revendication 7, dans lequel R¹ représente phényle substitué ou non substitué.

9. Composé selon la revendication 8, dans lequel R¹ est un élément choisi parmi phényle et phényle substitué par un ou plusieurs parmi halogène, CF₃ et OCF₃.

10. Composé selon l'une quelconque des revendications précédentes, dans lequel R² est un groupe alkyle acyclique saturé en C₁-C₆ substitué ou non substitué.

11. Composé selon la revendication 10, dans lequel R² est un groupe alkyle acyclique saturé en C₁-C₄.

12. Composé selon la revendication 1, ayant la formule : 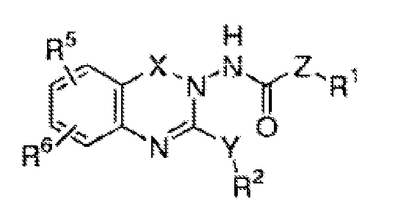 dans laquelle
- X représente CO ;
- Z est un élément choisi parmi -CH₂-, -CHF-, -CF₂-, - CH=CH- et -N(R⁴) (CR^{4a}R^{4b})ₛ⁻, où
- R⁴ est un élément choisi parmi H et un groupe alkyle en C₁-C₅ substitué ou non substitué ;
- R^{4a} et R^{4b} sont des éléments choisis indépendamment parmi H, aryle substitué ou non substitué, hétéroaryle substitué ou non substitué, cycloalkyle en C₃-C₈ substitué ou non substitué, hétérocyclyle à 5-7 chaînons substitué ou non substitué et alkyle en C₁-C₈ substitué ou non substitué ;
- s est un entier de 1 à 3 ;
- Y représente S ;
- R¹ est un élément choisi parmi aryle substitué ou non substitué, hétéroaryle substitué ou non substitué, cycloalkyle en C₃-C₈ substitué ou non substitué, hétérocyclyle à 5-7 chaînons substitué ou non substitué et alkyle en C₁-C₈ substitué ou non substitué ;
- R² est un élément choisi parmi CF₃, alkyle en C₁-C₈ substitué ou non substitué, aryle substitué ou non substitué, hétéroaryle substitué ou non substitué, cycloalkyle en C₃-C₈ substitué ou non substitué, hétérocyclyle saturé à 3-7 chaînons substitué ou non substitué ;
- R⁵ et R⁶ sont choisis indépendamment parmi H, halo, CF₃ CF₃O, NO₂, CN, S(O)ₘR⁷, COOR⁸, CONR⁹R¹⁰, SO₂NR¹¹R¹², S(O)ₘCF₃, alkyle en C₁-C₆ substitué ou non substitué et cycloalkyle en C₃-C₇ substitué ou non substitué, où
- R⁷ et R⁸ sont indépendamment choisis parmi alkyle en C₁-C₅ substitué ou non substitué et cycloalkyle en C₃-C₇ substitué ou non substitué ;
- m est un entier de 0 à 2 ; et
- R⁹, R¹⁰, R¹¹ et R¹² sont choisis indépendamment parmi H, alkyle en C₁-C₅ substitué ou non substitué, cycloalkyle en C₃-C₇ substitué ou non substitué, et R⁹ et R¹⁰ ou R¹¹ et R¹², conjointement avec l'atome d'azote auquel ils sont attachés, sont réunis facultativement pour former un cycle à 5 à 7 chaînons.

13. Composé selon la revendication 12, dans lequel
- Z représente CH₂ ; et
- R¹ représente phényle substitué ou non substitué.

14. Composé selon l'une des revendications 12 ou 13, dans lequel :
- R² est un élément choisi parmi alkyle en C₁-C₄ substitué ou non substitué, cycloalkyle en C₃-C₆ substitué ou non substitué et hétérocyclyle en C₃-C₆ substitué ou non substitué ; et
- R⁵ et R⁶ sont des éléments choisis indépendamment parmi H, halo, CF₃, OCF₃, alkyle en C₁-C₅ substitué ou non substitué, SCF₃ et CN.

15. Composé selon la revendication 1, ayant la formule : 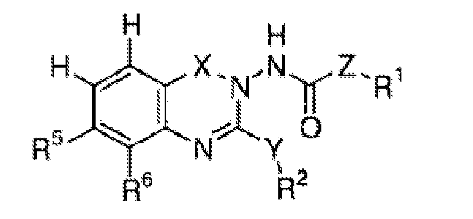 dans laquelle :
- X représente CO ;
- Z est un élément choisi parmi -CH₂-, -CHF-, -CF₂-, - CH=CH- et -N(R⁴) (CR^{4a}R^{4b})ₛ⁻, où
- R⁴ est un élément choisi parmi H et un groupe alkyle en C₁-C₅ substitué ou non substitué ;
- R^{4a} et R^{4b} sont des éléments choisis indépendamment parmi H, aryle substitué ou non substitué, hétéroaryle substitué ou non substitué, cycloalkyle en C₃-C₈ substitué ou non substitué, hétérocyclyle à 5-7 chaînons substitué ou non substitué et alkyle en C₁-C₈ substitué ou non substitué ;
- s est un entier de 1 à 3 ;
- R¹ est un élément choisi parmi aryle substitué ou non substitué, hétéroaryle substitué ou non substitué, cycloalkyle en C₃-C₈ substitué ou non substitué, hétérocyclyle à 5-7 chaînons substitué ou non substitué, alkyle en C₁-C₈ substitué ou non substitué ;
- Y représente S ;
- R² est un élément choisi parmi CF₃, alkyle en C₁-C₈ substitué ou non substitué, aryle substitué ou non substitué, hétéroaryle substitué ou non substitué, cycloalkyle en C₃-C₈ substitué ou non substitué, hétérocyclyle saturé à 3-7 chaînons substitué ou non substitué ;
- R⁵ et R⁶ sont choisis indépendamment parmi halo, CF₃ CF₃O, NO₂, CN, S(O)ₘR⁷, COOR⁸, CONR⁹R¹⁰, SO₂NR¹¹R¹², S(O)ₘCF₃, alkyle en C₁-C₆ substitué ou non substitué et cycloalkyle en C₃-C₇ substitué ou non substitué, où
- R⁷ et R⁸ sont choisis indépendamment parmi alkyle en C₁-C₅ substitué ou non substitué et cycloalkyle en C₃-C₇ substitué ou non substitué ;
- m est un entier de 0 à 2 ; et
- R⁹, R¹⁰, R¹¹ et R¹² sont choisis indépendamment parmi H, alkyle en C₁-C₅ substitué ou non substitué, cycloalkyle en C₃-C₇ substitué ou non substitué et R⁹ et R¹⁰ ou R¹¹ et R¹², conjointement avec l'atome d'azote auquel ils sont attachés, sont facultativement réunis pour former un noyau à 5 à 7 chaînons.

16. Composé selon la revendication 15, dans lequel
- Z représente CH₂ ;
et
- R¹ représente phényle substitué ou non substitué.

17. Composé selon la revendication 15, dans lequel :
- R² est un élément choisi parmi alkyle en C₁-C₄ substitué ou non substitué, cycloalkyle en C₃-C₆ substitué ou non substitué et hétérocyclyle en C₃-C₇ substitué ou non substitué ; et
- R⁵ et R⁶ sont des éléments choisis indépendamment parmi halo, CF₃, OCF₃, alkyle en C₁-C₅ substitué ou non substitué, SCF₃ et CN.

18. Composition selon la revendication 1, dans lequel le composé a une structure choisie parmi les formules suivantes :

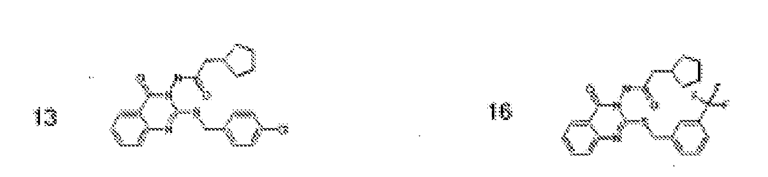

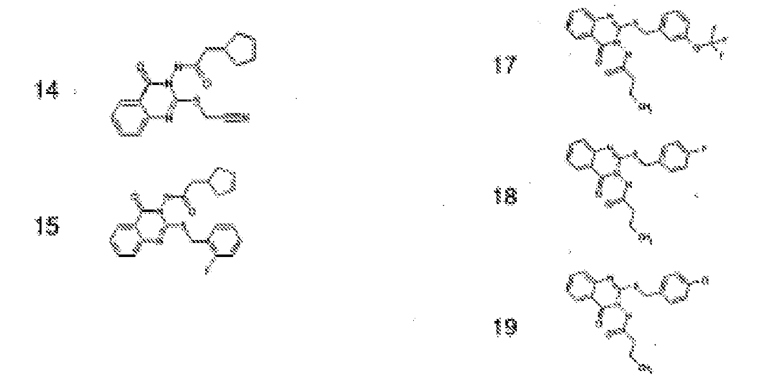

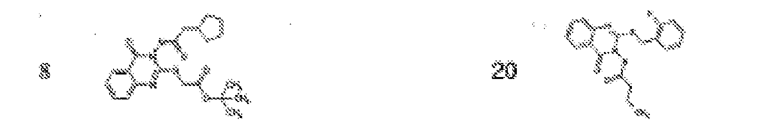

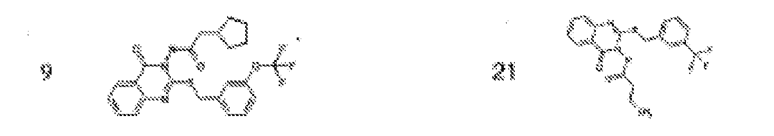

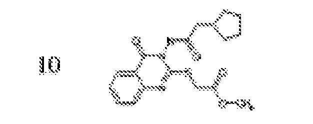

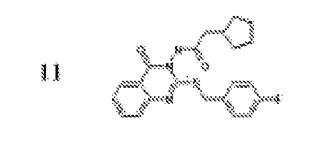

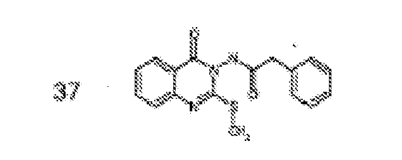

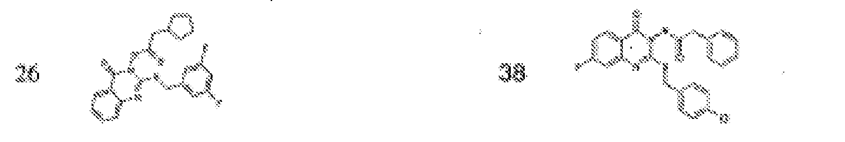

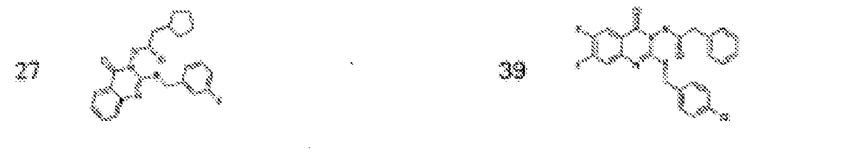

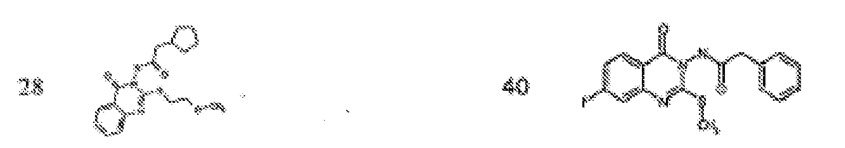

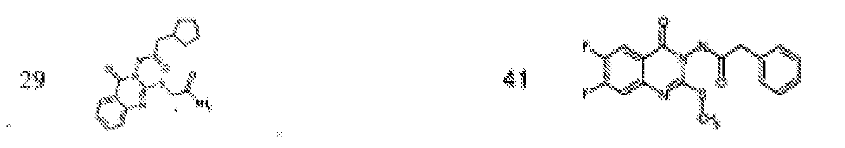

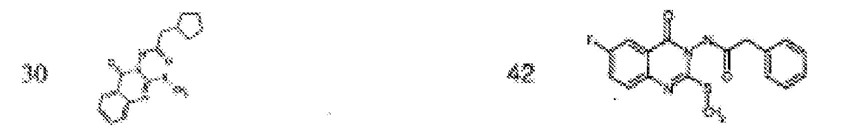

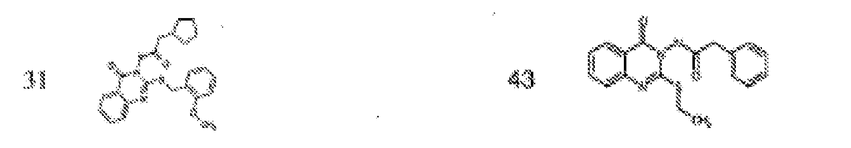

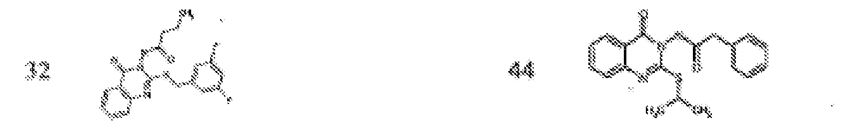

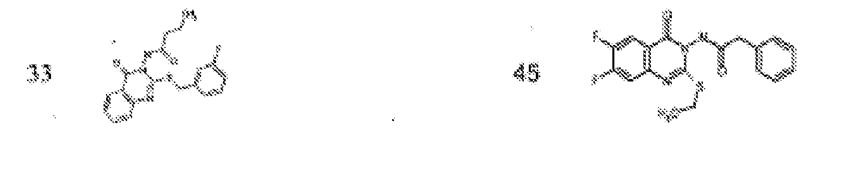

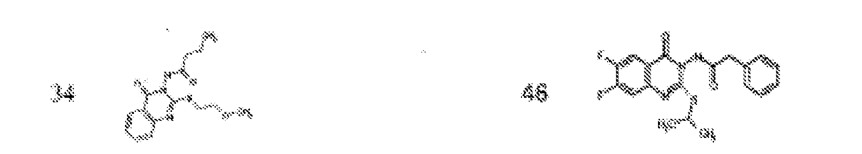

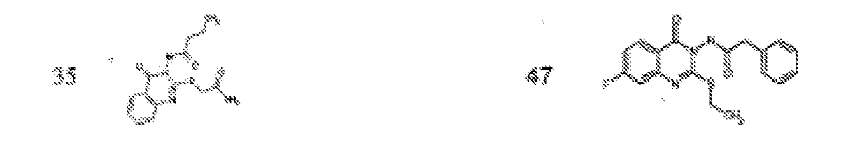

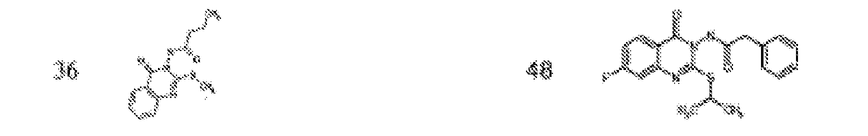

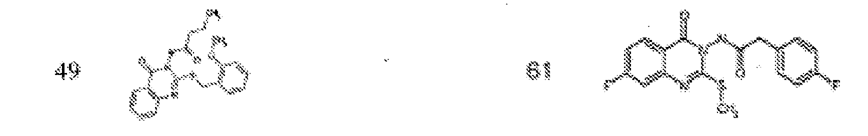

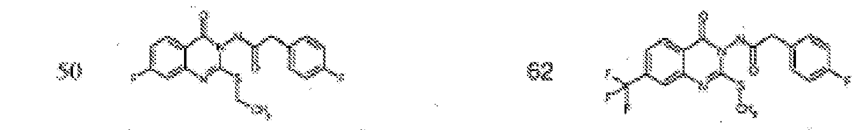

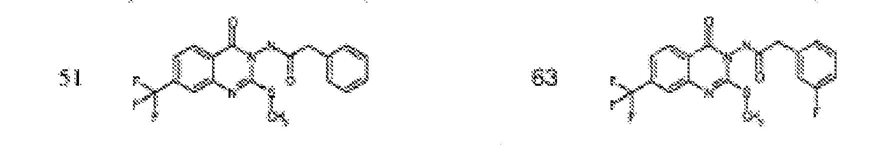

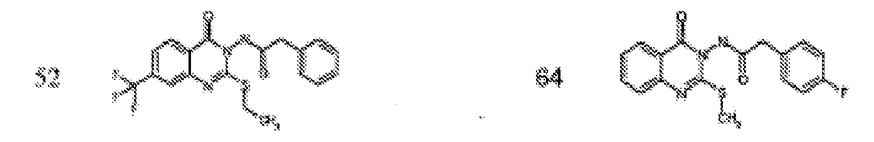

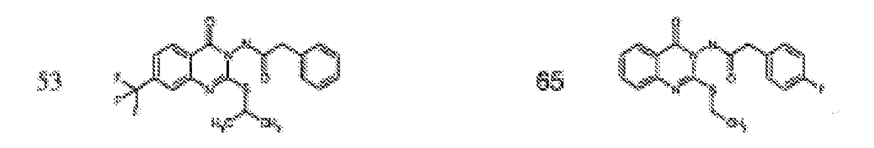

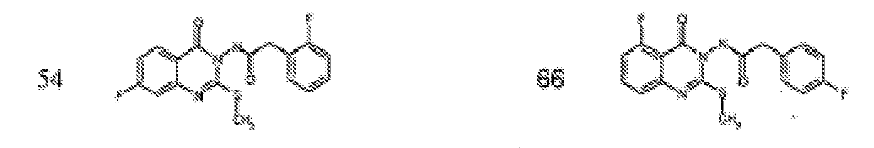

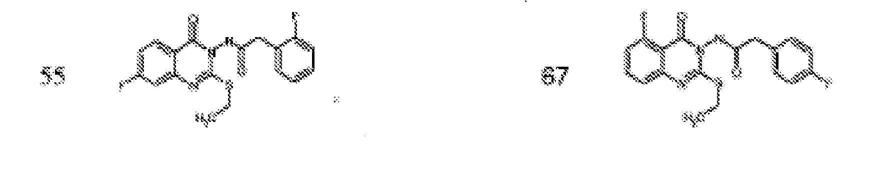

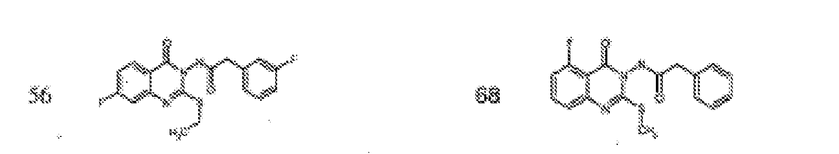

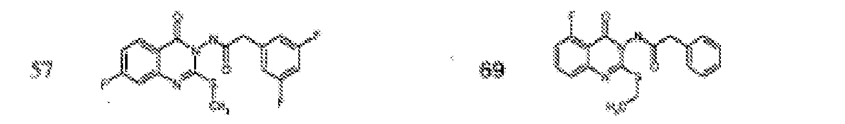

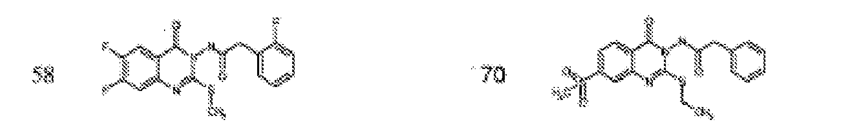

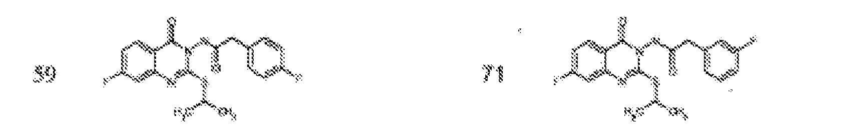

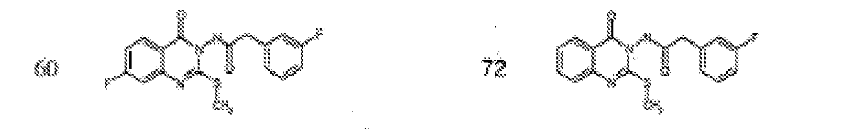

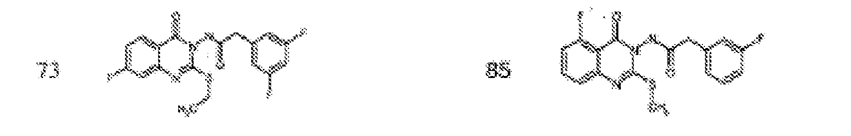

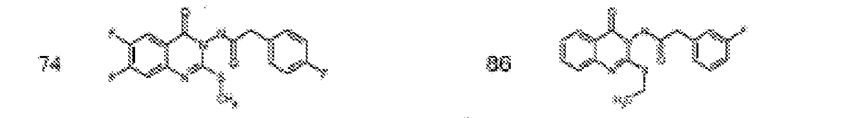

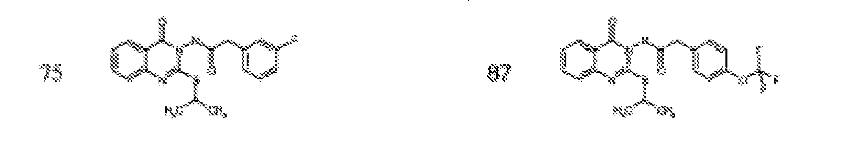

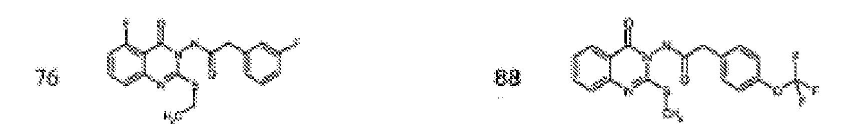

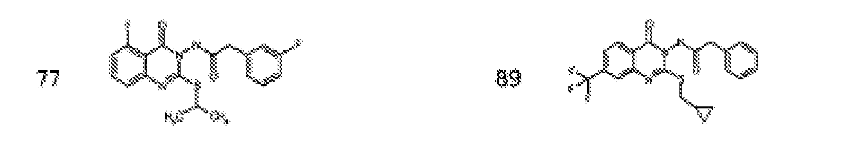

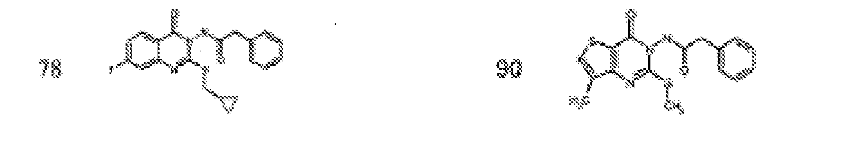

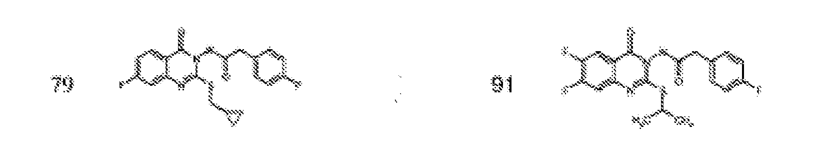

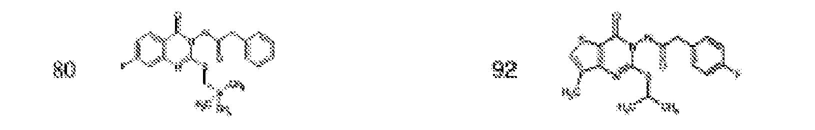

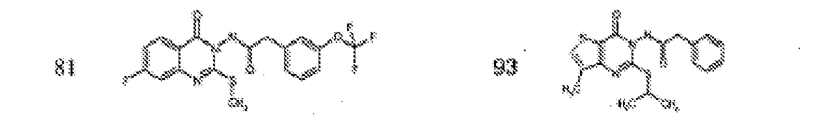

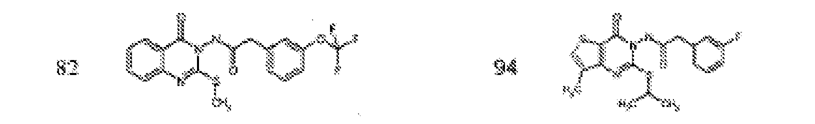

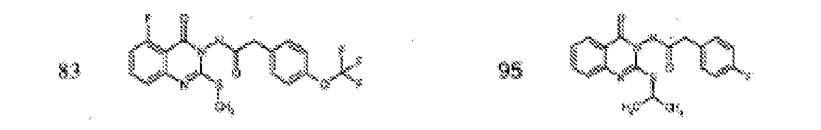

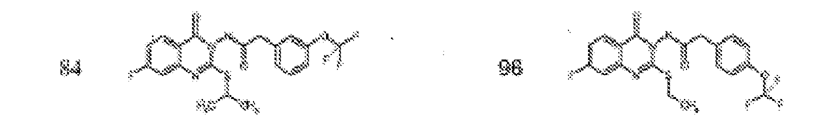

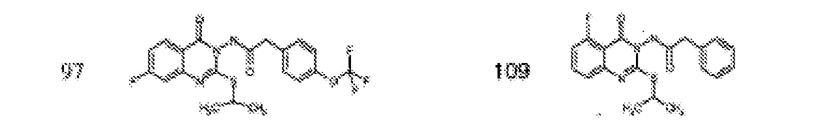

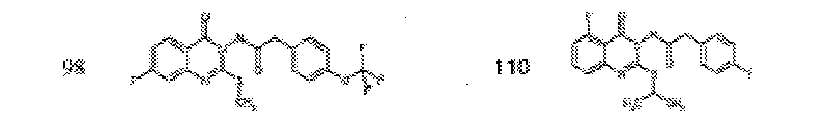

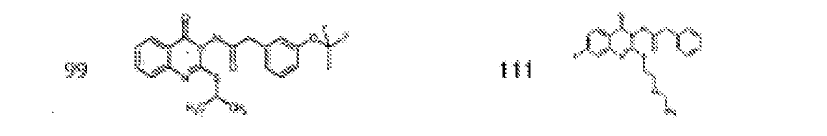

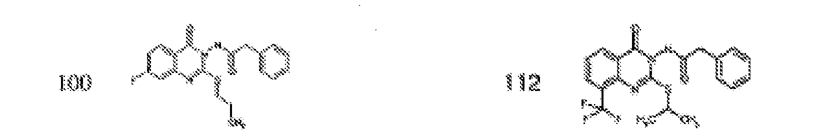

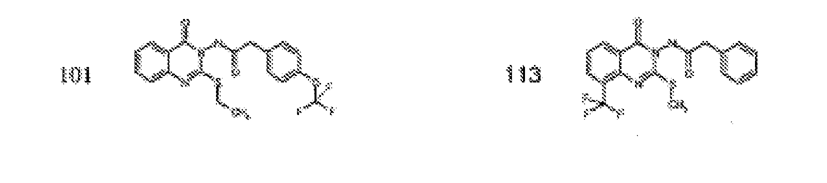

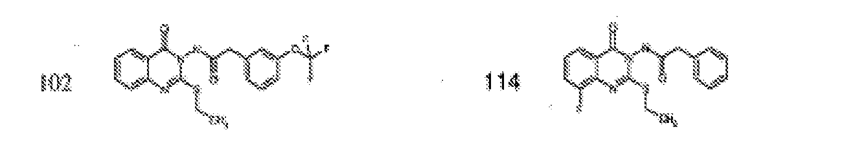

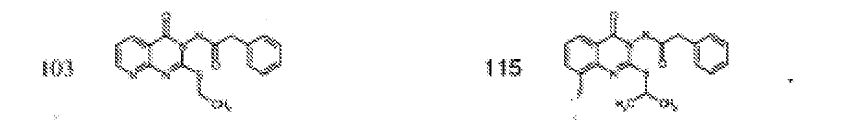

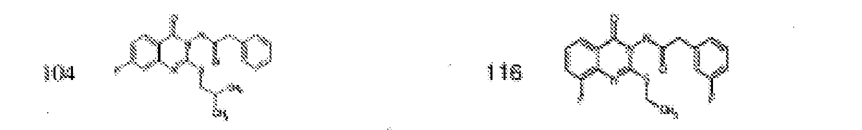

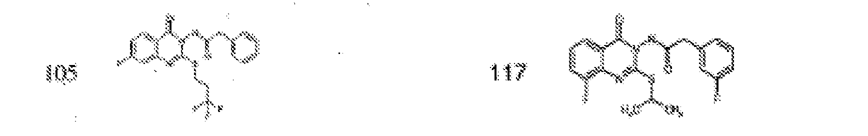

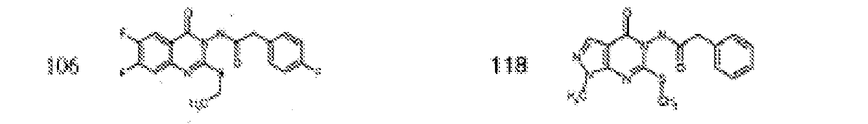

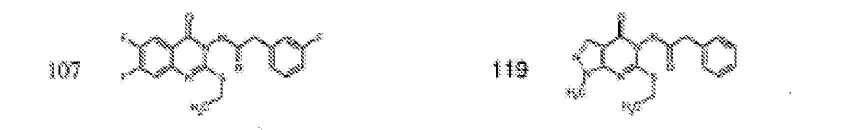

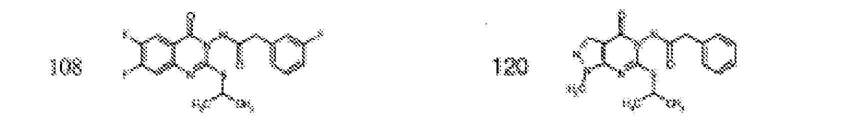

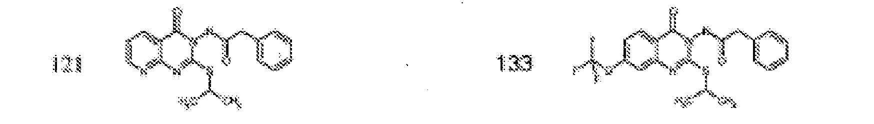

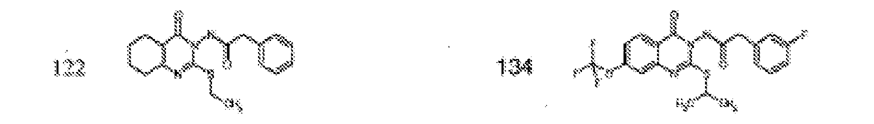

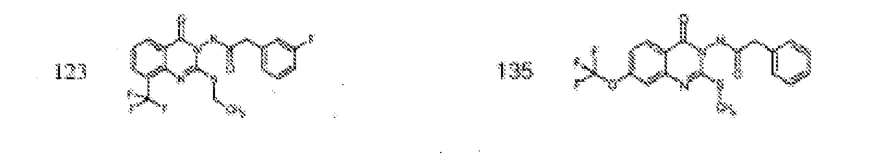

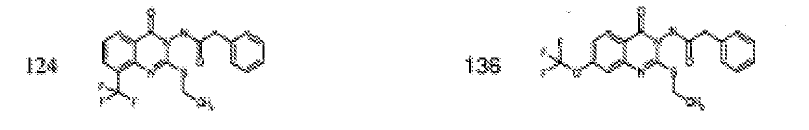

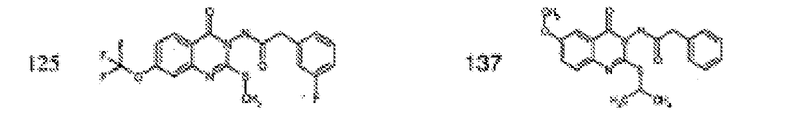

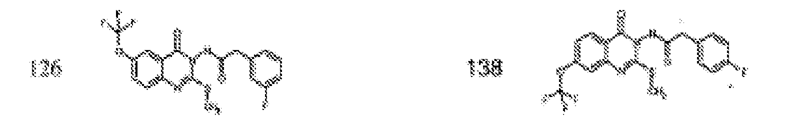

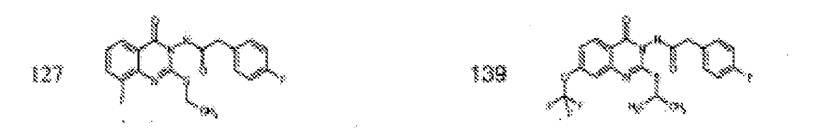

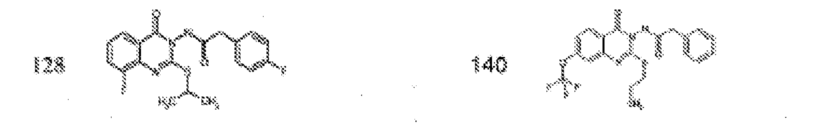

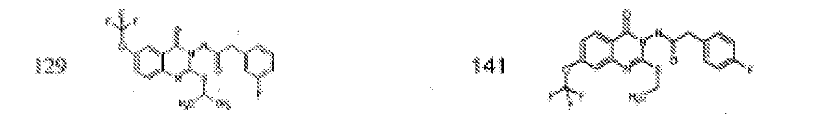

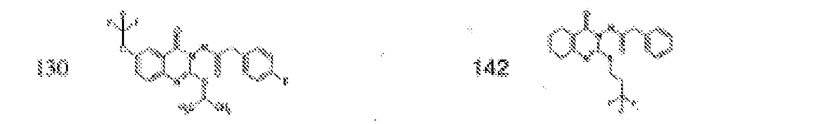

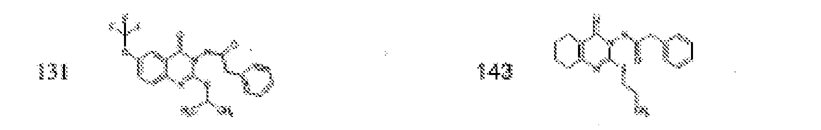

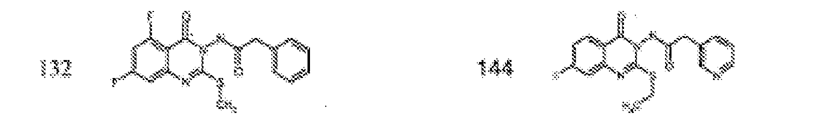

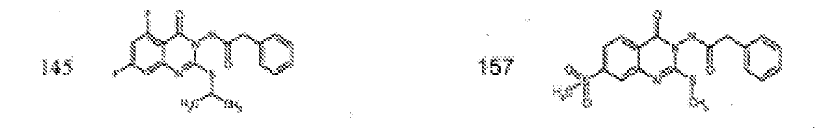

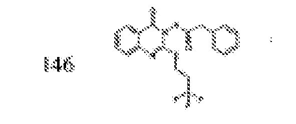

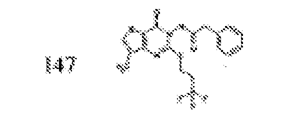

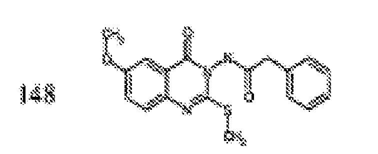

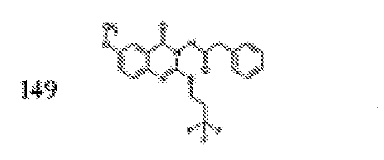

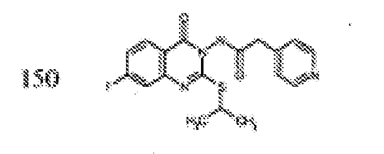

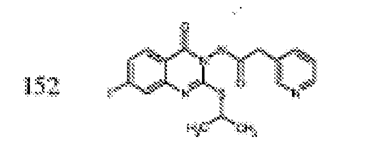

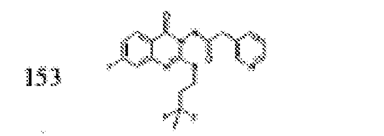

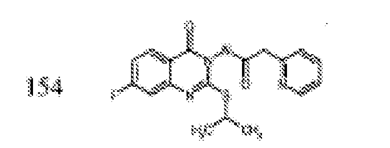

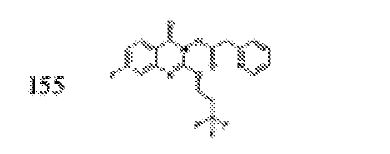

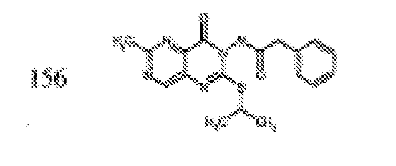

19. Composé selon la revendication 18, dans lequel le composé est choisi parmi les composés 40, 44, 45, 46, 47, 48, 50, 51, 52, 53, 54, 55, 56, 57, 59, 60, 61, 62, 63, 64, 65, 66, 67, 68, 73 ou 74.

20. Composé selon la revendication 18, dans lequel le composé est choisi parmi les composés 41, 42, 71, 78-81, 84, 91, 96-98, 100, 104-108, 111, 132 et 145.

21. Composé selon la revendication 1, dans lequel le composé a une structure choisie parmi les formules suivantes :

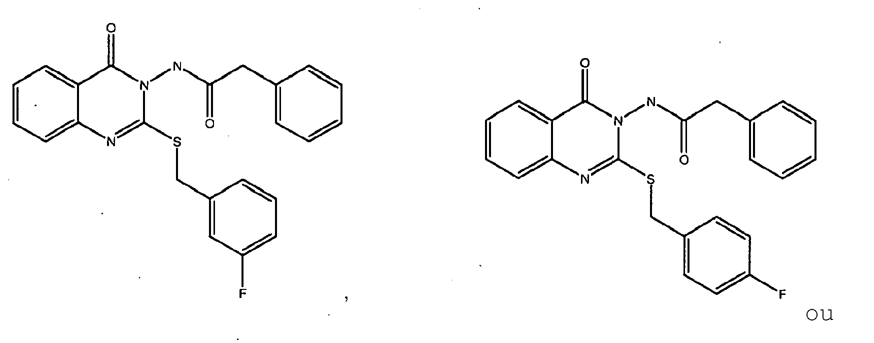

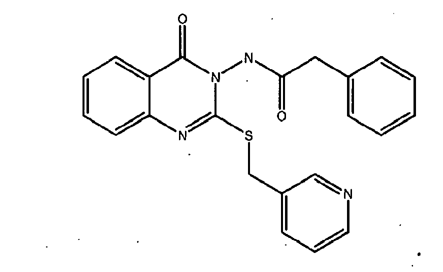

22. Composition pharmaceutique comprenant un excipient pharmaceutiquement acceptable et une quantité efficace d'un composé tel que défini à l'une quelconque des revendications précédentes.

23. Composé selon l'une quelconque des revendications 1 à 21 destiné à être utilisé comme produit pharmaceutique.

24. Composé selon l'une quelconque des revendications 1 à 21 pour augmenter le flux ionique dans un canal potassique dépendant du voltage en vue d'une utilisation dans le traitement d'un état à médiation par les canaux potassiques dépendant du voltage.

25. Procédé d'augmentation du flux ionique à travers les canaux potassiques dépendant du voltage dans une cellule, ledit procédé comprenant la mise en contact de ladite cellule avec une quantité d'ouverture des canaux potassiques d'un composé tel que défini à l'une quelconque des revendications 1 à 21, à la condition que le procédé ne soit pas un procédé de traitement du corps humain ou animal par thérapie.

26. Procédé selon la revendication 25, dans lequel ledit canal potassique dépendant du voltage est responsable du courant M et/ou les canaux sont des canaux potassiques dépendant du voltage KCNQ dans une cellule.

27. Utilisation d'un composé tel que défini à l'une quelconque des revendications 1 à 21 pour la fabrication d'un médicament pour le traitement d'un trouble ou état du système nerveux central ou périphérique par modulation d'un canal potassique dépendant du voltage KCNQ.

28. Utilisation selon la revendication 27, dans lequel ledit trouble ou état est choisi parmi les troubles de la dégénérescence neuronale, la migraine, l'ataxie, la maladie de Parkinson, les troubles bipolaires, la spasticité, les troubles de l'humeur, les tumeurs cérébrales, les troubles psychotiques, la myokymie, les crises, l'épilepsie, la perte auditive, la perte de la vision, la maladie d'Alzheimer, la perte de mémoire liée à l'âge, les déficiences de l'apprentissage, les maladies des motoneurones, la névralgie essentielle du trijumeau, la dégénérescence rétinienne, la pression intraoculaire élevée, le glaucome et l'accident vasculaire cérébral.

29. Utilisation selon la revendication 28, dans laquelle ledit état ou trouble est l'épilepsie ou les crises.

30. Utilisation selon la revendication 28, dans laquelle ledit état ou trouble est la perte auditive.

31. Utilisation selon la revendication 27, dans laquelle ledit état ou trouble est la douleur ou l'anxiété.

32. Utilisation selon la revendication 28, dans laquelle ledit état ou trouble est la dégénérescence neuronale.

33. Utilisation selon la revendication 28, dans laquelle ledit état ou trouble est la dégénérescence rétinienne.

34. Utilisation d'un composé selon l'une quelconque des revendications 1 à 21 pour la fabrication d'un médicament pour le traitement d'un élément choisi parmi l'épilepsie, la dégénérescence rétinienne, la douleur, l'anxiété, la dégénérescence neuronale et le trouble bipolaire par modulation d'un canal potassique dépendant du voltage.

35. Utilisation selon la revendication 34, dans laquelle ladite douleur est un élément choisi parmi la douleur neuropathique, la douleur diabétique, la douleur somatique, la douleur cutanée, la douleur viscérale, la douleur inflammatoire, la douleur du cancer, la douleur de la migraine ou la douleur musculo-squelettique.
